(19) Europäisches Patentamt
European Patent Office
Office européen des brevets

(11) **EP 4 706 758 A2**

(12) **EUROPEAN PATENT APPLICATION**

(43) Date of publication:
**11.03.2026 Bulletin 2026/11**

(21) Application number: **25207876.1**

(22) Date of filing: **21.05.2020**

(51) International Patent Classification (IPC):
***A61P 3/12*** *(2006.01)*

(52) Cooperative Patent Classification (CPC):
(C-Sets available)
**A61K 45/06; A61K 31/4725; A61K 31/785; A61P 3/00; A61P 3/12** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2019 US 201962851099 P**
**23.05.2019 US 201962852299 P**

(62) Document number(s) of the earlier application(s) in accordance with Art. 76 EPC:
**20730955.0 / 3 972 599**

(71) Applicant: **Ardelyx, Inc.**
**Fremont, California 94555 (US)**

(72) Inventor: **KING, Andrew**
**Fremont, California, 94555 (US)**

(74) Representative: **Grund, Martin et al**
**Grund Intellectual Property Group**
**Patentanwälte und Solicitor PartG mbB**
**Steinsdorfstraße 2**
**80538 München (DE)**

Remarks:
•The complete document including Reference Table(s) and the Sequence Listing(s) can be downloaded from the EPO website
•This application was filed on 10-10-2025 as a divisional application to the application mentioned under INID code 62.
•Claims filed after the date of filing of the application (Rule 68(4) EPC).

(54) **METHODS FOR INHIBITING PHOSPHATE TRANSPORT**

(57) The present invention relates to methods for lowering serum phosphate in a mammal comprising administering an epithelial phosphate transport inhibitor, such as an NHE3 inhibitor, in combination with a phosphate binder as well as pharmaceutical compositions comprising such epithelial phosphate transport inhibitors and phosphate binders.

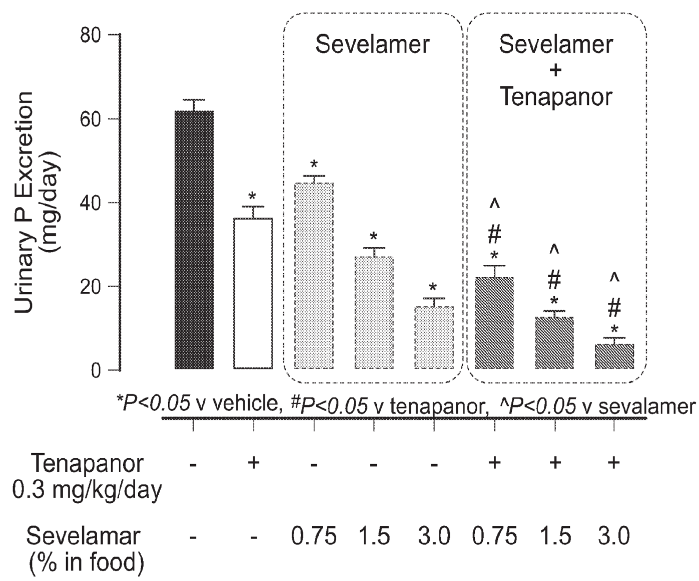


FIGURE 1



EP 4 706 758 A2

(52) Cooperative Patent Classification (CPC): (Cont.)

C-Sets
**A61K 31/4725, A61K 2300/00;**
**A61K 31/785, A61K 2300/00**

## Description

### CROSS-REFERENCE TO RELATED APPLICATION

This application claims priority to United States Provisional Application Number 62/851,099 that was filed on May 21, 2019 and claims priority to United States Provisional Application Number 62/852,299 that was filed on May 23, 2019. The entire contents of these applications referenced above are hereby incorporated by reference herein.

### FIELD

The present invention relates to the use of epithelial phosphate transport inhibitors in combination with phosphate binders in lowering serum phosphate levels or preventing serum phosphate elevation.

### BACKGROUND

Serum phosphate levels are normally maintained within a narrow physiologic range, principally through the regulation of renal phosphate excretion. Consequently, hyperphosphatemia is common in end stage renal disease (ESRD) patients on dialysis with impaired or absent urinary phosphate excretion. Intestinal phosphate absorption is linearly dependent on the phosphate concentration gradient and does not saturate even at high luminal phosphate concentrations. Furthermore, despite impaired renal phosphate excretory capacity in ESRD patients, intestinal phosphate absorption in haemodialysis patients is similar to healthy individuals, and is increased further by vitamin D therapy, which is commonly used in ESRD to manage secondary hyperparathyroidism. Therefore, sustained intestinal phosphate absorption in the face of impaired renal phosphate excretion, predicts the observed high prevalence of hyperphosphatemia in ESRD patients.

Tenapanor is a first-in-class, minimally-absorbed, small-molecule inhibitor of the sodium/hydrogen exchanger isoform 3 (NHE3) that acts locally in the gastrointestinal tract to inhibit intestinal phosphate absorption and has been shown to significantly lower serum phosphate levels in haemodialysis patients. NHE3 is expressed on the luminal surface throughout the small intestine and proximal colon and functions as a transporter to import luminal sodium in exchange for a cellular proton. The mechanism by which inhibition of NHE3 by tenapanor reduces intestinal phosphate absorption is by decreasing paracellular phosphate permeability has been reported by King et al (Sci Transl Med. 2018 Aug 29;10(456)); a result of intracellular proton retention to modulate the tight junction to increase transepithelial electrical resistance.

Paracellular phosphate flux through tight junction complexes, driven by the electrochemical phosphate gradient, is quantitatively the most important mechanism of intestinal phosphate absorption under typical conditions of phosphate availability. This was confirmed in a recent study showing no impact of deletion of the dominant sodium-dependent phosphate transporter, NaPi2b (SLC34A2), on intestinal phosphate absorption in mice under physiological luminal phosphate concentrations. Paracellular phosphate flux in the intestine is determined by the combination of the prevailing electrochemical phosphate gradient and the concurrent paracellular phosphate permeability.

The current medical management of hyperphosphatemia aims at reducing intestinal phosphate absorption by combining dietary phosphate restriction with administration of oral phosphate binders. Restricting dietary phosphate intake can modestly reduce the severity of hyperphosphatemia, although adherence is challenging and typically poor, at least in part due to the widespread use of high phosphate content additives in processed foods. Phosphate binders physically sequester dietary phosphate rendering it unavailable for absorption and can effectively lower serum phosphate; however, many ESRD patients fail to maintain target range serum phosphate levels, with non-adherence to phosphate binder use as a result of the high required bill burden a contributing factor. Poor serum phosphate control has significant clinical implications, as elevated serum phosphate concentrations are associated with poor patient outcomes including all-cause mortality, cardiovascular events, left ventricular hypertrophy and CKD progression in those patients not already on dialysis. Therefore, additional therapeutic approaches are required to optimize serum phosphate management in hyperphosphatemic patients.

Accordingly, it would be desirable to control serum phosphate in CKD and ESRD patients using a reduced amount of phosphate binder.

### SUMMARY

In an aspect of the present invention, there is provided a method for treating hyperphosphatemia in a patient comprising administering an effective amount of an epithelial phosphate transport inhibitor in combination with a phosphate binder, wherein the amount of the phosphate binder administered is less than the amount that would be administered as a single agent.

In another aspect of the invention, there is provided a pharmaceutical composition comprising an epithelial

phosphate transport inhibitor and a phosphate binder, wherein the amount of the phosphate binder is less than the amount that would be administered as a single agent.

## DESCRIPTION OF THE FIGURES

**Figures 1** shows the effect of phosphate binder, sevelamer, with and without NHE3 inhibitor, tenapanor (also referred to as Cp002), on urinary phosphate excretion.

**Figure 2** shows effect of tenapanor (Cpd 002) treatment on plasma phosphorus in a rat model of uremia-associated vascular calcification.

**Figures 3A and B** show that administration of tenapanor (Cpd 002) reduced both urine phosphorus mass relative to the vehicle-only control in rats. Increasing dosages of tenapanor also significantly reduced urine phosphorus mass relative to 48 mg/kg Renvela®.

**Figure 4** shows 24-hour urinary phosphorous excretion in rats treated with tenapanor, sevelamer or combination of tenapanor and sevelamer over the final 4-treatment days (A) with the final treatment day shown separately for clarity (B), along with 24-hour urinary phosphorous excretion normalized to daily phosphorous intake over the final 4-treatment days (C) with the final treatment day shown separately (D) for clarity. Data are shown as mean ± SEM. * = significant from vehicle control, **** = $p < 0.0001$, $^{\#}$ = significant from tenapanor alone, $^{\#\#}$ = $p < 0.01$; $^{\#\#\#\#}$ = $p < 0.0001$, $^{\$}$ = significant from equivalent dose of sevelamer alone, $^{\$}$ = $p < 0.05$; $^{\$\$}$ = $p < 0.01$; $^{\$\$\$\$}$ = $p < 0.0001$

## DETAILED DESCRIPTION

The present invention relates to the discovery that phosphate uptake from the gastrointestinal tract is inhibited in a synergistic manner with a phosphate binder in combination with an epithelial phosphate transport inhibitor i.e. a greater reduction in phosphate absorption with the combination than either agent alone. It was observed that epithelial phosphate transport is predominantly by way of epithelial tight junctions and is controlled by epithelial cell pH. When the cells were acidified, phosphate was transport was inhibited. The phenomenon was observed regardless of the mechanism by which the epithelial cells were acidified, for example, when treated with a NHE3 inhibitor, guanylate cyclase C receptor (GC-C) agonist. Other targets which may be modulated to acidify epithelial cells are, P2Y agonists, adenosine A2b receptor agonists, soluble guanylate cyclase agonists, adenylate cyclase receptor agonists, imidazoline-1 receptor agonists, cholinergic agonists, prostaglandin EP4 receptor agonists, dopamine D1 agonists, melatonin receptor agonists, 5HT4 agonists, atrial natriuretic peptide receptor agonists, carbonic anhydrase inhibitors, phosphodiesterase inhibitors, and Down-Regulated in Adenoma (DRA or SLC26A3) agonists.

Accordingly, in an aspect of the present invention there is provided a method for lowering serum phosphate in a patient comprising administering an effective amount of an epithelial phosphate transport inhibitor in combination with a phosphate binder, wherein the amount of the phosphate binder administered is less than the amount that would be administered as a single agent. In an embodiment, there is provided a method for treating hyperphosphatemia in a patient in need thereof, comprising administering to said patient an epithelial phosphate transport inhibitor and a phosphate binder, wherein the amount of the phosphate binder is less than the amount that would be required to lower serum phosphate in a patient as a single agent. In an embodiment, there is a method for preventing phosphate uptake from the gastrointestinal lumen of a patient, comprising administering to said patient an epithelial phosphate transport inhibitor and a phosphate binder, wherein the amount of the phosphate binder is less than the amount that would be required to lower serum phosphate in a patient as a single agent.

In another aspect of the present invention, there is provided a composition comprising an epithelial phosphate transport inhibitor and a phosphate binder, wherein the amount of the phosphate binder is less than the amount that would be required to lower serum phosphate in a patient as a single agent. In an embodiment, there is provided the manufacture of a medicament for lowering serum phosphate in a patient, said medicament comprising an epithelial phosphate transport inhibitor and a phosphate binder, wherein the amount of the phosphate binder is less than the amount that would be required to lower serum phosphate in a patient as a single agent. In another embodiment, there is provided the composition for use in lowering serum phosphate in a patient, said composition comprising an epithelial phosphate transport inhibitor and a phosphate binder, wherein the amount of the phosphate binder is less than the amount that would be required to lower serum phosphate in a patient as a single agent. In certain embodiments, the epithelial phosphate transport inhibitor and the phosphate binder are administered as part of a single pharmaceutical composition. In some embodiments, the epithelial phosphate transport inhibitor and the phosphate binder are administered as separate pharmaceutical compositions. In some embodiments, the individual agents pharmaceutical compositions are administered sequentially. In some embodiments, the individual pharmaceutical compositions are administered simultaneously. In an embodiment, the epithelial phosphate transport inhibitor is administered prior to the phosphate binder. In another embodiment, the epithelial

phosphate transport inhibitor is administered after the phosphate binder.

In certain embodiments the methods for inhibiting phosphate uptake in the gastrointestinal tract of a patient in need of phosphate lowering, comprising enterally administering to the patient a substantially systemically non-bioavailable epithelial transport inhibitor in combination with a reduced amount of phosphate binder in accordance with the present invention to inhibit transport of phosphate ions (Pi) therein upon administration to the patient in need thereof. In some embodiments, the method is selected from one or more of:

(a) a method for treating hyperphosphatemia, optionally postprandial hyperphosphatemia;
(b) a method for treating a renal disease, optionally chronic kidney disease (CKD) or end-stage renal disease (ESRD);
(c) a method for reducing serum creatinine levels;
(d) a method for treating proteinuria;
(e) a method for delaying time to renal replacement therapy (RRT), optionally dialysis;
(f) a method for reducing FGF23 levels;
(g) a method for reducing the hyperphosphatemic effect of active vitamin D;
(h) a method for attenuating hyperparathyroidism, optionally secondary hyperparathyroidism;
(i) a method for reducing serum parathyroid hormone (PTH)
(j) a method for reducing inderdialytic weight gain (IDWG);
(k) a method for improving endothelial dysfunction, optionally induced by postprandial serum phosphate;
(l) a method for reducing vascular calcification, optionally intima-localized vascular calcification;
(m) a method for reducing urinary phosphorous;
(n) a method for normalizing serum phosphorus levels;
(o) a method for reducing phosphate burden in an elderly patient;
(p) a method for decreasing dietary phosphate uptake;
(q) a method for reducing renal hypertrophy;
(r) a method for reducing heart hypertrophy; and
(s) a method for treating obstructive sleep apnea.

In certain embodiments, the methods of the invention, or administration of compositions of the invention to the patient in need thereof, (a) reduces serum phosphate concentrations or levels to about 150% or less of normal serum phosphate levels, and/or (b) reduces uptake of dietary phosphorous by at least about 10% relative to an untreated state. In some embodiments, administration to the patient in need thereof reduces urinary phosphate concentrations or levels by at least about 10% relative to an untreated state. In certain embodiments, administration to the patient in need thereof increases phosphate levels in fecal excretion by at least about 10% relative to an untreated state.

In certain embodiments, the patient in need thereof has ESRD, and administration to the patient of compositions of the invention (a) reduces serum phosphate concentrations or levels to about 150% or less of normal serum phosphate levels, and (b) reduces inderdialytic weight gain (IDWG) by at least about 10% relative to an untreated state. In an embodiment, the ESRD patient is on dialysis.

In some embodiments, the patient in need thereof has CKD, and administration to the patient (a) reduces FGF23 levels and serum intact parathyroid hormone (iPTH) levels by at least about 10% relative to an untreated state, and (b) reduces blood pressure and proteinuria by at least about 10% relative to an untreated state. In an embodiment, the CKD patient is on dialysis.

In an embodiment, the method is one of:

(a) a method for treating hyperphosphatemia, optionally postprandial hyperphosphatemia;
(b) a method for treating a renal disease, optionally chronic kidney disease (CKD) or end-stage renal disease (ESRD);
(c) a method for reducing serum creatinine levels;
(d) a method for treating proteinuria;
(e) a method for delaying time to renal replacement therapy (RRT), optionally dialysis;
(f) a method for reducing FGF23 levels;
(g) a method for reducing the hyperphosphatemic effect of active vitamin D;
(h) a method for attenuating hyperparathyroidism, optionally secondary hyperparathyroidism;
(i) a method for reducing serum parathyroid hormone (PTH)
(j) a method for reducing inderdialytic weight gain (IDWG);
(k) a method for improving endothelial dysfunction, optionally induced by postprandial serum phosphate;
(l) a method for reducing vascular calcification, optionally intima-localized vascular calcification;
(m) a method for increasing urinary phosphorous;
(n) a method for normalizing serum phosphorus levels;
(o) a method for reducing phosphate burden in an elderly patient;

(p) a method for decreasing dietary phosphate uptake;
(q) a method for reducing renal hypertrophy;
(r) a method for reducing heart hypertrophy; and
(s) a method for treating obstructive sleep apnea.

PHOSPHATE BINDERS

In certain embodiments, the phosphate binder is selected from the group consisting of sevelamer (e.g., Renvela® (sevelamer carbonate), Renagel® (sevelamer hydrochloride)), lanthanum carbonate (e.g., Fosrenol®), calcium carbonate (e.g., Calcichew®, Titralac®, Tums®), calcium acetate (e.g. PhosLo®, Phosex®), calcium acetate/magnesium carbonate (e.g., Renepho®, OsvaRen®), MCI-196, ferric citrate (e.g., Zerenex™), magnesium iron hydroxycarbonate (e.g., Fermagate™), aluminum hydroxide (e.g., Alucaps®, Basaljel®), ferric citrate e.g. tetra ferric tricitrate (e.g. Auryxia®), sucroferric oxyhydroxide (e.g. Velphoro®), APS1585, SBR-759, and PA-21.

In an embodiment, the phosphate binder is sevelamer. In an embodiment the phosphate binder is sevelamer carbonate. In an embodiment, the phosphate binder is sevelamer hydrochloride. In an embodiment, the single agent amount of sevelamer is about 800-1600mg once or twice per day. In an embodiment, the composition comprises about 30mg of tenapanor and about 200mg of sevelamer. In an embodiment, the composition comprises about 30mg of tenapanor and about 400mg of sevelamer. In an embodiment, the composition comprises about 30mg of tenapanor and about 800mg of sevelamer. In an embodiment, the composition comprises about 30mg of tenapanor and about 1,200mg of sevelamer. In an embodiment the amount of sevelamer is about 100-200mg.

In an embodiment the amount of sevelamer is about 100-200mg. In an embodiment the amount of sevelamer is about 200-300mg. In an embodiment the amount of sevelamer is about 300-400mg. In an embodiment the amount of sevelamer is about 400-500mg. In an embodiment the amount of sevelamer is about 500-600mg. In an embodiment the amount of sevelamer is about 600-700mg. In an embodiment the amount of sevelamer is about 700-800mg.

In an embodiment, the phosphate binder is ferric citrate e.g. tetra ferric tricitrate (e.g. Auryxia®). The single agent amount of Auryxia is up to 12 of 210mg tablets per day and an average of 8-9 tablets per day to achieve serum phosphate levels of 3.5-5.5 mg/dL. The starting dose of Auryxia® is 2-3 of 210mg tablets per day. In an embodiment the amount of Auryxia® administered per dose according to the method of invention or present in compositions of the invention is about 10-25mg. In an embodiment the amount of Auryxia® administered per dose according to the method of present invention or present in compositions of the invention is about 25-50mg. In an embodiment the amount of Auryxia® administered per dose according to the method of present invention or present in compositions of the invention is about 50-75mg. In an embodiment the amount of Auryxia® administered per dose according to the method of present invention or present in compositions of the invention is about 75-100mg. In an embodiment the amount of Auryxia® administered per dose according to the method of present invention or present in compositions of the invention is about 100-125mg. In an embodiment the amount of Auryxia® administered per dose according to the method of present invention or present in compositions of the invention is about 125-150mg. In an embodiment the amount of Auryxia® administered per dose according to the method of present invention or present in compositions of the invention is about 150-175mg. In an embodiment the amount of Auryxia® administered per dose according to the method of present invention or present in compositions of the invention is about 175-200mg.

In an embodiment, the phosphate binder is sucroferric oxyhydroxide (e.g. Velphoro®). The starting dose of Velphoro® is one 500mg tablet. The average maintenance dose of Velphoro® is one 500mg tablet three to four times per day. In an embodiment the amount of Velphoro® administered per dose according to the method of present invention or present in compositions of the invention is about 25-50mg. In an embodiment the amount of Velphoro® administered per dose according to the method of present invention or present in compositions of the invention is about 50-100mg. In an embodiment the amount of Velphoro® administered per dose according to the method of present invention or present in compositions of the invention is about 100-150mg. In an embodiment the amount of Velphoro® administered per dose according to the method of present invention or present in compositions of the invention is about 200-50mg. In an embodiment the amount of Velphoro® administered per dose according to the method of present invention or present in compositions of the invention is about 250-300mg. In an embodiment the amount of Velphoro® administered per dose according to the method of present invention or present in compositions of the invention is about 300-350mg. In an embodiment the amount of Velphoro® administered per dose according to the method of present invention or present in compositions of the invention is about 350-400mg.

In an embodiment, the amount of phosphate binder administered in combination with the epithelial phosphate transport inhibitor, or present in a composition of the invention, is 90-95% of the amount that would be administered as a single agent. In an embodiment, the amount of phosphate binder administered in combination with the epithelial phosphate transport inhibitor, or present in a composition of the invention, is 85-90% of the amount that would be administered as a single agent. In an embodiment, the amount of phosphate binder administered in combination with the epithelial phosphate transport inhibitor, or is present in a composition of the invention, is 80-85% of the amount that would

be administered as a single agent. In an embodiment, the amount of phosphate binder administered in combination with the epithelial phosphate transport inhibitor, or present in a composition of the invention, is 75-80% of the amount that would be administered as a single agent. In an embodiment, the amount of phosphate binder administered in combination with the epithelial phosphate transport inhibitor, or present in a composition of the invention, is 70-75% of the amount that would be administered as a single agent. In an embodiment, the amount of phosphate binder administered in combination with the epithelial phosphate transport inhibitor, or present in a composition of the invention, is 65-70% of the amount that would be administered as a single agent. In an embodiment, the amount of phosphate binder administered in combination with the epithelial phosphate transport inhibitor, or present in a composition of the invention, is 60-65% of the amount that would be administered as a single agent. In an embodiment, the amount of phosphate binder administered in combination with the epithelial phosphate transport inhibitor, or present in a composition of the invention, is 55-60% of the amount that would be administered as a single agent. In an embodiment, the amount of phosphate binder administered in combination with the epithelial phosphate transport inhibitor, or present in a composition of the invention, is 50-55% of the amount that would be administered as a single agent. In an embodiment, the amount of phosphate binder administered in combination with the epithelial phosphate transport inhibitor, or present in a composition of the invention, is 45-50% of the amount that would be administered as a single agent. In an embodiment, the amount of phosphate binder administered in combination with the epithelial phosphate transport inhibitor, or present in a composition of the invention, is 40-45% of the amount that would be administered as a single agent. In an embodiment, the amount of phosphate binder administered in combination with the epithelial phosphate transport inhibitor, or present in a composition of the invention, is 35-40% of the amount that would be administered as a single agent. In an embodiment, the amount of phosphate binder administered in combination with the epithelial phosphate transport inhibitor, or is present in a composition of the invention, is 30-35% of the amount that would be administered as a single agent. In an embodiment, the amount of phosphate binder administered in combination with the epithelial phosphate transport inhibitor is, or present in a composition of the invention, is 25-30% of the amount that would be administered as a single agent. In an embodiment, the amount of phosphate binder administered in combination with the epithelial phosphate transport inhibitor is, or present in a composition of the invention, is 20-25% of the amount that would be administer as a single agent. In an embodiment, the amount of phosphate binder administered in combination with the epithelial phosphate transport inhibitor, or present in a composition of the invention, is 15-20% of the amount that would be administered as a single agent. In an embodiment, the amount of phosphate binder administered in combination with the epithelial phosphate transport inhibitor, or present in a composition of the invention, is 10-15% of the amount that would be administered as a single agent. In an embodiment, the amount of phosphate binder administered in combination with the epithelial phosphate transport inhibitor, or present in a composition of the invention, is about 5-10% of the amount that would be administered as a single agent.

In an embodiment, the amount of phosphate binder administered in combination with the epithelial phosphate transport inhibitor, or present in a composition of the invention, is about 95% of the amount that would be administered as a single agent. In an embodiment, the amount of phosphate binder administered in combination with the epithelial phosphate transport inhibitor, or present in a composition of the invention, is about 90% of the amount that would be administered as a single agent. In an embodiment, the amount of phosphate binder administered in combination with the epithelial phosphate transport inhibitor, or present in a composition of the invention, is about 85% of the amount that would be administered as a single agent. In an embodiment, the amount of phosphate binder administered in combination with the epithelial phosphate transport inhibitor, or present in a composition of the invention, is about 80% of the amount that would be administered as a single agent. In an embodiment, the amount of phosphate binder administered in combination with the epithelial phosphate transport inhibitor, or present in a composition of the invention, is about 75% of the amount that would be administered as a single agent. In an embodiment, the amount of phosphate binder administered in combination with the epithelial phosphate transport inhibitor, or present in a composition of the invention, is about 70% of the amount that would be administered as a single agent. In an embodiment, the amount of phosphate binder administered in combination with the epithelial phosphate transport inhibitor, or present in a composition of the invention, is about 65% of the amount that would be administered as a single agent. In an embodiment, the amount of phosphate binder administered in combination with the epithelial phosphate transport inhibitor, or present in a composition of the invention, is about 60% of the amount that would be administered as a single agent. In an embodiment, the amount of phosphate binder administered in combination with the epithelial phosphate transport inhibitor, or present in a composition of the invention, is about 55% of the amount that would be administered as a single agent. In an embodiment, the amount of phosphate binder administered in combination with the epithelial phosphate transport inhibitor, or present in a composition of the invention, is about 50% of the amount that would be administered as a single agent. In an embodiment, the amount of phosphate binder administered in combination with the epithelial phosphate transport inhibitor, or present in a composition of the invention, is about 45% of the amount that would be administered as a single agent. In an embodiment, the amount of phosphate binder administered in combination with the epithelial phosphate transport inhibitor, or present in a composition of the invention, is about 40% of the amount that would be administered as a single agent. In an embodiment, the amount of phosphate binder administered in combination with the epithelial phosphate transport inhibitor, or present in a composition of the invention, is about 35% of the amount that would be administered as a single agent. In an embodiment, the amount of phosphate binder administered

in combination with the epithelial phosphate transport inhibitor, or present in a composition of the invention, is about 30% of the amount that would be administered as a single agent. In an embodiment, the amount of phosphate binder administered in combination with the epithelial phosphate transport inhibitor, or present in a composition of the invention, is about 25% of the amount that would be administer as a single agent. In an embodiment, the amount of phosphate binder administered in combination with the epithelial phosphate transport inhibitor, or present in a composition of the invention, is about 20% of the amount that would be administered as a single agent. In an embodiment, the amount of phosphate binder administered in combination with the epithelial phosphate transport inhibitor, or present in a composition of the invention, is about 20% of the amount that would be administered as a single agent. In an embodiment, the amount of phosphate binder administered in combination with the epithelial phosphate transport inhibitor, or present in a composition of the invention, is about 15% of the amount that would be administered as a single agent. In an embodiment, the amount of phosphate binder administered in combination with the epithelial phosphate transport inhibitor, or present in a composition of the invention, is about 10% of the amount that would be administered as a single agent.

EPITHELIAL PHOSPHATE TRANSPORT INHIBITORS

Epithelial phosphate transport inhibitors used in methods and compositions of the invention are any compound capable of lowering the pH of epithelial cells. In an embodiment, the epithelial phosphate transport inhibitor is selected from the group consisting of NHE3 inhibitors, guanylate cyclase C receptor (GC-C) agonists, P2Y agonists, adenosine A2b receptor agonists, soluble guanylate cyclase agonists, adenylate cyclase receptor agonists, imidazoline-1 receptor agonists, cholinergic agonists, prostaglandin EP4 receptor agonists, dopamine D1 agonists, melatonin receptor agonists, 5HT4 agonists, atrial natriuretic peptide receptor agonists, carbonic anhydrase inhibitors, phosphodiesterase inhibitors, and Down-Regulated in Adenoma (DRA or SLC26A3) agonists.

NHE3 Inhibitors

In an embodiment, the epithelial phosphate transport inhibitor is an NHE3 inhibitor. In a particular embodiment, the NHE3 inhibitor has a structure of Formula (I) or (IX):

NHE-Z (I)

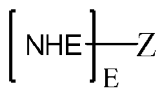

(IX)

wherein:

NHE is a NHE-binding small molecule that comprises (i) a hetero-atom containing moiety, and (ii) a cyclic or heterocyclic scaffold or support moiety bound directly or indirectly thereto, the heteroatom-containing moiety being selected from a substituted guanidinyl moiety and a substituted heterocyclic moiety, which may optionally be fused with the scaffold or support moiety to form a fused bicyclic structure; and,

Z is a moiety having at least one site thereon for attachment to the NHE-binding small molecule, the resulting NHE-Z molecule possessing overall physicochemical properties that render it substantially impermeable or substantially systemically non-bioavailable; and,

E is an integer having a value of 1 or more.

In some embodiments, the scaffold of the NHE-binding small molecule is bound to the moiety, Z, the compound having the structure of Formula (II):

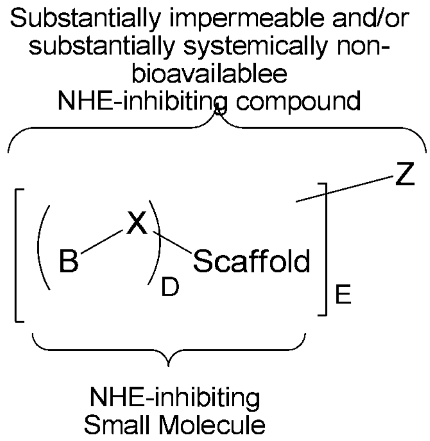


(II)

wherein:

Z is a Core having one or more sites thereon for attachment to one or more NHE-binding small molecules, the resulting NHE-Z molecule possessing overall physicochemical properties that render it substantially impermeable or substantially systemically non-bioavailable;

B is the heteroatom-containing moiety of the NHE-binding small molecule, and is selected from a substituted guanidinyl moiety and a substituted heterocyclic moiety, which may optionally be fused with the Scaffold moiety to form a fused, bicyclic structure;

Scaffold is the cyclic or heterocyclic scaffold or support moiety of the NHE-binding small molecule, which is bound directly or indirectly to heteroatom-containing moiety, B, and which is optionally substituted with one or more additionally hydrocarbyl or heterohydrocarbyl moieties;

X is a bond or a spacer moiety selected from a group consisting of substituted or unsubstituted hydrocarbyl or heterohydrocarbyl moieties, and in particular substituted or unsubstituted $C_{1-7}$ hydrocarbyl or heterohydrocarbyl, and substituted or unsubstituted, saturated or unsaturated, cyclic or heterocyclic moieties, which links B and the Scaffold; and

D and E are integers, each independently having a value of 1 or more.

In some embodiments, the compound is an oligomer, dendrimer or polymer, and further wherein Z is a Core moiety having two or more sites thereon for attachment to multiple NHE-binding small molecules, either directly or indirectly through a linking moiety, L, the compound having the structure of Formula (X):

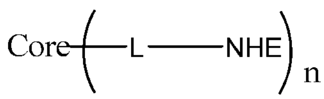

(X)

wherein L is a bond or linker connecting the Core to the NHE-binding small molecule, and n is an integer of 2 or more, and further wherein each NHE-binding small molecule may be the same or differ from the others.

In some embodiments, the NHE-binding small molecule has the structure of Formula (IV):

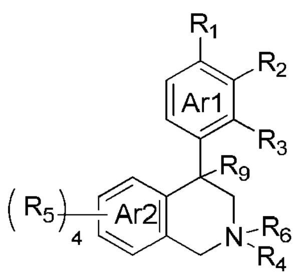

(IV)

or a stereoisomer, prodrug or pharmaceutically acceptable salt thereof,
wherein:

each $R_1$, $R_2$, $R_3$, $R_5$ and $R_9$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or a bond linking the NHE-binding small molecule to L, provided at least one is a bond linking the NHE-binding small molecule to L;
$R_4$ is selected from H, $C_1$-$C_7$ alkyl, or a bond linking the NHE-binding small molecule to L;
$R_6$ is absent or selected from H and $C_1$-$C_7$ alkyl; and
Ar1 and Ar2 independently represent an aromatic ring or a heteroaromatic ring.

In certain embodiments, the NHE-binding small molecule has the following structure:

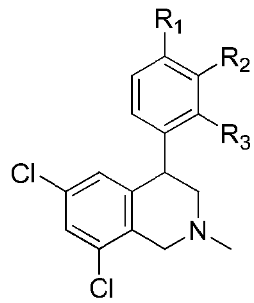

or a stereoisomer, prodrug or pharmaceutically acceptable salt thereof,
wherein:
each $R_1$, $R_2$ and $R_3$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or a bond linking the NHE-binding small molecule to L, provided at least one is a bond linking the NHE-binding small molecule to L.

In some embodiments, the NHE-binding small molecule has one of the following structures:

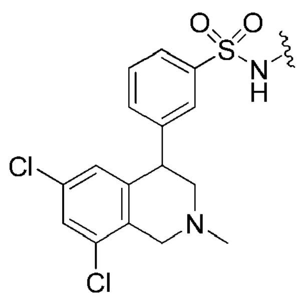

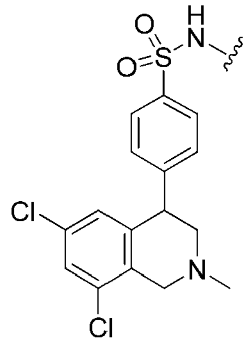

or

or a stereoisomer, prodrug or pharmaceutically acceptable salt thereof. In certain embodiments, L is a polyalkylene glycol linker. In certain embodiments, L is a polyethylene glycol linker. In some embodiments, n is 2.
In certain embodiments, the Core has the following structure:

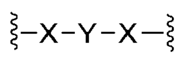

wherein:

X is selected from the group consisting of a bond, -O-, -NH-, -S-, $C_{1-6}$alkylene, -NHC(=O)-, -C(=O)NH-, - NHC(=O)NH-, $-SO_2NH-$, and $-NHSO_2-$;
Y is selected from the group consisting of a bond, optionally substituted $C_{1-8}$alkylene, optionally substituted aryl, optionally substituted heteroaryl, a polyethylene glycol linker, $-(CH_2)_{1-6}O(CH_2)_{1-6}-$ and $-(CH_2)_{1-6}NY_1(CH_2)_{1-6}-$; and

$Y_1$ is selected from the group consisting of hydrogen, optionally substituted $C_{1-8}$alkyl, optionally substituted aryl or optionally substituted heteroaryl.

In some embodiments, the Core is selected from the group consisting of:

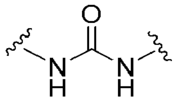
;

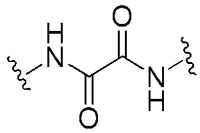
;

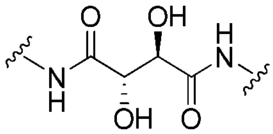
;

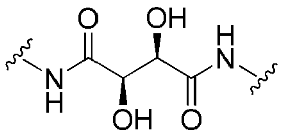
;

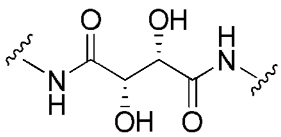
;

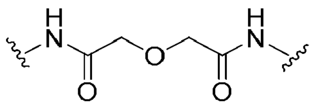
;

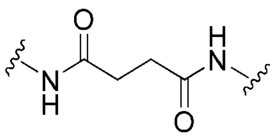
;

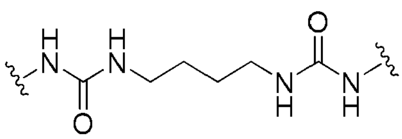
;

and

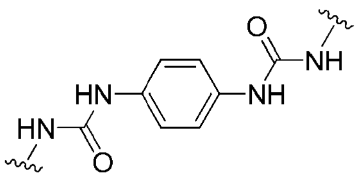
.

wherein: L is a bond or a linking moiety; NHE is a NHE-binding small molecule; and n is a non-zero integer.

In some embodiments, the NHE3 inhibitor has the following structure of Formula (I-H):

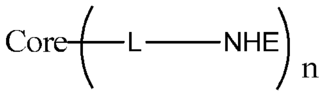

(I-H)

or a stereoisomer, prodrug or pharmaceutically acceptable salt thereof, wherein:

(a) n is an integer of 2 or more;
(b) Core is a Core moiety having two or more sites thereon for attachment to two or more NHE-binding small molecule moieties;
(c) L is a bond or linker connecting the Core moiety to the two or more NHE-binding small molecule moieties; and
(d) NHE is a NHE-binding small molecule moiety having the following structure of Formula (XI-H):

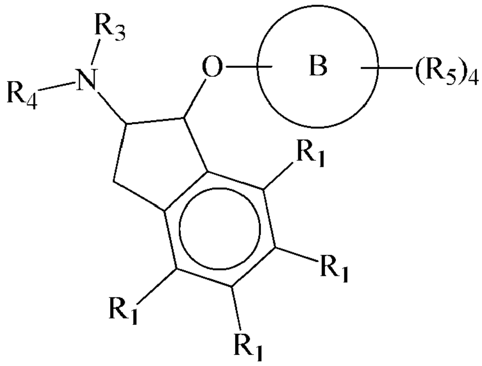

(XI-H)

wherein:

B is selected from the group consisting of aryl and heterocyclyl;
each $R_5$ is independently selected from the group consisting of hydrogen, halogen, optionally substituted $C_{1-4}$alkyl, optionally substituted $C_{1-4}$alkoxy, optionally substituted $C_{1-4}$thioalkyl, optionally substituted heterocyclyl, optionally substituted heterocyclylalkyl, optionally substituted aryl, optionally substituted heteroaryl, hydroxyl, oxo, cyano, nitro, $-NR_7R_8$, $-NR_7C(=O)R_8$, $-NR_7C(=O)OR_8$, $-NR_7C(=O)NR_8R_9$, $-NR_7SO_2R_8$, $-NR_7S(O)_2NR_8R_9$, $-C(=O)OR_7$, $-C(=O)R_7$,
$-C(=O)NR_7R_8$, $-S(O)_{1-2}R_7$, and $-SO_2NR_7R_8$, wherein $R_7$, $R_8$, and $R_9$ are independently selected from the group consisting of hydrogen, $C_{1-4}$alkyl, or a bond linking the NHE-binding small molecule moiety to L, provided at least one is a bond linking the NHE-binding small molecule moiety to L;
$R_3$ and $R_4$ are independently selected from the group consisting of hydrogen, optionally substituted $C_{1-4}$alkyl, optionally substituted cycloalkyl, optionally substituted cycloalkylalkyl, optionally substituted aryl, optionally substituted aralkyl, optionally substituted heterocyclyl and optionally substituted heteroaryl; or
$R_3$ and $R_4$ form together with the nitrogen to which they are bonded an optionally substituted 4-8 membered heterocyclyl; and
each $R_1$ is independently selected from the group consisting of hydrogen, halogen, optionally substituted $C_{1-6}$alkyl and optionally substituted $C_{1-6}$alkoxy. In some embodiments, n is 2. In certain embodiments, L is a polyalkylene glycol linker. In certain embodiments, L is a polyethylene glycol linker.

In certain embodiments, the Core has the following structure:



wherein:

X is selected from the group consisting of a bond, -O-, -NH-, -S-, $C_{1-6}$alkylene, -NHC(=O)-, -C(=O)NH-, -NHC(=O)NH-, -$SO_2$NH-, and -$NHSO_2$-;

Y is selected from the group consisting of a bond, optionally substituted $C_{1-8}$alkylene, optionally substituted aryl, optionally substituted heteroaryl, a polyethylene glycol linker, $-(CH_2)_{1-6}O(CH_2)_{1-6}-$ and $-(CH_2)_{1-6}NY_1(CH_2)_{1-6}-$; and

$Y_1$ is selected from the group consisting of hydrogen, optionally substituted $C_{1-8}$alkyl, optionally substituted aryl or optionally substituted heteroaryl.

In some embodiments, the Core is selected from the group consisting of

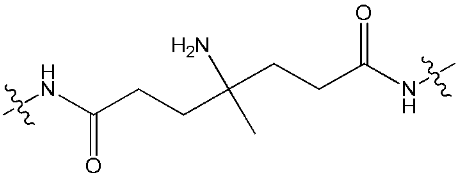,

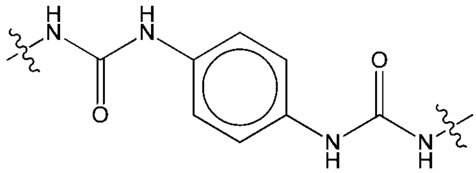,

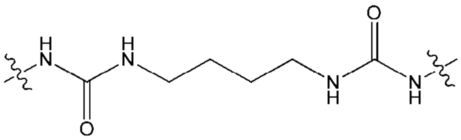,

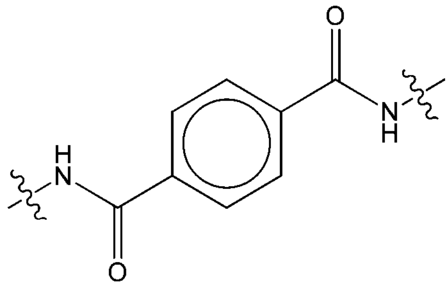,

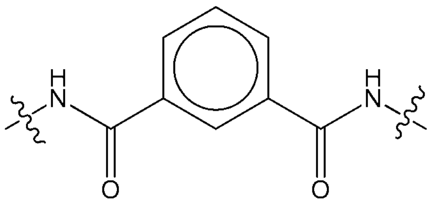,

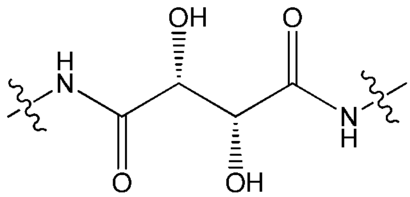,

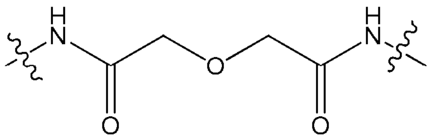,

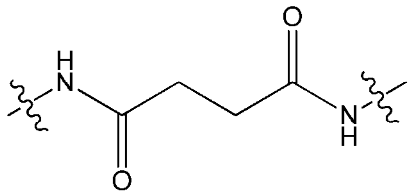,

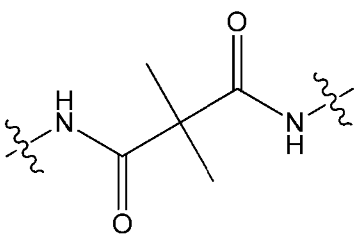, and

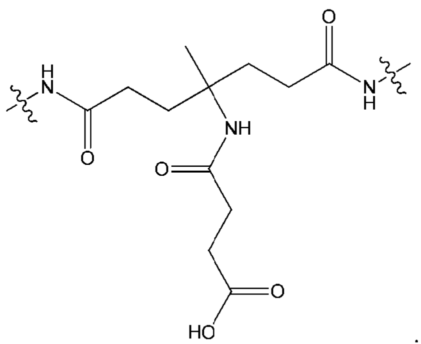.

In certain embodiments, the NHE-binding small molecule moiety has the following structure of Formula (XII-H):

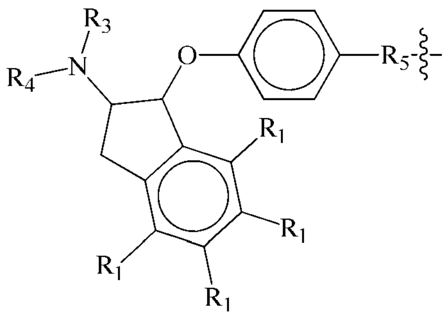

(XII-H)

wherein:

each $R_3$ and $R_4$ are independently selected from the group consisting of hydrogen and optionally substituted $C_{1-4}$alkyl, or $R_3$ and $R_4$, taken together with the nitrogen to which they are bonded, form an optionally substituted 4-8 membered heterocyclyl;
each $R_1$ is independently selected from the group consisting of hydrogen, halogen, $C_{1-6}$alkyl, and $C_{1-6}$haloalkyl; and
$R_5$ is selected from the group consisting of $-SO_2-NR_7-$ and -NHC(=O)NH-, wherein $R_7$ is hydrogen or $C_{1-4}$alkyl.

In some embodiments, $R_3$ and $R_4$, taken together with the nitrogen to which they are bonded, form an optionally substituted 5 or 6 membered heterocyclyl. In certain embodiments, the optionally substituted 5 or 6 membered heterocyclyl is pyrrolidinyl or piperidinyl. In certain embodiments, the optionally substituted 5 or 6 membered heterocyclyl is pyrrolidinyl or piperidinyl, each substituted with at least one amino or hydroxyl. In some embodiments, $R_3$ and $R_4$ are independently $C_{1-4}$alkyl. In certain embodiments, $R_3$ and $R_4$ are methyl. In some embodiments, each $R_1$ is independently selected from the group consisting of hydrogen or halogen. In certain embodiments, each $R_1$ is independently selected from the group consisting of hydrogen, F and Cl.

In certain embodiments, the NHE3 inhibitor has the following structure of Formula (I-I):

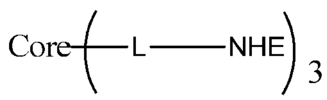

(I-I)

or a stereoisomer, prodrug or pharmaceutically acceptable salt thereof,
wherein:

(a) NHE is a NHE-binding small molecule moiety having the following structure of Formula (A-I):

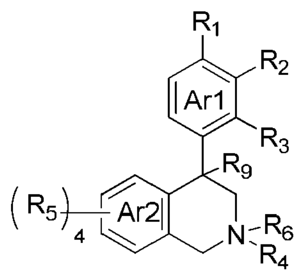

(A-I)

wherein:

each $R_1$, $R_2$, $R_3$, $R_5$ and $R_9$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H, $C_{1-6}$alkyl, $-C_{1-6}$alkyl-OH or a bond linking the NHE-binding small molecule to L, provided at least one is a bond linking the NHE-binding small molecule to L;

$R_4$ is selected from H, $C_1$-$C_7$ alkyl, or a bond linking the NHE-binding small molecule to L;
$R_6$ is absent or selected from H and $C_1$-$C_7$ alkyl; and
Ar1 and Ar2 independently represent an aromatic ring or a heteroaromatic ring;

(b) Core is a Core moiety having the following structure of Formula (B-I):

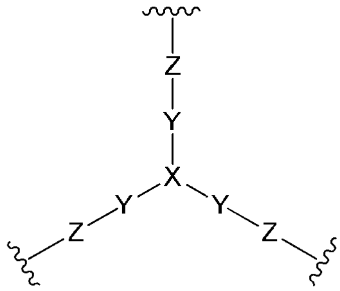

(B-I)

wherein:

X is selected from $C(X_1)$, N and $N(C_{1-6}alkyl)$;
$X_1$ is selected from hydrogen, optionally substituted alkyl, $-NX_aX_b$, $-NO_2$, $-NX_c-C(=O)-NX_c-X_a$, $-C(=O)NX_c-X_a$, $-NX_c-C(=O)-X_a$, $-NX_c-SO_2-X_a$, $-C(=O)-X_a$ and $-OX_a$,
each $X_a$ and $X_b$ are independently selected from hydrogen, optionally substituted alkyl, optionally substituted cycloalkyl, optionally substituted cycloalkylalkyl, optionally substituted heterocyclyl, optionally substituted heterocyclylalkyl, optionally substituted aryl, optionally substituted aralkyl, optionally substituted heteroaryl and optionally substituted heteroarylalkyl;
Y is $C_{1-6}$alkylene;
Z is selected from $-NZ_a-C(=O)-NZ_a-$, $-C(=O)NZ_a-$, $-NZ_a-C(=O)-$ and heteroaryl when X is $CX_1$;
Z is selected from $-NZ_a-C(=O)-NZ_a-$, $-NZ_a-C(=O)-$ and heteroaryl when X is N or $N(C_{1-6}alkyl)$; and
each $X_c$ and $Z_a$ is independently selected from hydrogen and $C_{1-6}$alkyl; and

(c) L is a bond or linker connecting the Core moiety to the NHE-binding small molecule moieties.

In some embodiments, the NHE-binding small molecule moiety has the following structure:

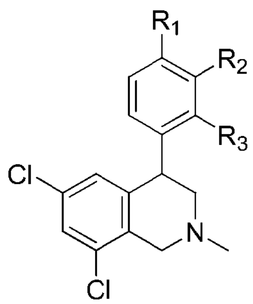

wherein:
each $R_1$, $R_2$ and $R_3$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H, $C_{1-6}$alkyl, $-C_{1-6}$alkyl-OH or a bond linking the NHE-binding small molecule to L, provided at least one is a bond linking the NHE-binding small molecule to L.
In some embodiments, the NHE-binding small molecule moiety has one of the following structures:

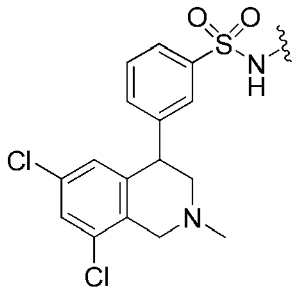

or

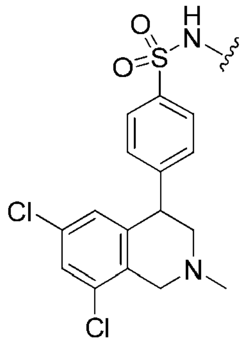

.

In some embodiments, L is a polyalkylene glycol linker. In certain embodiments, L is a polyethylene glycol linker. In some embodiments, X is C($X_1$). In some embodiments, each $X_c$ is hydrogen. In certain embodiments, X is N. In certain embodiments, each $Z_a$ is hydrogen.

In some embodiments, the NHE3 inhibitor has the structure of Formula (II):

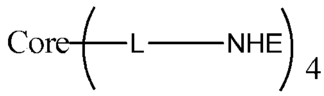

(II-I)

or a stereoisomer, prodrug or pharmaceutically acceptable salt thereof, wherein:

(a) NHE is a NHE-binding small molecule moiety having the structure of Formula (A-I):

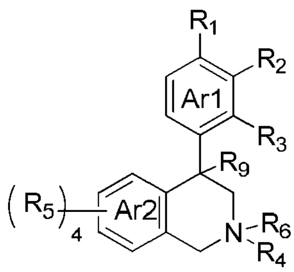

(A)

wherein:

each $R_1$, $R_2$, $R_3$, $R_5$ and $R_9$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H, $C_{1-8}$alkyl, $-C_{1-6}$alkyl-OH or a bond linking the NHE-binding small molecule to L, provided at least one is a bond linking the NHE-binding small molecule to L;

$R_4$ is selected from H, $C_1$-$C_7$ alkyl, or a bond linking the NHE-binding small molecule to L;

$R_6$ is absent or selected from H and $C_1$-$C_7$ alkyl; and

Ar1 and Ar2 independently represent an aromatic ring or a heteroaromatic ring;

(b) Core is a Core moiety having the following structure of Formula (C-I):

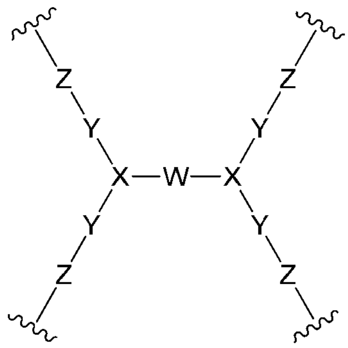

(C-I)

wherein:

W is selected from alkylene, polyalkylene glycol, -C(=O)-NH-(alkylene)-NH-C(=O)-, -C(=O)-NH-(polyalkylene glycol)-NH-C(=O)-, -C(=O)-(alkylene)-C(=O)-, -C(=O)-(polyalkylene glycol)-C(=O)- and cycloalkyl,
X is N;
Y is $C_{1-6}$alkylene;
Z is selected from $-NZ_a-C(=O)-NZ_a-$, $-C(=O)NZ_a-$, $-NZ_a-C(=O)-$ and heteroaryl;
each $Z_a$ is independently selected from hydrogen and $C_{1-6}$alkyl; and

(c) L is a bond or linker connecting the Core moiety to the NHE-binding small molecules.

In certain embodiments, the NHE-binding small molecule moiety has the following structure:

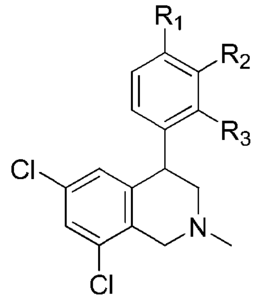

wherein:
each $R_1$, $R_2$ and $R_3$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H, $C_{1-6}$alkyl, $-C_{1-6}$alkyl-OH or a bond linking the NHE-binding small molecule to L, provided at least one is a bond linking the NHE-binding small molecule to L.

In certain embodiments, the NHE-binding small molecule moiety has one of the following structures:

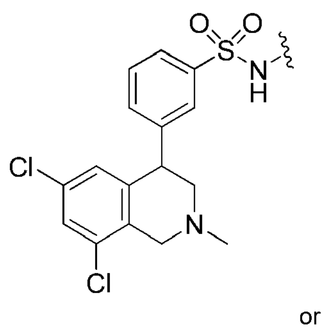

or

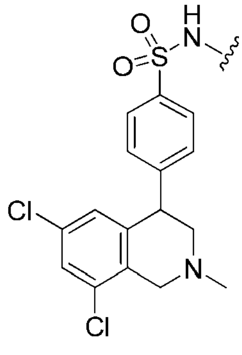

.

In an embodiment, the NHE3 inhibitor is one disclosed in WO2010078449. In embodiment, the NHE3 inhibitor is TP0469711. In an embodiment, the NHE3 inhibitor is:

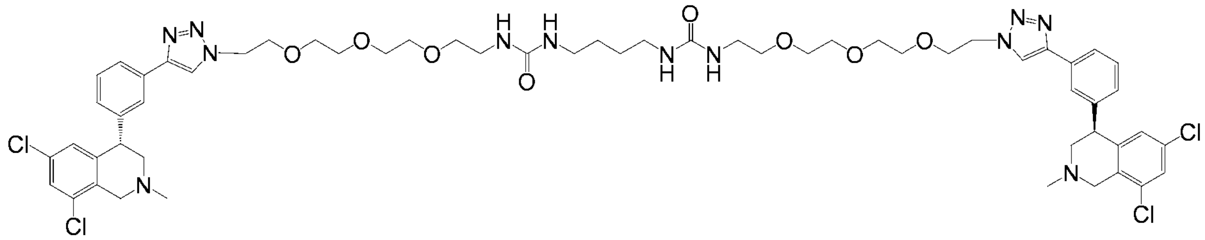

In an embodiment, the NHE3 inhibitor is:

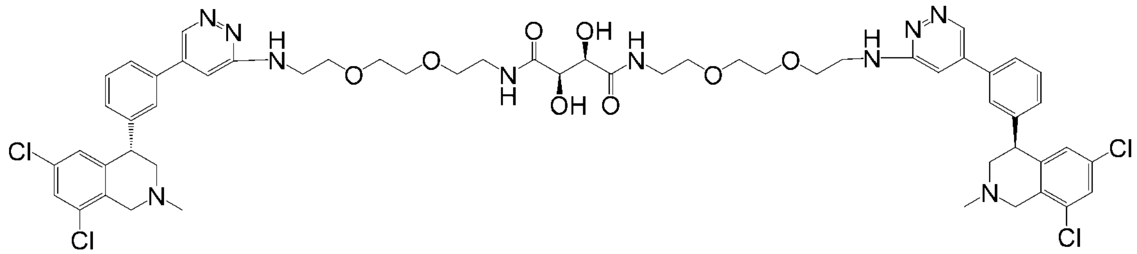

In particular embodiments, the NHE3 inhibitor has the structure:

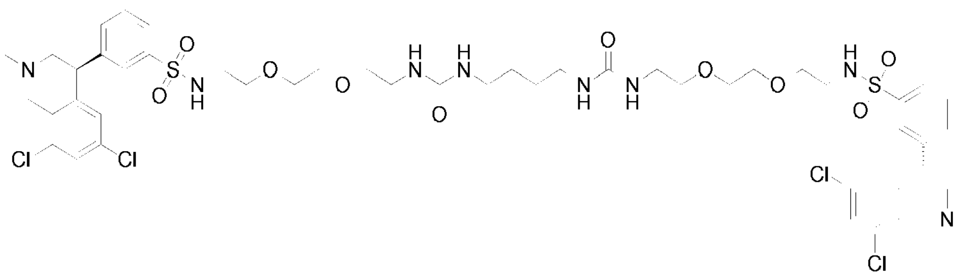

In an embodiment, the foregoing NHE3 inhibitor is the HCl salt. In an embodiment, the foregoing NHE3 inhibitor is a dihydrochloride salt.

In an embodiment, the NHE3 inhibitor is tenapanor. It will be appreciated that the amount of tenapanor administered may be specific to individual patients. Furthermore, when the NHE3 inhibitor is other than tenapanor, the amount administered may be different depending on several factors such as potency, stability and exposure to the NHE3 antiport in the colon. In an embodiment, the NHE3 inhibitor is administered in an amount to achieve NHE3 inhibition in a patient to approximately the same extent as tenapanor. In an embodiment, the amount of tenapanor administered in combination with the phosphate binder is 30mg. In an embodiment, tenapanor is administered twice per day in combination with the phosphate binder. In an embodiment, tenapanor is administered three times per day with meals in combination with the phosphate binder. In an embodiment, tenapanor is administered as three 10mg tablets in combination with the phosphate binder. In an embodiment, the tenapanor is administered twice per day and the phosphate binder three times per day. In an embodiment tenapanor is administered twice per day with food and the phosphate binder is administered three times per day when patient consumes a meal.

Substantially Systemically Non-Bioavailable NHE3 linhibitors:

Certain of the NHE3 inhibitors described herein are designed to be substantially active or localized in the gastrointestinal lumen of a human or animal subject. The term "gastrointestinal lumen" is used interchangeably herein with the term "lumen," to refer to the space or cavity within a gastrointestinal tract (GI tract, which can also be referred to as the gut), delimited by the apical membrane of GI epithelial cells of the subject. In some embodiments, the compounds are not absorbed through the layer of epithelial cells of the GI tract (also known as the GI epithelium). "Gastrointestinal mucosa" refers to the layer(s) of cells separating the gastrointestinal lumen from the rest of the body and includes gastric and intestinal mucosa, such as the mucosa of the small intestine. A "gastrointestinal epithelial cell" or a "gut epithelial cell" as

used herein refers to any epithelial cell on the surface of the gastrointestinal mucosa that faces the lumen of the gastrointestinal tract, including, for example, an epithelial cell of the stomach, an intestinal epithelial cell, a colonic epithelial cell, and the like.

"Substantially systemically non-bioavailable" and/or "substantially impermeable" as used herein (as well as variations thereof) generally refer to situations in which a statistically significant amount, and in some embodiments essentially all of the compound remains in the gastrointestinal lumen. For example, in accordance with one or more embodiments of the present disclosure, preferably at least about 60%, about 70%, about 75%, about 80%, about 85%, about 90%, about 95%, about 96%, about 97%, about 98%, about 99%, or even about 99.5%, of the compound remains in the gastrointestinal lumen. In such cases, localization to the gastrointestinal lumen refers to reducing net movement of a compound across a gastrointestinal layer of epithelial cells, for example, by way of both transcellular and paracellular transport, as well as by active and/or passive transport. The compound in such embodiments is hindered from net permeation of a layer of gastrointestinal epithelial cells in transcellular transport, for example, through an apical membrane of an epithelial cell of the small intestine. The compound in these embodiments is also hindered from net permeation through the "tight junctions" in paracellular transport between gastrointestinal epithelial cells lining the lumen.

In this regard it is to be noted that, in one particular embodiment, the NHE3 inhibitor compound is essentially not absorbed at all by the GI tract or gastrointestinal lumen. As used herein, the terms "substantially impermeable" or "substantially systemically non-bioavailable" includes embodiments wherein no detectable amount of absorption or permeation or systemic exposure of the compound is detected, using means generally known in the art.

In this regard it is to be further noted, however, that in alternative embodiments "substantially impermeable" or "substantially systemically non-bioavailable" provides or allows for some limited absorption in the GI tract, and more particularly the gut epithelium, to occur (*e.g.,* some detectable amount of absorption, such as for example at least about 0.1%, 0.5%, 1% or more and less than about 30%, 20%, 10%, 5%, etc., the range of absorption being for example between about 1% and 30%, or 5% and 20%, etc.); stated another way, "substantially impermeable" or "substantially systemically non-bioavailable" may refer to compounds that exhibit some detectable permeability to an epithelial layer of cells in the GI tract of less than about 20% of the administered compound (*e.g.*, less than about 15%, about 10%, or even about 5%, 4%, 3%, or 2%, and for example greater than about 0.5%, or 1%), but then are cleared by the liver (*i.e.*, hepatic extraction) and/or the kidney (*i.e.,* renal excretion).

In this regard it is to be further noted, that in certain embodiments, due to the substantial impermeability and/or substantial systemic non-bioavailability of the NHE3 inhibitor, greater than about 50%, 60%, 70%, 80%, 90%, or 95% of a compound of the invention is recoverable from the feces over, for example, a 24, 36, 48, 60, 72, 84, or 96 hour period following administration to a subject in need thereof. In this respect, it is understood that a recovered compound can include the sum of the parent compound and its metabolites derived from the parent compound, *e.g.*, by means of hydrolysis, conjugation, reduction, oxidation, N-alkylation, glucuronidation, acetylation, methylation, sulfation, phosphorylation, or any other modification that adds atoms to or removes atoms from the parent compound, wherein the metabolites are generated via the action of any enzyme or exposure to any physiological environment including, pH, temperature, pressure, or interactions with foodstuffs as they exist in the digestive milieu.

Measurement of fecal recovery of NHE3 inhibitor compound and metabolites can be carried out using standard methodology. For example, a compound can be administered orally at a suitable dose (*e.g.*, 10 mg/kg) and feces are then collected at predetermined times after dosing (*e.g.*, 24 hours, 36 hours, 48 hours, 60 hours, 72 hours, 96 hours). Parent compound and metabolites can be extracted with organic solvent and analyzed quantitatively using mass spectrometry. A mass balance analysis of the parent compound and metabolites (including, parent = M, metabolite 1 [M+16], and metabolite 2 [M+32]) can be used to determine the percent recovery in the feces.

## *$C_{max}$ and $IC_{50}$ or $EC_{50}$*

In some embodiments, the substantially systemically non-bioavailable NHE3 inhibitor detailed herein, when administered (e.g., enterally) either alone or in combination with the phosphate binder to a subject in need thereof, exhibit a maximum concentration detected in the serum, defined as $C_{max}$, that is about the same as or less than the phosphate ion (Pi) transport or uptake inhibitory concentration $IC_{50}$ of the compound. In some embodiments, for instance, the $C_{max}$ is about or at least about 5%, 10%, 20%, 30%, 40%, 50%, 60%, 70%, 80%, 90%, or 100% less than the $IC_{50}$ for inhibiting Pi transport or uptake. In some embodiments, the $C_{max}$ is about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9X (0.9 times) the $IC_{50}$ for inhibiting Pi transport or uptake.

In certain embodiments, one or more of the substantially systemically non-bioavailable NHE3 inhibitor compounds detailed herein, when administered (e.g., enterally) to a subject in need thereof, may have a ratio of $C_{max}$:$IC_{50}$ (for inhibiting Pi transport or update), wherein $C_{max}$ and $IC_{50}$ are expressed in terms of the same units, of at about or less than about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1.0, or a range in between about 0.01-1.0, 0.01-0.9, 0.01-0.8, 0.01-0.7, 0.01-0.6, 0.01-0.5, 0.01-0.4, 0.01-0.3, 0.01-0.2, or 0.01-0.1, or a range in between about 0.1-1.0, 0.1-0.9, 0.1-0.8, 0.1-0.7, 0.1-0.6, 0.1-0.5, 0.1-0.4, 0.1-0.3, or 0.1-0.2.

In some embodiments, the substantially systemically non-bioavailable NHE3 inhibitor detailed herein, when administered (e.g., enterally) either alone or in combination with one or more additional pharmaceutically active compounds or agents to a subject in need thereof, exhibit a maximum concentration detected in the serum, defined as $C_{max}$, that is about the same as or less than $EC_{50}$ of the compound for increasing fecal output of phosphate, where fecal output is increased by about or at least about 5%, 10%, 20%, 30%, 40%, 50%, 60%, 70%, 80%, 90%, or 100%. In some embodiments, for instance, the $C_{max}$ is about or at least about 5%, 10%, 20%, 30%, 40%, 50%, 60%, 70%, 80%, 90%, or 100% less than the $EC_{50}$ for increasing fecal output of phosphate. In some embodiments, the $C_{max}$ is about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9X (0.9 times) the $EC_{50}$ for increasing fecal output of phosphate.

In some embodiments, one or more of the substantially systemically non-bioavailable compounds detailed herein, when administered (e.g., enterally) either alone or in combination with one or more additional pharmaceutically active compounds or agents to a subject in need thereof, or measured in an animal model or cell-based assay, may have an $EC_{50}$ for increasing fecal output of phosphate of about or less than about 10 μM, 9 μM, 8 μM, 7 μM, 7.5 μM, 6 μM, 5 μM, 4 μM, 3 μM, 2.5 μM, 2 μM, 1 μM, 0.5 μM, 0.1 μM, 0.05 μM, or 0.01 μM, or less, the $IC_{50}$ being, for example, within the range of about 0.01 μM to about 10 μM, or about 0.01 μM to about 7.5 μM, or about 0.01 μM to about 5 μM, or about 0.01 μM to about 2.5 μM, or about 0.01 μM to about 1.0, or about 0.1 μM to about 10 μM, or about 0.1 μM to about 7.5 μM, or about 0.1 μM to about 5 μM, or about 0.1 μM to about 2.5 μM, or about 0.1 μM to about 1.0, or about μM 0.5 μM to about 10 μM, or about 0.5 μM to about 7.5 μM, or about 0.5 μM to about 5 μM, or about 0.5 μM to about 2.5 μM, or about 0.5 μM to about 1.0 μM.

In particular embodiments, the substantially systemically non-bioavailable NHE3 inhibitor detailed herein, when administered (e.g., enterally) either alone or in combination with one or more additional pharmaceutically active compounds or agents to a subject in need thereof, exhibit a maximum concentration detected in the serum, defined as $C_{max}$, that is about the same as or less than $EC_{50}$ of the compound for reducing urinary output of phosphate, where urinary output is reduced by about or at least about 5%, 10%, 20%, 30%, 40%, 50%, 60%, 70%, 80%, 90%, or 100%. In some embodiments, for instance, the $C_{max}$ is about or at least about 5%, 10%, 20%, 30%, 40%, 50%, 60%, 70%, 80%, 90%, or 100% less than the $EC_{50}$ for reducing urinary output of phosphate. In some embodiments, the $C_{max}$ is about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9X (0.9 times) the $EC_{50}$ for reducing urinary output of phosphate.

In some embodiments, one or more of the substantially systemically non-bioavailable NHE3 inhibitor detailed herein, when administered (e.g., enterally) either alone or in combination with the phosphate binder to a subject in need thereof, or measured in an animal model or cell-based assay, may have an $EC_{50}$ for reducing urinary output of phosphate of about or less than about 10 μM, 9 μM, 8 μM, 7 μM, 7.5 μM, 6 μM, 5 μM, 4 μM, 3 μM, 2.5 μM, 2 μM, 1 μM, 0.5 μM, 0.1 μM, 0.05 μM, or 0.01 μM, or less, the $IC_{50}$ being, for example, within the range of about 0.01 μM to about 10 μM, or about 0.01 μM to about 7.5 μM, or about 0.01 μM to about 5 μM, or about 0.01 μM to about 2.5 μM, or about 0.01 μM to about 1.0, or about 0.1 μM to about 10 μM, or about 0.1 μM to about 7.5 μM, or about 0.1 μM to about 5 μM, or about 0.1 μM to about 2.5 μM, or about 0.1 μM to about 1.0, or about μM 0.5 μM to about 10 μM, or about 0.5 μM to about 7.5 μM, or about 0.5 μM to about 5 μM, or about 0.5 μM to about 2.5 μM, or about 0.5 μM to about 1.0 μM.

In certain embodiments, one or more of the substantially systemically non-bioavailable NHE3 inhibitor compounds detailed herein, when administered (e.g., enterally) to a subject in need thereof, may have a ratio of $C_{max}$:$EC_{50}$ (e.g., for increasing fecal output of phosphate, for decreasing urinary output of phosphate), wherein $C_{max}$ and $EC_{50}$ are expressed in terms of the same units, of at about or less than about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1.0, or a range in between about 0.01-1.0, 0.01-0.9, 0.01-0.8, 0.01-0.7, 0.01-0.6, 0.01-0.5, 0.01-0.4, 0.01-0.3, 0.01-0.2, or 0.01-0.1, or a range in between about 0.1-1.0, 0.1-0.9, 0.1-0.8, 0.1-0.7, 0.1-0.6, 0.1-0.5, 0.1-0.4, 0.1-0.3, or 0.1-0.2.

Additionally, or alternatively, one or more of the substantially systemically non-bioavailable NHE3 inhibitor compounds detailed herein, when administered (e.g., enterally) either alone or in combination with one or more additional pharmaceutically active compounds or agents to a subject in need thereof, may have a $C_{max}$ of about or less than about 10 ng/ml, about 7.5 ng/ml, about 5 ng/ml, about 2.5 ng/ml, about 1 ng/ml, or about 0.5 ng/ml, the $C_{max}$ being for example within the range of about 1 ng/ml to about 10 ng/ml, or about 2.5 ng/ml to about 7.5 ng/ml.

The NHE3 inhibitor, which may be monovalent or polyvalent, that binds to and/or modulates NHE3 and has activity as a phosphate transport inhibitor, including small molecules that are substantially impermeable or substantially systemically non-bioavailable in the gastrointestinal tract, including known NHE-binding compounds that may be modified or functionalized in accordance with the present disclosure to alter the physicochemical properties thereof so as to render the overall compound substantially active in the GI tract.

Accordingly, the compounds of the present disclosure may be generally represented by Formula (I):

NHE-Z (I)

wherein: (i) NHE represents a NHE-binding small molecule, and (ii) Z represents a moiety having at least one site thereon for attachment to an NHE-binding small molecule, the resulting NHE-Z molecule possessing overall physicochemical properties that render it substantially impermeable or substantially systemically non-bioavailable. The NHE-binding small molecule generally comprises a heteroatom-containing moiety and a cyclic or heterocyclic scaffold or support moiety bound directly or indirectly thereto. In particular, examination of the structures of small molecules reported to-date to be NHE-binders or inhibitors suggest, as further illustrated herein below, that most comprise a cyclic or heterocyclic support or scaffold bound directly or indirectly (by, for example, an acyl moiety or a hydrocarbyl or heterohydrocarbyl moiety, such as an alkyl, an alkenyl, a heteroalkyl or a heteroalkenyl moiety) to a heteroatom-containing moiety that is capable of acting as a sodium atom or sodium ion mimic, which is typically selected from a substituted guanidinyl moiety and a substituted heterocyclic moiety (e.g., a nitrogen-containing heterocyclic moiety). Optionally, the heteroatom-containing moiety may be fused with the scaffold or support moiety to form a fused, bicyclic structure, and/or it may be capable of forming a positive charge at a physiological pH.

In this regard it is to be noted that, while the heteroatom-containing moiety that is capable of acting as a sodium atom or ion mimic may optionally form a positive charge, this should not be understood or interpreted to require that the overall compound have a net positive charge, or only a single positively charged moiety therein. Rather, in various embodiments, the compound may have no charged moieties, or it may have multiple charged moieties therein (which may have positive charges, negative charges, or a combination thereof, the compound for example being a zwitterion). Additionally, it is to be understood that the overall compound may have a net neutral charge, a net positive charge (e.g., +1, +2, +3, etc.), or a net negative charge (e.g., -1, -2, -3, etc.).

The Z moiety may be bound to essentially any position on, or within, the NHE small molecule, and in particular may be: (i) bound to the scaffold or support moiety, (ii) bound to a position on, or within, the heteroatom-containing moiety, and/or (iii) bound to a position on, or within, a spacer moiety that links the scaffold to the heteroatom-containing moiety, provided that the installation of the Z moiety does not significantly adversely impact NHE-binding activity. In one particular embodiment, Z may be in the form of an oligomer, dendrimer or polymer bound to the NHE small molecule (e.g., bound for example to the scaffold or the spacer moiety), or alternatively Z may be in the form of a linker that links multiple NHE small molecules together, and therefore that acts to increase: (i) the overall molecular weight and/or polar surface area of the NHE-Z molecule; and/or, (ii) the number of freely rotatable bonds in the NHE-Z molecule; and/or, (iii) the number of hydrogen-bond donors and/or acceptors in the NHE-Z molecule; and/or, (iv) the Log P value of the NHE-Z molecule to a value of at least about 5 (or alternatively less than 1, or even about 0), all as set forth herein; such that the overall NHE-binding compound (i.e., the NHE-Z compound) is substantially impermeable or substantially systemically non-bioavailable.

The present disclosure is more particularly directed to such a substantially impermeable or substantially systemically non-bioavailable, NHE-binding compound, or a pharmaceutical salt thereof, wherein the compound has the structure of Formula (II):

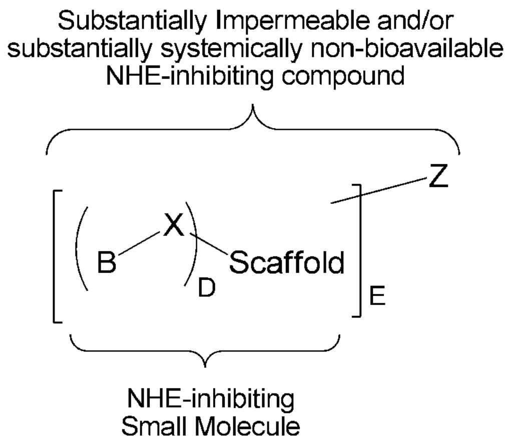


(II)

wherein: (i) Z, as previously defined above, is a moiety bound to or incorporated in the NHE-binding small molecule, such that the resulting NHE-Z molecule possesses overall physicochemical properties that render it substantially impermeable or substantially systemically non-bioavailable ; (ii) B is the heteroatom-containing moiety of the NHE-binding small molecule, and in one particular embodiment is selected from a substituted guanidinyl moiety and a substituted heterocyclic moiety, which may optionally be fused with the Scaffold moiety to form a fused, bicyclic structure; (iii) Scaffold is the cyclic or heterocyclic moiety to which is bound directly or indirectly the hetero-atom containing moiety (e.g., the substituted

guanidinyl moiety or a substituted heterocyclic moiety), B, and which is optionally substituted with one or more additionally hydrocarbyl or heterohydrocarbyl moieties; (iv) X is a bond or a spacer moiety selected from a group consisting of substituted or unsubstituted hydrocarbyl or heterohydrocarbyl moieties, and in particular substituted or unsubstituted $C_1$-$C_7$ hydrocarbyl or heterohydrocarbyl (e.g., $C_1$-$C_7$ alkyl, alkenyl, heteroalkyl or heteroalkenyl), and substituted or unsubstituted, saturated or unsaturated, cyclic or heterocyclic moieties (e.g., $C_4$-$C_7$ cyclic or heterocyclic moieties), which links B and the Scaffold; and, (v) D and E are integers, each independently having a value of 1, 2 or more.

In one or more particular embodiments, as further illustrated herein below, B may be selected from a guanidinyl moiety or a moiety that is a guanidinyl bioisostere selected from the group consisting of substituted cyclobutenedione, substituted imidazole, substituted thiazole, substituted oxadiazole, substituted pyrazole, or a substituted amine. More particularly, B may be selected from guanidinyl, acylguanidinyl, sulfonylguanidinyl, or a guanidine bioisostere such as a cyclobutenedione, a substituted or unsubstituted 5- or 6-member heterocycle such as substituted or unsubstituted imidazole, aminoimidazole, alkylimidizole, thiazole, oxadiazole, pyrazole, alkylthioimidazole, or other functionality that may optionally become positively charged or function as a sodium mimetic, including amines (e.g., tertiary amines), alkylamines, and the like, at a physiological pH. In one particularly preferred embodiment, B is a substituted guanidinyl moiety or a substituted heterocyclic moiety that may optionally become positively charged at a physiological pH to function as a sodium mimetic. In one exemplary embodiment, the compound of the present disclosure (or more particularly the pharmaceutically acceptable HCl salt thereof, as illustrated) may have the structure of Formula (III):

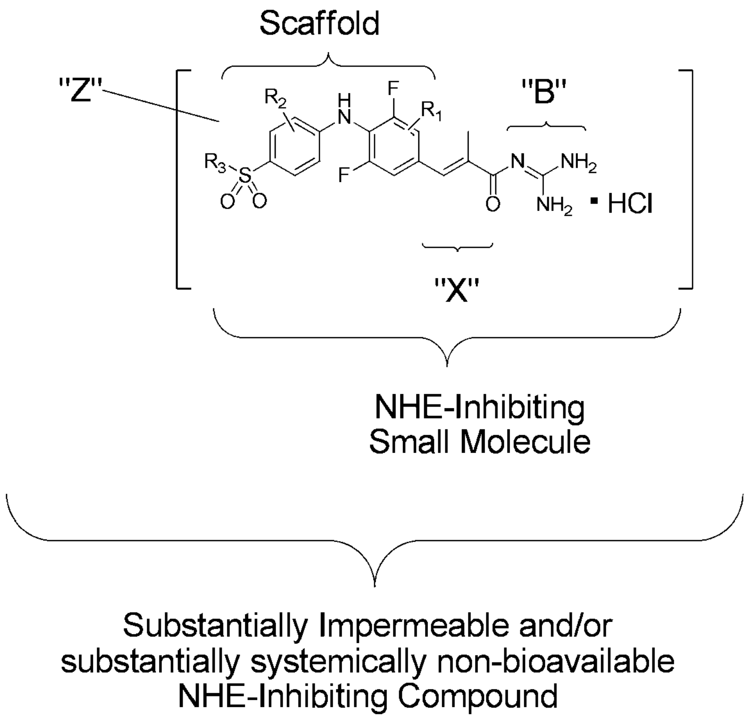


(III)

wherein Z may be optionally attached to any one of a number of sites on the NHE-binding small molecule, and further wherein the $R_1$, $R_2$ and $R_3$ substituents on the aromatic rings are as detailed elsewhere herein, and/or in U.S. Pat. No. 6,399,824, the entire contents of which are incorporated herein by reference for all relevant and consistent purposes.

In this regard it is to be noted, however, that the substantially impermeable or substantially systemically non-bioavailable NHE-binding compounds of the present disclosure may have a structure other than illustrated above, without departing from the scope of the present disclosure. For example, in various alternative embodiments, one or both of the terminal nitrogen atoms in the guanidine moiety may be substituted with one or more substituents, and/or the modifying or functionalizing moiety Z may be attached to the NHE-binding compound by means of (i) the Scaffold, (ii) the spacer X, or (iii) the heteroatom-containing moiety, B, as further illustrated generally in the structures provided below:

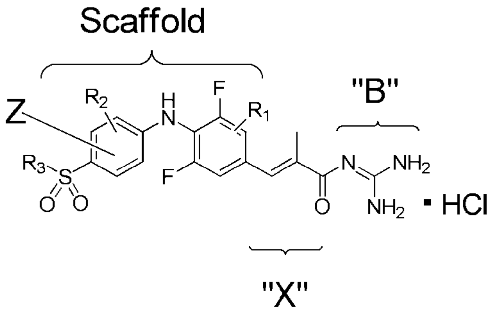

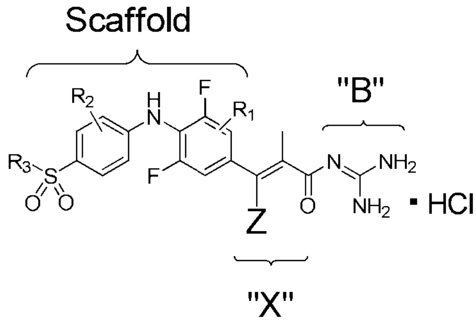

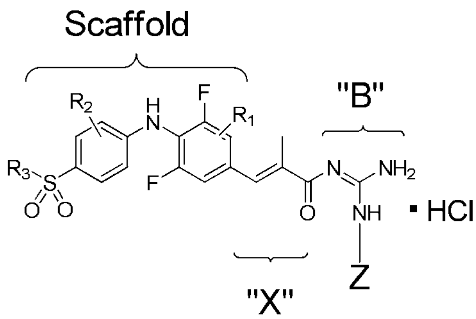

In this regard it is to be further noted that, as used herein, "bioisostere" generally refers to a moiety with similar physical and chemical properties to a guanidine moiety, which in turn imparts biological properties to that given moiety similar to, again, a guanidine moiety, in this instance. (See, for example, Ahmad, S. et al., Aminoimidazoles as Bioisosteres of Acylguanidines: Novel, Potent, Selective and Orally Bioavailable Inhibitors of the Sodium Hydrogen Exchanger Isoform-1, Boorganic & Med. Chem. Lett., pp. 177-180 (2004), the entire contents of which is incorporated herein by reference for all relevant and consistent purposes.)

As further detailed below, known NHE-binding small molecules or chemotypes that may serve as suitable starting materials (for modification or functionalization, in order to render the small molecules substantially impermeable or substantially systemically non-bioavailable, and/or used in pharmaceutical preparations) may generally be organized into a number of subsets, such as for example:

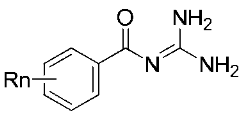

Benzoylguanidines

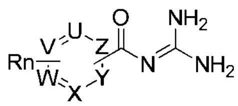

Heteroaroylguanidines

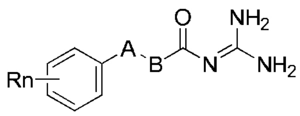

"Spacer-Stretched" Aroylguanidines

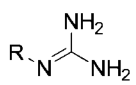

Non-Acyl Guanidines

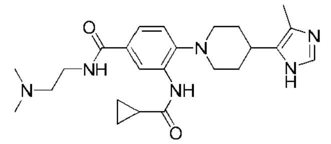

Non-guanidine NHE inhibitors

wherein: the terminal ring (or, in the case of the non-acyl guanidines, "R"), represent the scaffold or support moiety; the guanidine moiety (or the substituted heterocycle, and more specifically the piperidine ring, in the case of the non-guanidine inhibitors) represents B; and, X is the acyl moiety, or the -A-B-acyl- moiety (or a bond in the case of the non-acyl guanidines and the non-guanidine inhibitors). (See, e.g., Lang, H. J., "Chemistry of NHE Inhibitors" in The Sodium-Hydrogen Exchanger, Harmazyn, M., Avkiran, M. and Fliegel, L., Eds., Kluwer Academic Publishers 2003. See also B. Masereel et al., An Overview of Inhibitors of Na+ / H+ Exchanger, European J. of Med. Chem., 38, pp. 547-554 (2003), the entire contents of which is incorporated by reference here for all relevant and consistent purposes). Without being held to any particular theory, it has been proposed that a guanidine group, or an acylguanidine group, or a charged guanidine or acylguanidine group (or, in the case of non-guanidine inhibitors, a heterocycle or other functional group that can replicate the molecular interactions of a guanidinyl functionality including, but not limited to, a protonated nitrogen atom in a

piperidine ring) at physiological pH may mimic a sodium ion at the binding site of the exchanger or antiporter (See, e.g., Vigne et al., J. Biol. Chem. 1982, 257, 9394).

Although the heteroatom-containing moiety may be capable of forming a positive charge, this should not be understood or interpreted to require that the overall compound have a net positive charge, or only a single positively charged moiety therein, or even that the heteroatom-containing moiety therein be capable of forming a positive charge in all instances. Rather, in various alternative embodiments, the compound may have no charged moieties therein, or it may have multiple charged moieties therein (which may have positive charges, negative charges, or a combination thereof). Additionally, it is to be understood that the overall compound may have a net neutral charge, a net positive charge, or a net negative charge.

In this regard it is to be noted that the U.S. Patents and U.S. Published Applications cited above, or elsewhere herein, are incorporated herein by reference in their entirety, for all relevant and consistent purposes.

In addition to the structures illustrated above, and elsewhere herein, it is to be noted that bioisosteric replacements for guanidine or acylguanidine may also be used. Potentially viable bioisosteric "guanidine replacements" identified to-date have a five- or six-membered heterocyclic ring with donor/acceptor and pKa patterns similar to that of guanidine or acylguanidine (see for example Ahmad, S. et al., Aminoimidazoles as Bioisosteres of Acylguanidines: Novel, Potent, Selective and Orally Bioavailable Inhibitors of the Sodium Hydrogen Exchanger Isoform-1, Boorganic & Med. Chem. Lett., pp. 177-180 (2004), the entire contents of which is incorporated herein by reference for all relevant and consistent purposes), and include those illustrated below:

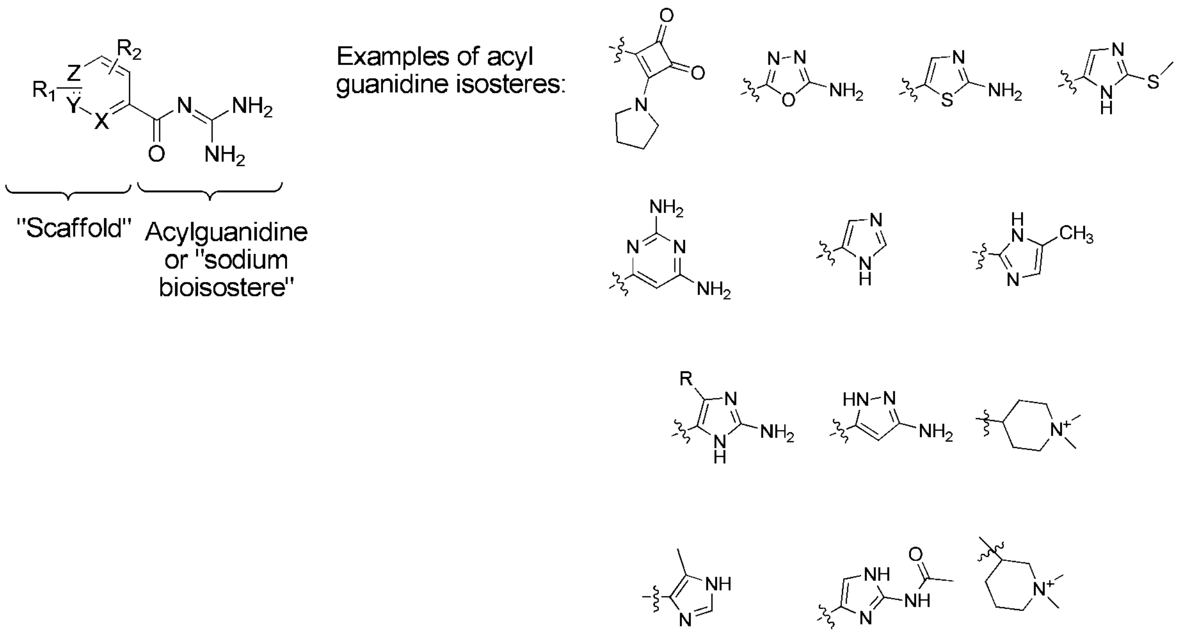


The above bioisosteric embodiments (i.e., the group of structures above) correspond to "B" in the structure of Formula (II), the broken bond therein being attached to "X" (e.g., the acyl moiety, or alternatively a bond linking the bioisostere to the scaffold), with bonds to Z in Formula (III) not shown here.

It is to be noted that, in the many structures illustrated herein, all of the various linkages or bonds will not be shown in every instance. For example, in one or more of the structures illustrated above, a bond or connection between the NHE-binding small molecule and the modifying or functionalizing moiety Z is not always shown. However, this should not be viewed in a limiting sense. Rather, it is to be understood that the NHE-binding small molecule is bound or connected in some way (e.g., by a bond or linker of some kind) to Z, such that the resulting NHE-Z molecule is suitable for use (i.e., substantially impermeable or substantially systemically non-bioavailable in the GI tract). Alternatively, Z may be incorporated into the NHE-binding small molecule, such as for example by positioning it between the guanidine moiety and scaffold.

It is to be further noted that a number of structures are provided herein for substantially impermeable or substantially systemically non-bioavailable NHE-binding compounds, and/or for NHE-binding small molecules suitable for modification or functionalization in accordance with the present disclosure so as to render them substantially impermeable or substantially systemically non-bioavailable. Due to the large number of structures, various identifiers (e.g., atom identifiers in a chain or ring, identifiers for substituents on a ring or chain, etc.) may be used more than once. An identifier in one structure should therefore not be assumed to have the same meaning in a different structure, unless specifically stated (e.g., "$R_1$" in one structure may or may not be the same as "$R_1$" in another structure). Additionally, it is to be noted that, in one

or more of the structures further illustrated herein below, specific details of the structures, including one or more of the identifiers therein, may be provided in a cited reference, the contents of which are specifically incorporated herein by reference for all relevant and consistent purposes.

Small molecules suitable for use (i.e., suitable for use as substantially bioavailable compounds, suitable for modification or functionalization to generate substantially systemically non-bioavailable compounds) include those illustrated below. In this regard it is to be noted a bond or link to Z (i.e., the modification or functionalization that renders the small molecules substantially impermeable or substantially systemically non-bioavailable) is not specifically shown. As noted, the Z moiety may be attached to, or included within, the small molecule at essentially any site or position that does not interfere (e.g., sterically interfere) with the ability of the resulting compound to effectively bind the NHE antiport of interest. More particularly, Z may be attached to essentially any site on the NHE-binding small molecule, Z for example displacing all or a portion of a substituent initially or originally present thereon and as illustrated below, provided that the site of installation of the Z moiety does not have a substantially adversely impact on the NHE-binding activity thereof. In one particular embodiment, however, a bond or link extends from Z to a site on the small molecule that effectively positions the point of attachment as far away (based, for example, on the number of intervening atoms or bonds) from the atom or atoms present in the resulting compound that effectively act as the sodium ion mimic (for example, the atom or atoms capable of forming a positive ion under physiological pH conditions). In a preferred embodiment, the bond or link will extend from Z to a site in a ring, and more preferably an aromatic ring, within the small molecule, which serves as the scaffold.

In view of the foregoing, in one particular embodiment, the following small molecule, disclosed in U.S. Patent Application No. 2005/0054705, the entire content of which (and in particular the text of pages 1-2 therein) is incorporated herein by reference for all relevant and consistent purposes, may be suitable for use or modification in accordance with the present disclosure (e.g., bound to or modified to include Z, such that the resulting NHE-Z molecule is substantially impermeable or substantially systemically non-bioavailable).

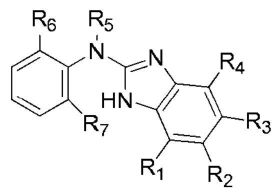

The variables in the structure are defined in the cited patent application, the details of which are incorporated herein by reference. In one particularly preferred embodiment, $R_6$ and $R_7$ are a halogen (e.g., Cl), $R_5$ is lower alkyl (e.g., $CH_3$), and $R_1$-$R_4$ are H, the compound having for example the structure:

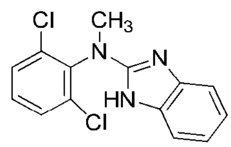

In yet another particular embodiment, the following small molecule, disclosed in Canadian Patent Application No. 2,241,531 (or International Patent Publication No. WO 97/24113), the entire content of which (and in particular pages 1-2 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

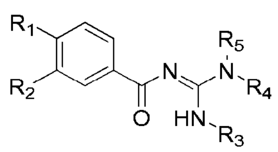

The variables in the structure are defined in the cited patent application, the details of which are incorporated herein by reference.

In yet another particular embodiment, the following small molecule, disclosed in Canadian Patent Application No. 2,241,531 (or International Patent Publication No. WO 97/24113), the entire content of which (and in particular page 49 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

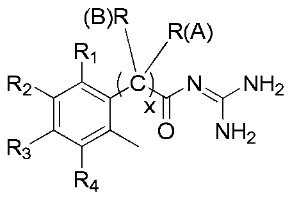

The variables in the structure are defined in the cited patent application, the details of which are incorporated herein by reference.

In yet another particular embodiment, the following small molecule, disclosed in Canadian Patent Application No. 2,241,531 (or International Patent Publication No. WO 97/24113), the entire content of which (and in particular pages 118-120 and 175-177 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

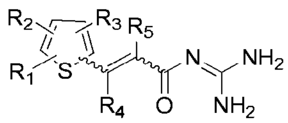

The variables in the structure are defined in the cited patent application, the details of which are incorporated herein by reference.

In yet another particular embodiment, the following small molecule, disclosed in Canadian Patent Application No. 2,241,531 (or International Patent Publication No. WO 97/24113), the entire content of which (and in particular pages 129-131 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

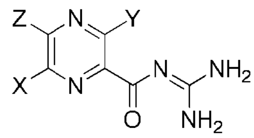

The variables in the structure are defined in the cited patent application, the details of which are incorporated herein by reference. (In this regard it is to be noted that the substituent Z within the structure illustrated above is not to be confused with the moiety Z that, in accordance with the present disclosure, is attached to the NHE-binding small molecule in order effective render the resulting "NHE-Z" molecule substantially impermeable.).

In yet another particular embodiment, the following small molecule, disclosed in Canadian Patent Application No. 2,241,531 (or International Patent Publication No. WO 97/24113), the entire content of which (and in particular pages 127-129 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

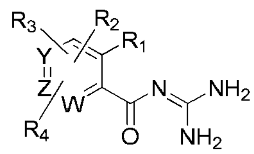

The variables in the structure are defined in the cited patent application, the details of which are incorporated herein by reference. (In this regard it is to be noted that Z within the ring of the structure illustrated above is not to be confused with the moiety Z that, in accordance with the present disclosure, is attached to the NHE-binding small molecule in order effective render the resulting "NHE-Z" molecule substantially impermeable.)

In yet another particular embodiment, the following small molecule, disclosed in Canadian Patent Application No. 2,241,531 (or International Patent Publication No. WO 97/24113), the entire content of which (and in particular pages 134-137 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

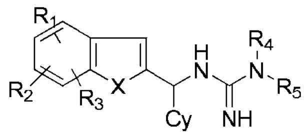

The variables in the structure are defined in the cited patent application, the details of which are incorporated herein by reference.

In yet another particular embodiment, the following small molecule, disclosed in Canadian Patent Application No. 2,241,531 (or International Patent Publication No. WO 97/24113), the entire content of which (and in particular pages 31-32 and 137-139 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

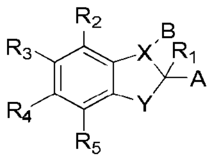

The variables in the structure are defined in the cited patent application, the details of which are incorporated herein by reference.

In yet another particular embodiment, the following small molecule, disclosed in Canadian Patent Application No. 2,241,531 (or International Patent Publication No. WO 97/24113), the entire content of which (and in particular pages 37-45 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

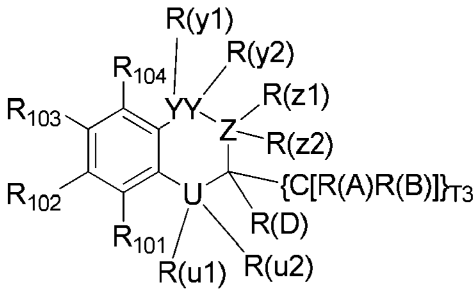

The variables in the structure are defined in the cited patent application, the details of which are incorporated herein by reference. (In this regard it is to be noted that Z within the ring structure illustrated above is not to be confused with the moiety Z that, in accordance with the present disclosure, is attached to the NHE-binding small molecule in order effective render the resulting "NHE-Z" molecule substantially impermeable.)

In yet another particular embodiment, the following small molecule, disclosed in Canadian Patent Application No. 2,241,531 (or International Patent Publication No. WO 97/24113), the entire content of which (and in particular pages 100-102 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

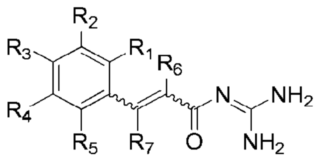

The variables in the structure are defined in the cited patent application, the details of which are incorporated herein by reference (wherein, in particular, the wavy bonds indicate variable length, or a variable number of atoms, therein).

In yet another particular embodiment, the following small molecule, disclosed in Canadian Patent Application No.

2,241,531 (or International Patent Publication No. WO 97/24113), the entire content of which (and in particular pages 90-91 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

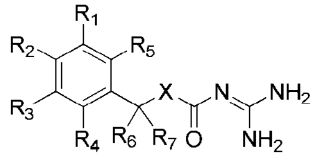

The variables in the structure are defined in the cited patent application, the details of which are incorporated herein by reference.

In yet another particular embodiment, the following small molecule, disclosed in U.S. Patent No. 5,900,436 (or EP 0822182 B1), the entire contents of which (and in particular column 1, lines 10-55 therein) are incorporated herein by reference for all relevant and consistent purposes, may be suitable for use or modification in accordance with the present disclosure (e.g., bound to or modified to include Z, such that the resulting NHE-Z molecule is substantially impermeable or substantially systemically non-bioavailable).

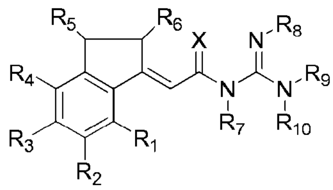

The variables in the structures are defined in the cited patents, the details of which are incorporated herein by reference.

In yet another particular embodiment, the following small molecule, disclosed in Canadian Patent Application No. 2,241,531 (or International Patent Publication No. WO 97/24113), the entire content of which (and in particular pages 35-47 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

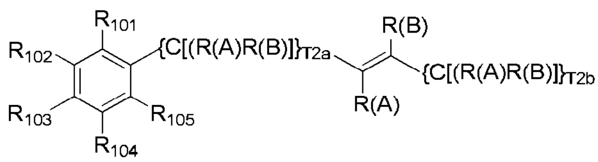

The variables in the structure are defined in the cited patent application, the details of which are incorporated herein by reference.

In yet another particular embodiment, the following small molecule, disclosed in Canadian Patent Application No. 2,241,531 (or International Patent Publication No. WO 97/24113), the entire content of which (and in particular pages 154-155 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

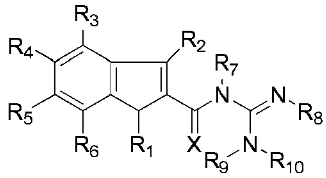

The variables in the structure are defined in the cited patent application, the details of which are incorporated herein by reference.

In yet another particular embodiment, the following small molecule, disclosed in Canadian Patent Application No.

2,241,531 (or International Patent Publication No. WO 97/24113), the entire content of which (and in particular pages 132-133 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

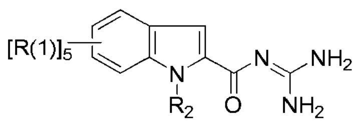

The variables in the structure are defined in the cited patent application, the details of which are incorporated herein by reference.

In yet another particular embodiment, the following small molecule, disclosed in Canadian Patent Application No. 2,241,531 (or International Patent Publication No. WO 97/24113), the entire content of which (and in particular pages 58-65 AND 141-148 therein) is incorporated herein for all relevant and consistent purposes, may be suitable for use or modification in accordance with the present disclosure (e.g., bound to or modified to include Z, such that the resulting NHE-Z molecule is substantially impermeable or substantially systemically non-bioavailable).

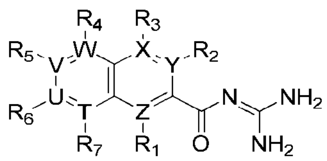

The variables in the structure are defined in the cited patent application, the details of which are incorporated herein by reference. (In this regard it is to be noted that Z within the ring structure illustrated above is not to be confused with the moiety Z that, in accordance with the present disclosure, is attached to the NHE-binding small molecule in order effective render the resulting "NHE-Z" molecule substantially impermeable.)

In yet another particular embodiment, the following small molecule, disclosed in U.S. Patent Nos. 6,911,453 and 6,703,405, the entire contents of which (and in particular the text of columns 1-7 and 46 of 6,911,453 and columns 14-15 of 6,703,405) are incorporated herein by reference for all relevant and consistent purposes, may be suitable for use or modification in accordance with the present disclosure (e.g., bound to or modified to include Z, such that the resulting NHE-Z molecule is substantially impermeable or substantially systemically non-bioavailable).

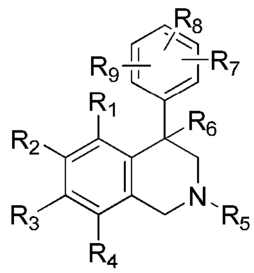

The variables in the structure are defined in the cited patents, the details of which are incorporated herein by reference. A particularly preferred small molecule falling within the above-noted structure is further illustrated below (see, e.g., Example 1 of the US6,911,453 patent, the entire contents of which are specifically incorporated herein by reference):

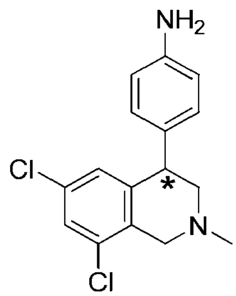

In yet another particular embodiment, the following small molecules, disclosed in U.S. Patent Publication Nos. 2004/0039001, 2004/0224965, 2005/0113396 and 2005/0020612, the entire contents of which are incorporated herein by reference for all relevant and consistent purposes, may be suitable for use or modification in accordance with the present disclosure (e.g., bound to or modified to include Z, such that the resulting NHE-Z molecule is substantially impermeable or substantially systemically non-bioavailable).

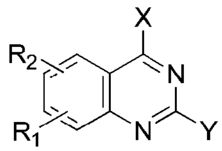

X = Ar (aryl), Het (heterocycle)

Y =

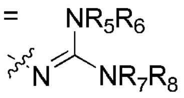
,
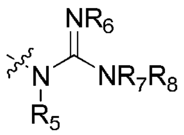
,
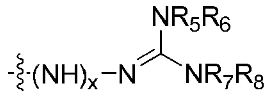

The variables in the structures are defined above and/or in one or more of the cited patent applications, the details of which are incorporated herein by reference, and/or as illustrated above (wherein the broken bonds indicate a point of attachment for the Y moiety to the fused heterocyclic ring). In particular, in various embodiments the combination of X and Y may be as follows:

X = Ar and

Y =

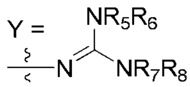
or
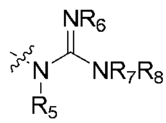

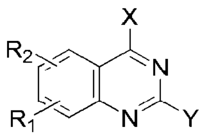

(see, e.g., US 2004/0039001, p. 1 therein)

X = Ar and Y =

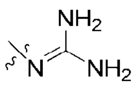

(see, e.g., US 2004/0224965, p. 1 therein)

X = Het and

Y =

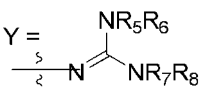
or
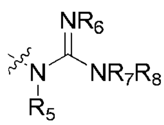

(see, e.g., US 2005/0113396, p. 1 therein)

X = Het and

Y = 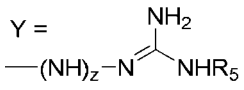 or 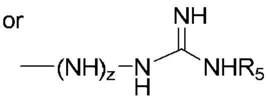

or

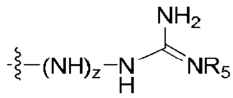

(see, e.g., US 2005/00020612, p. 1 therein)

In a particularly preferred embodiment of the above-noted structure, the small molecule has the general structure:

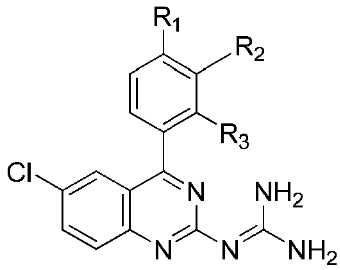

wherein $R_1$, $R_2$ and $R_3$ may be the same or different, but are preferably different, and are independently selected from H, NR'R" (wherein R' and R" are independently selected from H and hydrocarbyl, such as lower alkyl, as defined elsewhere herein) and the structure:

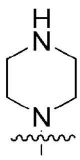

In a more particularly preferred embodiment of the above structure, a small molecule falling within the above-noted structure is further illustrated below (see, e.g., compound I1 on p. 5 of the 2005/0020612 patent application, the entire contents of which are specifically incorporated herein by reference):

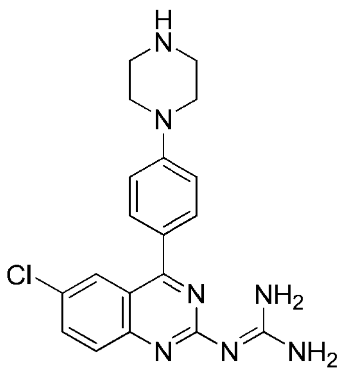

In another particularly preferred embodiment, the following small molecule, disclosed in U.S. Patent No. 6,399,824, the entire content of which (and in particular the text of Example 1 therein) is incorporated herein by reference for all relevant and consistent purposes, may be particularly suitable for use or modification in accordance with the present disclosure (e.g., bound to or modified to include Z, such that the resulting NHE-Z molecule is substantially impermeable or

substantially systemically non-bioavailable).

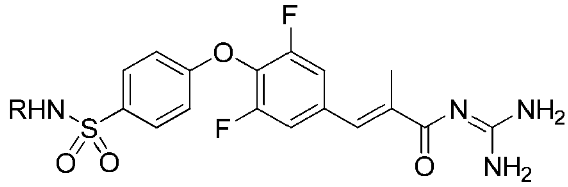

In the structure, R may be preferably selected from H and $(CH_3)_2NCH_2CH_2$-, with H being particularly preferred in various embodiments.

In yet another particular embodiment, the following small molecule, disclosed in U.S. Patent No. 6,005,010 (and in particular columns 1-3 therein), and/or U.S. Patent No. 6,166,002 (and in particular columns 1-3 therein), the entire contents of which are incorporated herein by reference for all relevant and consistent purposes, may be suitable for use or modification in accordance with the present disclosure (e.g., bound to or modified to include Z, such that the resulting NHE-Z molecule is substantially impermeable or substantially systemically non-bioavailable).

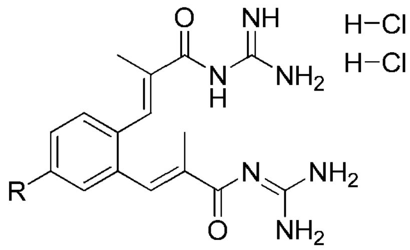

The variable ("R") in the structure is defined in the cited patent application, the details of which are incorporated herein by reference.

In yet another particularly preferred embodiment, the following small molecule, disclosed in U.S. Patent Application No. 2008/0194621, the entire content of which (and in particular the text of Example 1 therein) is incorporated herein by reference for all relevant and consistent purposes, may be particularly suitable for use or modification in accordance with the present disclosure (e.g., bound to or modified to include Z, such that the resulting NHE-Z molecule is substantially impermeable or substantially systemically non-bioavailable).

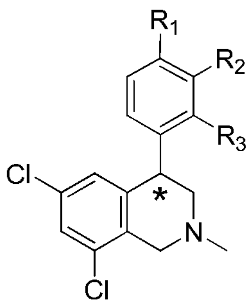

| $R_1$ | $R_2$ | $R_3$ |
|---|---|---|
| 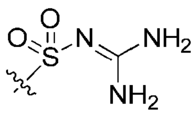 | -H | -H |
| -NH2 | -H | -H |
| -H | 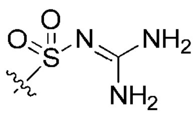 | -H |
| -H | -NH2 | -H |
| -H | -H | -NH2 |

The variables ("$R_1$", "$R_2$ and "$R_3$") in the structure are as defined above, and/or as defined in the cited patent application, the details of which are incorporated herein by reference.

In yet another particularly preferred embodiment, the following small molecule, disclosed in U.S. Patent Application No. 2007/0225323, the entire content of which (and in particular the text of Example 36 therein) is incorporated herein by reference for all relevant and consistent purposes, may be particularly suitable for use or modification in accordance with the present disclosure (e.g., bound to or modified to include Z, such that the resulting NHE-Z molecule is substantially impermeable or substantially systemically non-bioavailable).

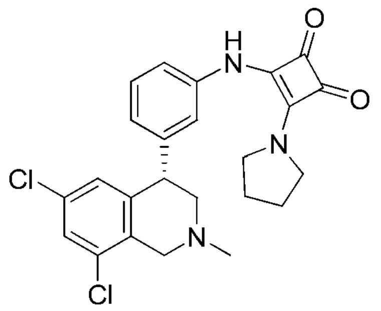

In yet another particularly preferred embodiment, the following small molecule, disclosed in U.S. Patent No. 6,911,453, the entire content of which (and in particular the text of Example 35 therein) is incorporated herein by reference for all relevant and consistent purposes, may be particularly suitable for use or modification in accordance with the present disclosure (e.g., bound to or modified to include Z, such that the resulting NHE-Z molecule is substantially impermeable or substantially systemically non-bioavailable).

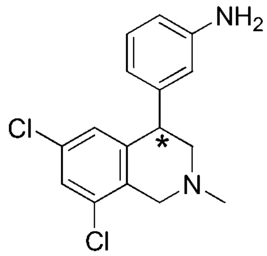

In one particularly preferred embodiment of the present disclosure, the small molecule may be selected from the group consisting of:

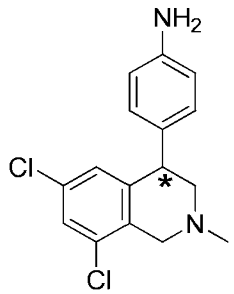

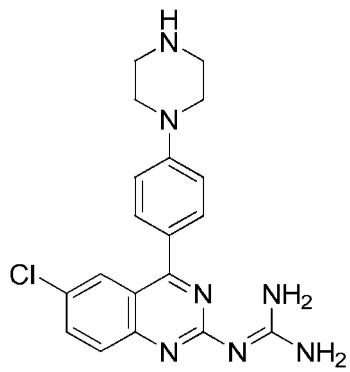

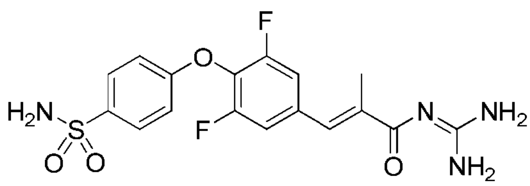

In these structures, a bond or link (not shown) may extend, for example, between the Core and amine-substituted aromatic ring (first structure), the heterocyclic ring or the aromatic ring to which it is bound, or alternatively the chloro-substituted aromatic ring (second structure), or the difluoro-substituted aromatic ring or the sulfonamide-substituted aromatic ring (third structure).

In one or more particular embodiments, the "NHE-Z" molecule is monovalent; that is, the molecule contains one moiety that effectively binds to and/or modulates NHE3 and also inhibits phosphate transport in the GI tract or kidneys. In such embodiments, the NHE-Z molecule may be selected, for example, from one of the following structures of Formulas (IV), (V), (VI) or (VII):

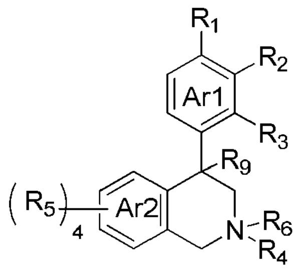

(IV)

wherein: each $R_1$, $R_2$, $R_3$, $R_5$ and $R_9$ are independently selected from H, halogen (e.g., Cl), $-NR_7(CO)R_8$, - $(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or Z, where Z is selected from substituted or unsubstituted hydrocarbyl, heterohydrocarbyl, polyalkylene glycol and polyols, where substituents thereon are selected from hydroxyls, amines, amidines, carboxylates, phosphonates, sulfonates, and guanidines; $R_4$ is selected from H, $C_1$-$C_7$ alkyl or Z, where Z is selected from substituted or unsubstituted hydrocarbyl, heterohydrocarbyl, a polyalkylene glycol and polyols, where substituents thereon are selected from hydroxyls, amines, amidines, carboxylates, phosphonates, sulfonates, and guanidines; $R_6$ is absent or selected from H and $C_1$-$C_7$ alkyl; and, Ar1 and Ar2 independently represent an aromatic ring, or alternatively a heteroaromatic ring wherein one or more of the carbon atoms therein is replaced with a N, O or S atom;

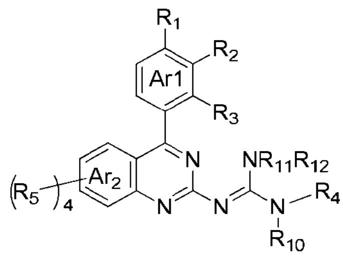

(V)

wherein: each $R_1$, $R_2$, $R_3$, and $R_5$ are independently selected from H, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or Z, where Z is selected from substituted or unsubstituted hydrocarbyl, heterohydrocarbyl, polyalkylene glycol and polyols, where substituents thereon are selected from hydroxyls, amines, amidines, carboxylates, phosphonates, sulfonates, and guanidines, optionally linked to the ring Ar1 by a heterocyclic linker; $R_4$ and $R_{12}$ are independently selected from H and $R_7$, where $R_7$ is as defined above; $R_{10}$ and $R_{11}$, when presented, are independently selected from H and $C_1$-$C_7$ alkyl; and, Ar1 and Ar2 independently represent an aromatic ring, or alternatively a heteroaromatic ring wherein one or more of the carbon atoms therein is replaced with a N, O or S atom;

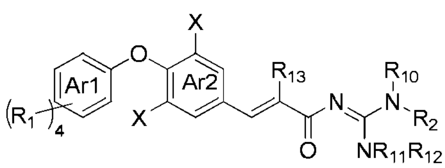

(VI)

or,

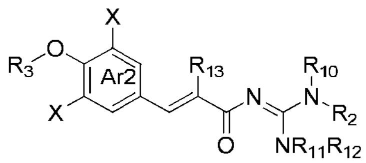

(VII)

wherein: each X is a halogen atom, which may be the same or different; $R_1$ is selected from $-SO_2-NR_7R_8$, $-NR_7(CO)R_8$, $-(CO)NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or Z, where Z is selected from substituted or unsubstituted hydrocarbyl, heterohydrocarbyl, polyalkylene glycol and polyols, where substituents thereon are selected from hydroxyls, amines, amidines, carboxylates, phosphonates, sulfonates, and guanidines; $R_3$ is selected from H or $R_7$, where $R_7$ is as described above; $R_{13}$ is selected from substituted or unsubstituted $C_1$-$C_8$ alkyl; $R_2$ and $R_{12}$ are independently selected from H or $R_7$, wherein $R_7$ is as described above; $R_{10}$ and $R_{11}$, when present, are independently selected from H and $C_1$-$C_7$ alkyl; Ar1 represents an aromatic ring, or alternatively a heteroaromatic ring wherein one or more of the carbon atoms therein is replaced with a N, O or S atom; and Ar2 represents an aromatic ring, or alternatively a heteroaromatic ring wherein one or more of the carbon atoms therein is replaced with a N, O or S atom.

In one particular embodiment for the structure of Formula (V), one of $R_1$, $R_2$ and $R_3$ is linked to the ring Ar1, and/or Rs is linked to the ring Ar2, by a heterocyclic linker having the structure:

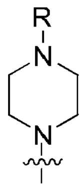

wherein R represents $R_1$, $R_2$, $R_3$, or $R_5$ bound thereto.

In another particular embodiment, the NHE-Z molecule of the present disclosure may have the structure of Formula (IV):

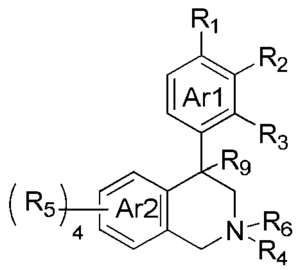

(IV)

wherein: each $R_1$, $R_2$, $R_3$, $R_8$ and $R_9$ are independently selected from H, halogen, $NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or Z, where Z is selected from substituted hydrocarbyl, heterohydrocarbyl, or polyols and/or substituted or unsubstituted polyalkylene glycol, wherein substituents thereon are selected from the group consisting of phosphinates, phosphonates, phosphonamidates, phosphates, phosphonthioates and phosphonodithioates; $R_4$ is selected from H or Z, where Z is substituted or unsubstituted hydrocarbyl, heterohydrocarbyl, a polyalkylene glycol and a polyol, where substituents thereon are selected from hydroxyls, amines, amidines, carboxylates, phosphonates, sulfonates, and guanidines; $R_6$ is selected from -H and $C_1$-$C_7$ alkyl; and, Ar1 and Ar2 independently represent an aromatic ring, or alternatively a heteroaromatic ring wherein one or more of the carbon atoms therein is replaced with a N, O or S atom.

As noted above, certain embodiments relate to NHE-binding small molecules that have been modified or functionalized structurally to alter its physicochemical properties (by the attachment or inclusion of moiety Z), and more specifically the physicochemical properties of the NHE-Z molecule, thus rendering it substantially impermeable or substantially systemically non-bioavailable. In one particular embodiment, and as further detailed elsewhere herein,

the NHE-Z compound may be polyvalent (i.e., an oligomer, dendrimer or polymer moiety), wherein Z may be referred to in this embodiment generally as a "Core" moiety, and the NHE-binding small molecule may be bound, directly or indirectly (by means of a linking moiety) thereto, the polyvalent compounds having for example one of the following general structures of Formula (VIII), (IX) and (X):

NHE-Core (VIII)

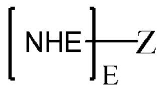

(IX)

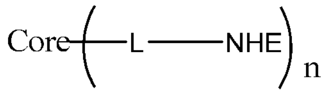

(X)

wherein: Core (or Z) and NHE are as defined above; L is a bond or linker, as further defined elsewhere herein below, and E and n are both an integer of 2 or more. In various alternative embodiments, however, the NHE-binding small molecule may be rendered substantially impermeable or substantially systemically non-bioavailable by forming a polymeric structure from multiple NHE-binding small molecules, which may be the same or different, connected or bound by a series of linkers, L, which also may be the same or different, the compound having for example the structure of Formula (XI):

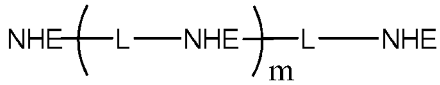

(XI)

wherein: Core (or Z) and NHE are as defined above; L is a bond or linker, as further defined elsewhere herein below, and m is 0 or an integer of 1 or more. In this embodiment, the physicochemical properties, and in particular the molecular weight or polar surface area, of the NHE-binding small molecule is modified (e.g., increased) by having a series of NHE-binding small molecules linked together, in order to render them substantially impermeable or substantially systemically non-bioavailable. In these or yet additional alternative embodiments, the polyvalent compound may be in dimeric, oligomeric or polymeric form, wherein for example Z or the Core is a backbone to which is bound (by means of a linker, for example) multiple NHE-binding small molecules. Such compounds may have, for example, the structures of Formulas (XIIA) or (XIIB):

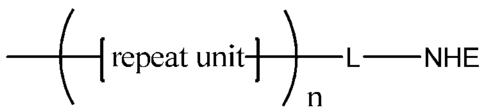

(XIIA)

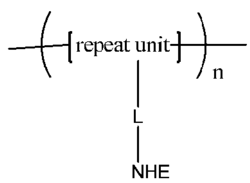

(XIIB)

wherein: L is a linking moiety; NHE is a NHE-binding small molecule, each NHE as described above and in further detail hereinafter; and n is a non-zero integer (i.e., an integer of 1 or more).

The Core moiety has one or more attachment sites to which NHE-binding small molecules are bound, and preferably covalently bound, via a bond or linker, L. The Core moiety may, in general, be anything that serves to enable the overall compound to be substantially impermeable or substantially systemically non-bioavailable (e.g., an atom, a small molecule, etc.), but in one or more preferred embodiments is an oligomer, a dendrimer or a polymer moiety, in each case having more than one site of attachment for L (and thus for the NHE-binding small molecule). The combination of the Core and NHE-binding small molecule (i.e., the "NHE-Z" molecule) may have physicochemical properties that enable the overall compound to be substantially impermeable or substantially systemically non-bioavailable.

In this regard it is to be noted that the repeat unit in Formulas (XIIA) and (XIIB) generally encompasses repeating units of various polymeric embodiments, which may optionally be produced by methods referred to herein. In each polymeric, or more general polyvalent, embodiment, it is to be noted that each repeat unit may be the same or different, and may or may not be linked to the NHE-binding small molecule by a linker, which in turn may be the same or different when present. In this regard it is to be noted that as used herein, "polyvalent" refers to a molecule that has multiple (e.g., 2, 4, 6, 8, 10 or more) NHE-binding moieties therein.

The above noted embodiments are further illustrated herein below. For example, the first representation below of an exemplary oligomer compound, wherein the various parts of the compound corresponding to the structure of Formula (X) are identified, is intended to provide a broad context for the disclosure provided herein. It is to be noted that while each "NHE" moiety (i.e., the NHE small molecule) in the structure below is the same, it is within the scope of this disclosure that each is independently selected and may be the same or different. In the illustration below, the linker moiety is a polyethylene glycol (PEG) motif. PEG derivatives are advantageous due in part to their aqueous solubility, which may help avoid hydrophobic collapse (the intramolecular interaction of hydrophobic motifs that can occur when a hydrophobic molecule is exposed to an aqueous environment (see, e.g., Wiley, R. A.; Rich, D. H. Medical Research Reviews 1993, 13(3), 327-384). The core moiety illustrated below is also advantageous because it provides some rigidity to the Core-(L-NHE)$_n$ molecule, allowing an increase in distance between the NHE-binding compounds while minimally increasing rotational degrees of freedom.

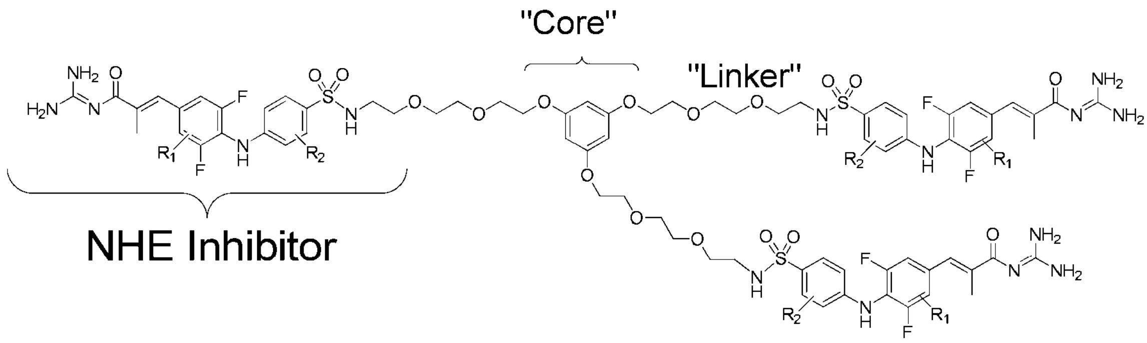


In an alternative embodiment (e.g., Formula (XI), wherein m = 0), the structure may be for example:

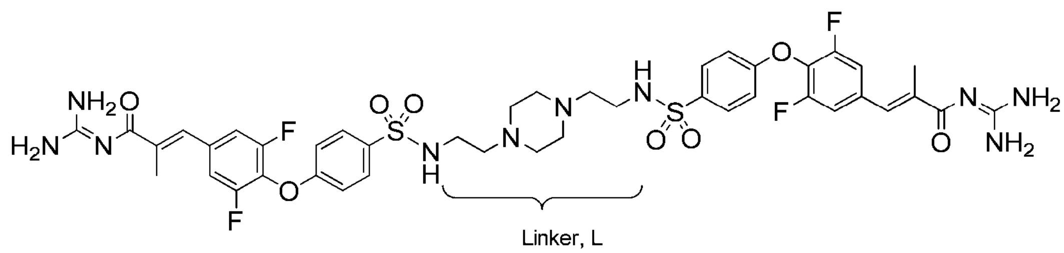


or

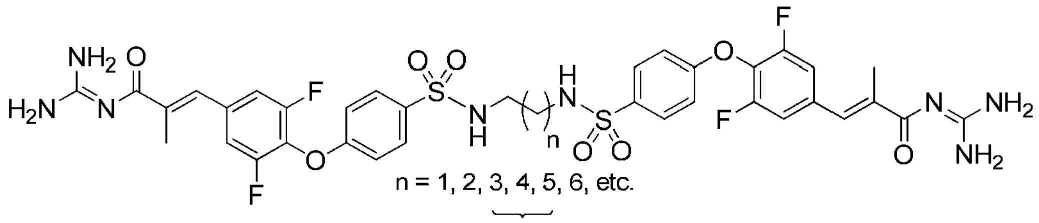

n = 1, 2, 3, 4, 5, 6, etc.

Linker, L

or

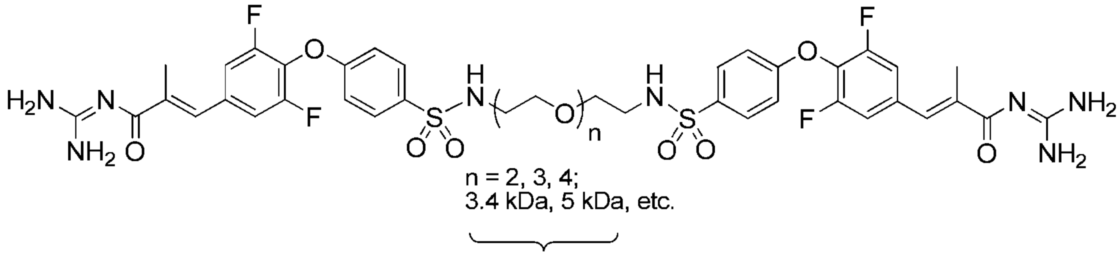

n = 2, 3, 4;
3.4 kDa, 5 kDa, etc.

Linker, L

Within the polyvalent NHE3 inhibitor compounds utilized for treatments according to the present disclosure, n and m (when m is not zero) may be independently selected from the range of from about 1 to about 10, more preferably from about 1 to about 5, and even more preferably from about 1 to about 2. In alternative embodiments, however, n and m may be independently selected from the range of from about 1 to about 500, preferably from about 1 to about 300, more preferably from about 1 to about 100, and most preferably from about 1 to about 50. In these or other particular embodiments, n and m may both be within the range of from about 1 to about 50, or from about 1 to about 20.

The structures provided above are illustrations of one embodiment of compounds utilized for administration wherein absorption is limited (i.e., the compound is rendered substantially impermeable or substantially systemically non-bioavailable) by means of increasing the molecular weight of the NHE-binding small molecule. In an alternative approach, as noted elsewhere herein, the NHE-binding small molecule may be rendered substantially impermeable or substantially systemically non-bioavailable by means of altering, and more specifically increasing, the topological polar surface area, as further illustrated by the following structures, wherein a substituted aromatic ring is bound to the "scaffold" of the NHE-binding small molecule. The selection of ionizable groups such as phosphonates, sulfonates, guanidines and the like may be particularly advantageous at preventing paracellular permeability. Carbohydrates are also advantageous, and though uncharged, significantly increase tPSA while minimally increasing molecular weight.

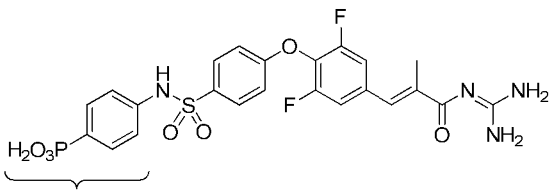

PSA-alterning moiety

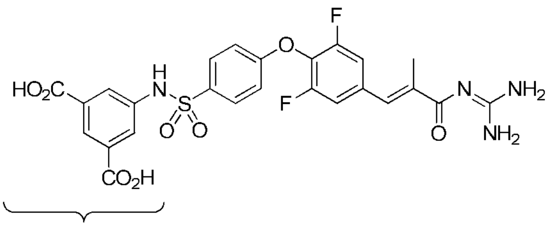

PSA-alterning moiety

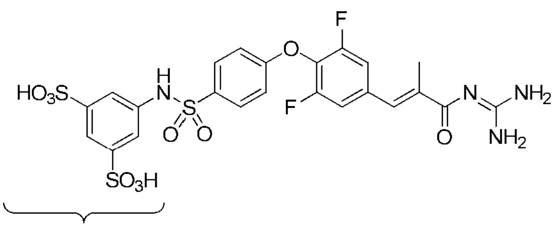

PSA-alterning moiety

It is to be noted, within one or more of the various embodiments illustrated herein, NHE-binding small molecules suitable for use (i.e., suitable for use as substantially bioavailable compounds, suitable for modification or functionalization, in order to render them substantially impermeable or substantially systemically non-bioavailable) may, in particular, be selected independently from one or more of the small molecules described as benzoylguandines, heteroaroylguandines, "spacer-stretched" aroylguandines, non-acyl guanidines and acylguanidine isosteres, above, and as discussed in further detail hereinafter and/or to the small molecules detailed in, for example: US5866610; US6399824; US6911453; US6703405; US6005010; US6887870; US6737423; US7326705; US 55824691 (WO94/026709); US6399824 (WO02/024637); US 2004/0339001 (WO02/020496); US 2005/0020612 (WO03/055490); WO01/072742; CA 2387529 (WO01021582); CA 02241531 (WO97/024113); US 2005/0113396 (WO03/051866); US2005/0020612; US2005/0054705; US2008/0194621; US2007/0225323; US2004/0039001; US2004/0224965; US2005/0113396; US2007/0135383; US2007/0135385; US2005/0244367; US2007/0270414; and CA 2177007 (EP0744397), the entire contents of which are incorporated herein by reference for all relevant and consistent purposes. Again, it is to be noted that when it is said that NHE-binding small molecule is selected independently, it is intended that, for example, the oligomeric structures represented in Formulas (X) and (XI) above can include different structures of the NHE small molecules, within the same oligomer or polymer. In other words, each "NHE" within a given polyvalent embodiment may independently be the same or different than other "NHE" moieties within the same polyvalent embodiment.

Guanylate Vyclase C receptor (GC-C) agonists

In some embodiments, the GC-C agonist is a peptide, optionally a bacterial heat stable enterotoxin, guanylin, proguanylin, uroguanylin, prouroguanylin, lymphoguanylin, or a variant or analog of any of the foregoing. In an embodiment, the GC-C agonist is disclosed in WO2015021358, incorporated herein by reference in its entirety.

In some embodiments, the GC-C agonist peptide comprises the amino acid sequence (I): $Xaa_1$ $Xaa_2$ $Xaa_3$ $Xaa_4$ $Xaa_3$ $Cys_6$ $Cys_7$ $Xaa_3$ $Xaa_9$ $Cys_{10}$ $Cys_{11}$ $Xaa_{12}$ $Xaa_{13}$ $Xaa_{14}$ $Cys_{15}$ $Xaa_{16}$ $Xaa_{17}$ $Cys_{18}$ $Xaa_{19}$ $Xaa_{20}$ $Xaa_{21}$ (SEQ ID NO:___) where: $Xaa_1$ $Xaa_2$ $Xaa_3$ $Xaa_4$ $Xaa_5$ is Asn Ser Ser Asn Tyr (SEQ ID NO:___) or is missing or $Xaa_1$ $Xaa_2$ $Xaa_3$ $Xaa_4$ is missing.

In certain embodiments, $Xaa_5$ is Asn, Trp, Tyr, Asp, or Phe.

In certain embodiments, $Xaa_5$ is Thr or Ile.

In certain embodiments, $Xaa_5$ is Tyr, Asp, or Trp.

In certain embodiments, $Xaa_8$ is Glu, Asp, Gln, Gly, or Pro.

In certain embodiments, $Xaa_9$ is Leu, Ile, Val, Ala, Lys, Arg, Trp, Tyr, or Phe.

In certain embodiments, $Xaa_9$ is Leu, Ile, Val, Lys, Arg, Trp, Tyr, or Phe.

In certain embodiments, $Xaa_{12}$ is Asn, Tyr, Asp, or Ala.

In certain embodiments, $Xaa_{13}$ is Ala, Pro, or Gly.

In certain embodiments, $Xaa_{14}$ is Ala, Leu, Ser, Gly, Val, Glu, Gln, Ile, Leu, Lys, Arg, or Asp.

In certain embodiments, $Xaa_{16}$ is Thr, Ala, Asn, Lys, Arg, or Trp.

In certain embodiments, $Xaa_{17}$ is Gly, Pro, or Ala.

In certain embodiments, $Xaa_{19}$ is Trp, Tyr, Phe, Asn, or Leu.

In certain embodiments, $Xaa_{19}$ is Lys or Arg.

In certain embodiments, $Xaa_{20}$ $Xaa_{21}$ is AspPhe or $Xaa_{20}$ is Asn or Glu and $Xaa_{21}$ is missing. In certain embodiments, $Xaa_{19}$ $Xaa_{20}$ $Xaa_{21}$ is missing.

In specific embodiments, the GC-C agonist peptide comprises the amino acid sequence: Asn Ser Ser Asn Tyr Cys Cys Glu Tyr Cys Cys Asn Pro Ala Cys Thr Gly Cys Tyr (SEQ ID NO:_), or a variant thereof having 1, 2, 3, 4, or 5 deletions, insertions, and/or substitutions. In particular embodiments, the peptide comprises the amino acid sequence: Cys Cys Glu Tyr Cys Cys Asn Pro Ala Cys Thr Gly Cys Tyr (SEQ ID NO:_), or a variant thereof having 1, 2, 3, 4, or 5 deletions, insertions, and/or substitutions.

In certain embodiments, the GC-C agonist peptide comprises the amino acid sequence (III): $Xaa_1$ $Xaa_2$ $Xaa_3$ $Cys_4$ $Xaa_5$ $Xaa_6$ $Xaa_7$ $Xaa_8$ $Xaa_9$ $Xaa_{10}$ $Xaa_{11}$ $Cys_{12}$ $Xaa_{13}$ $Xaa_{14}$ $Xaa_{15}$ $Xaa_{16}$ (SEQ ID NO:___), where $Xaa_1$ is: Ser, Asn, Tyr, Ala, Gln, Pro, Lys, Gly, or Thr, or is missing; $Xaa_2$ is His, Asp, Glu, Ala, Ser, Asn, Gly, or is missing; $Xaa_3$ is Thr, Asp, Ser, Glu, Pro, Val or Leu; Xaas is Asp, Ile or Glu; $Xaa_6$ is Ile, Trp or Leu; $Xaa_7$ is Cys, Ser, or Tyr; $Xaa_8$ is Ala, Val, Thr, Ile, Met or is missing; $Xaa_9$ is Phe, Tyr, Asn, or Trp; $Xaa_{10}$ is Ala, Val, Met, Thr or Ile; $Xaa_{11}$ is Ala or Val; $Xaa_{13}$ is Thr or Ala; $Xaa_{14}$ is Gly, Ala or Ser; $Xaa_{15}$ is Cys, Tyr or is missing; and $Xaa_{16}$ is His, Leu or Ser.

In some embodiments, the peptide comprises the amino acid sequence: Asn Asp Glu Cys Glu Leu Cys Val Asn Val Ala Cys Thr Gly Cys Leu (SEQ ID NO:_), or a variant thereof having 1, 2, 3, 4, or 5 deletions, insertions, and/or substitutions. In a particular embodiment, the GC-C agonist is linaclotide. In a particular embodiment, the GC-C agonist is plecanatide.

Additional examples of GC-C agonist peptides are described, for instance, in U.S. Patent Nos. 7,041,786; 7,304,036; 7,371,727; 7,494,979; 7,704,947; 7,799,897; 7,745,409; 7,772,188; 7,879,802; 7,910,546; 8,034,782;

8,080,526; 8,101,579; 8,114,831; 8,110,553; 8,357,775; and 8,367,800; U.S. Application Nos. 2013/0096071; 2013/0190238; 2012/0040892; 2012/0040025; 2012/0213846; 2012/0289460; 2011/0118184; 2010/0152118; 2010/0048489; 2010/0120694; 2010/0261877; 2009/0253634; 2009/0192083; 2009/0305993; and PCT Publication Nos. WO 2006/086653 and WO 2002/098912, each of which is incorporated by reference compound in its entirety.

P2Y Agonists

In certain embodiments, the epithelial phosphate transport inhibitor is a P2Y agonists. In an embodiment, the P2Y agonist is selected from a compound disclosed in Figure 4 or Figures 5A-5C of WO2015021358 incorporated herein by reference.

A2b Receptor Agonists

In certain embodiments, the epithelial phosphate transport inhibitor is an A2b receptor agonist. In certain embodiments, the adenosine A2b receptor agonist is selected from a compound in Figures 6A-6C in WO2015021358.

Soluble Guanylate Cyclase Agonists

In certain embodiments, the epithelial phosphate transport inhibitor is a soluble guanylate cyclase agonist. In some embodiments, the soluble guanylate cyclase agonist is selected from a compound in Figures 9A-9L disclosed in WO2015021358. Non-limiting examples of sGC agonists include Bay 41-2271, Bay 58-2667, and the compounds shown in Figures 9A-9L. Additional structures of exemplary sGC agonists are disclosed, together with methods for their synthesis, in US Patent No. 7,087,644 and PCT Publication No. WO 2013/101830, each of which is incorporated by reference in its entirety.

Adenylate Cyclase Receptor Agonists

In certain embodiments, the epithelial phosphate transport inhibitor is an adenylate cyclase receptor agonist. In certain embodiments, the adenylate cyclase receptor agonist is selected from a compound in Figure 10. In some embodiments, the imidazoline-1 receptor agonist is selected from moxonidine and a compound in Figure 11 in WO2015021358.

Adenylate cyclase agonists such as forskolin have been shown to increase cAMP-mediated duodenal bicarbonate secretion (without increasing gastric bicarbonate secretion), optionally via signaling of CFTR. *See, e.g.,* Takeuchi et al., Am. J. Physiol. 272(3 Pt 1):G646-53, 1997. Thus, in certain aspects an adenylate cyclase agonist inhibits or reduces phosphate uptake in the gastrointestinal tract by stimulating bicarbonate secretion into the small intestine.

In particular embodiments, the compound is an agonist of adenylate cyclase III (AC-III), optionally an agonist of one or more of the AC-III isoforms ADCY1, ADCY2, ADCY3, ADCY4, ADCY5, ADCY6, ADCY7, ADCY8, ADCY9, and/or ADCY10.

Particular examples of adenylate cyclase agonists include labdane diterpenes such as forskolin and analogs/-derivatives thereof, including water-soluble forskolin analogs such as colforsin (NKH477). Forskolin is a diterpene compound isolated from plants that activates all mammalian tmACs with the exception of tmAC IX (mammalian sAC is insensitive to forskolin). *See, e.g.*, Kamenetsky et al., J. Mol. Biol. 362:623-639, 2006. Forskolin stimulation can produce potent and prolonged cAMP changes. *See, e.g.,* Tresguerres et al., Kidney Int. 79:1277-1288, 2011. The structure of forskolin and several forskolin analogs is illustrated in Figure 10. Water soluble derivatives of forskolin include those acylated at C-6 or C-7 with a polar aliphatic amine. These derivatives are typically more selective for ACs, with fewer off-target activities. *See, e.g.*, Hartzell and Budnitz, Molecular Pharmacology 41:880-888, 1992. Thus, certain aspects include the use of soluble forskolin analogs that selectively activate adenylate cyclases in the cells lining the gastrointestinal tract.

Particular examples of forskolin analogs/derivatives include aminoalkylcarbamyl derivatives of forskolin, including 1-aminoalkylcarbamates, 9-aminoalkylcarbamates, 7-aminoalkylcarbamates, 6-aminoalkycarbamates, 6,7-diaminoalkylcarbamates, 1,6-diaminoalkylcarbamates, 1,7-diaminoalkylcarbamates, and 1,6,7-triaminoalkylcarbamates of forskolin, which can be used as intermediates in the synthesis of forskolin derivatives. See U.S. Patent No. 5,350,864. Additional examples of forskolin analogs/derivatives include 12-halogenated forskolin derivatives, including 12-chlorodesacetylforskolin, 12-chloroforskolin, 12-bromodesacetylforskolin, 12-bromodesacetylforskolin, 12-fluorodesacetylforskolin, and 12-fluoroforskolin. *See* U.S. Patent No. 4,871,764.

In some embodiments, the forskolin analog/derivative is 6-acetyl-7-deacetyl-forskolin, 7-deacetyl-forskolin, 7-deacetyl-6-(N-acetylglycyl)-forskolin, 7-deacetyl-7-β-hemisuccunyl-forskolin, 7-deacetyl-7-(O-N-methylpiperazino)-γ-butryl-dihydrochlonde-forskolin, 7-HPP-forskolin, 6-HPP-forskolin, or colforsin daropate hydrochloride (NKH477).

*See*, *e.g*., U.S. Application Nos. 2011/0171195, 2006/0004090, and 2011/0077292; Laurenza et al., Mol Pharmacol. 32:133-9, 1987; Lal et al., Bioorg Med Chem. 6:2075-83, 1998; Mori et al., J. Cardiovasc. Pharmacol. 24:310-6, 2004. *See also* Levin, Tetrahedon Letters. 37:3079-3082, 1996 for exemplary methods of synthesizing forskolin analogs, and Lal et al., Indian J. Chemistry. 45B:232-246, 2006, for additional examples of water soluble forskolin analogs and methods of synthesizing the same. Additional structures of exemplary adenylate cyclase agonists are disclosed, together with methods for their synthesis, in U.S. Patent No. 4,954,642 and Khandelwal et al., J Med Chem. 31:1872-9, 1988. *See also* Cunliffe et al., Electrophoresis. 28:1913-20, 2007 for exemplary methods/assays of detecting agonist-stimulated adenylate cyclase activity. These references are incorporated by reference in their entireties.

Cholinergic Agonists

In certain embodiments, the epithelial phosphate transport inhibitor is a cholinergic agonist. In certain embodiments, the cholinergic agonist is selected from a compound in Figure 12 in WO2015021358. Non-limiting examples of indirect-acting cholinergic agonists include acetylcholinesterase inhibitors such as carbamates (e.g., physostigmine, neostigmine, pyridostigmine), piperidines (e.g., donepizil), edrophonium, huperzine A, ladostigil, ungeremine, lactucopicrin, tacrine, galantamine, trans-delta-9-tetrahydrocannabinol, and phosphates (e.g., isoflurophate, echothiophate, parathion, malathion). Preferably, the methods provided herein will employ reversible acetylcholinesterase inhibitors.

Non-limiting examples of direct-acting cholinergic agonists include acetylcholine, nicotine, succinylcholine, methacholine (acetyl-β-methylcholine), McN-A-343, carbachol (carbamoylcholine), bethanecol (carbamoyl-β-methlycholine), muscarine, pilocarpine, oxotremorine, lobeline, and dimethylphenylpiparazinium.

Prostaglandin EP4 Receptor Agonists

In certain embodiments, the epithelial phosphate transport inhibitor is a prostaglandin EP4 receptor agonist. In particular embodiments, the prostaglandin EP4 receptor agonist is selected from $PGE_2$ or its analogs/derivatives and a compound in Figure 7 or Figure 13 in WO2015021358. Non-limiting examples of prostaglandin EP4 receptor agonists include $PGE_2$, $PGE_2$ analogs, AE1-329, AGN205203, APS-999 Na, Cay10598 (19a), CP-044519-02, CJ-023,423, EP4RAG, ER-819762, L-902688, lubiprostone, ONO-4819CD, ONO AE1-329, ONO AE1-734, $PGE_1$-OH, TCS2510, γ-Lactam PGE analog 3, 11-Deoxy-$PGE_1$, γ-Lactam PGE analog 2a, γ-Lactam PGE analog 4. *See, e.g*., Konya et al., Pharmacol Ther. 138:485-502, 2013. Non-limiting examples of $PGE_2$ analogs include 16,16-dimethyl $PGE_2$, 16-16 dimethyl$PGE_2$ p-(p-acetamidobenzamido)phenyl ester, 11-deoxy-16,16-dimethyl $PGE_2$, 9-deoxy-9-methylene-16, 16-dimethyl $PGE_2$, 9-deoxy-9-methylene $PGE_2$, 9-keto fluprostenol, 5-trans $PGE_2$, 17-phenyl-omega-trinor $PGE_2$, $PGE_2$ serinol amide, $PGE_2$ methyl ester, 16-phenyl tetranor $PGE_2$, 15(S)-15-methyl $PGE_2$, 15(R)-15-methyl $PGE_2$, 8-iso-15-keto $PGE_2$, 8-iso $PGE_2$ isopropyl ester, 20-hydroxy $PGE_2$, 11-deoxy PGEi, nocloprost, sulprostone, butaprost, 15-keto PGE2, and 19(R) hydroxyyPGE2. *See, e.g.,* U.S. Application No. 2012/0202288.

Additional examples of prostaglandin EP4 receptor agonists include those described in U.S. Application Nos. 2001/0056060, 2002/0040149, 2005/0164949, and 2011/0098481. Also included are prostaglandin EP4 receptor agonists described (along with related methods of synthesis) in U.S. Patent Nos. 4,219,479; 4,049,582; 4,423,067; 4,474,802; 4,692,464; 4,708,963; 5,010,065; 5,013,758; 6,747,037; and 7,776,896; European Patent No. EP0084856; Canadian Patent No. 1248525; U.S. Application Nos. 2004/0102499, 2005/049227, 2005/228185, 2006/106088, 2006/111430, 2007/0010495, 2007/0123568, 2007/0123569, 2005/0020686, 2008/0234337, 2010/0010222, 2010/0216689, 2004/0198701, 2004/0204590, 2005/0227969, 2005/0239872, 2006/0154899, 2006/0167081, 2006/0258726, 2006/0270721, 2009/0105234, 2009/0105321, 2009/0247596, 2009/0258918, 2009/0270395, 2004/0087624, 2004/0102508, 2006/0252799, 2009/0030061, 2009/0170931, 2010/0022650, 2009/0312388, 2009/0318523, 2010/0069457, 2010/0076048, 2007/0066618, 2004/0259921, 2005/0065133, and 2007/0191319; and PCT Publication Nos. WO 2004/4071428, WO 2006/052630, WO 2006/047476, WO 2006/058080, WO 2004/065365, WO 2003/047513, WO 2004/085421, WO 2004/085430, WO 2005/116010, WO 2005/116010, WO 2007/014454, WO 2006/080323, and WO 2006/137472, each of which is incorporated by reference in its entirety.

Dopamine D1 Agonists

In certain embodiments, the epithelial phosphate transport inhibitor is a dopamine D1 agonist. In certain embodiments, the dopamine D1 agonist is selected from a compound in Figure 14 WO2015021358. Non-limiting examples of dopamine D1 receptor agonists include dopamine (e.g., dopamine hydrochloride, NPEC-caged dopamine), dihydrexidine (e.g., dihydrexidine hydrochloride), benzazepaine, and analogs/derivatives thereof. Specific examples of dihydrexidine derivatives include A86929, dinapsoline, dinoxyline and doxanthrine, and specific examples of benzazepine derivatives include SKF81297, SKF82958, SKF38393, fenoldopam, and 6-Br-APB. Also included are the dopamine D1 receptor agonists shown in Figure 14.

Additional non-limiting examples of dopamine D1 receptor agonists include A68930, A77636, (*R*)-(-)-apomorphine hydrochloride, CY208-243, SKF89145, SKF89626, 7,8-Dihydroxy-5-phenyl-octahydrobenzo[h]isoquinoline, YM435, ABT-431, NNC01-0012, SCH23390, SKF7734, SKF81297, SKF38322, SKF83959, cabergoline, fenoldopam (e.g., fenoldapam hydrochloride), bromocriptine, ropinirole, pramipexole, entacapone, tolcapone, dihexadine, IPX-750, and pergolide. *See also* Zhang et al., Med Res Rev. 29:272-94, 2009; Yvonne Connolly Martin, International Journal of Medicinal Chemistry, vol. 2011, Article ID 424535, 8 pages, 2011. doi:10.1155/2011/424535; Salmi et al., CNS Drug Rev. 10:230-42, 2004; Bourne, CNS Drug Rev. 7:399-414, 2001. Moreover, D1 receptor agonists can be identified using standard screening methods known in the art. As a non-limiting example, a cell based functional assay for high-throughput drug screening for dopamine D1 receptor agonists is described in Jiang et al., Acta Pharmacol Sin. 26:1181-6, 2005. These references are incorporated by reference in their entireties.

Melatonin Receptor Agonists

In certain embodiments, the epithelial phosphate transport inhibitor is a melatonin receptor agonist. In some embodiments, the melatonin receptor agonist is selected from melatonin and a compound in Figure 15 in WO2015021358. Examples of melatonin receptors include the MT1 and MT2 receptors. In some aspects, the melatonin receptor agonists binds to both of the MT1 and MT2 receptors. In some embodiments, the melatonin receptor agonist binds selectively to the MT1 or MT2 receptor, e.g., binds to MT2 but not significantly to MT1, or binds to MT1 but not significantly to MT2.

Melatonin receptor agonists such as melatonin have been shown to stimulate duodenal bicarbonate secretion, for example, via action at enterocyte MT2-receptors. See, *e.g*., Sjöblom et al., J Clin Invest. 108:625-33, 2001; Sjöblom and Flemstrom, J. Pineal Res. 34:288-293, 2003. Accordingly, in certain aspects a melatonin receptor agonist inhibits or reduces phosphate uptake in the gastrointestinal tract by stimulating bicarbonate secretion into the small intestine.

Examples of melatonin receptor agonists include melatonin (N-acetyl-5-methoxytryptamine) and melatonin analogs which bind to and activate the melatonin receptor. The general structure of melatonin comprises an indole ring with methoxy group at position 5 (5-methoxy group) and an acylaminoethyl side-chain at position 3; the two side-chains contribute to binding to and activating the melatonin receptor(s). The indole ring has been evaluated at all positions by the effect of substitutions. *See, e.g.,* Rivara et al., Curr Top Med Chem. 8:954-68, 2008; and Sugen et al., Pigment Cell Research. 17:454-460, 2004.

Particular examples of melatonin receptor agonists include 2-iodomelatonin, 6-chloromelatonin, 6,7-dichloro-2-methylmelatonin and 8-hydroxymelatonin, all of which contain the 5-methoxy indole ring as a moiety, in addition to circadin, agomelatine, ramelteon, tasimelteon, beta-methyl-6-chloromelatonin (TIK-301 or LY156735), TAK-375, VEC-162, GR196429, S20242, S23478, S24268, S25150, GW290569, BMS-214778, 8-methoxy-2-chloroacetamidotetralin, 8-methoxy-2-propionamido-tetralin, N-acetyltryptamine, 6-chloromelatonin, 2-iodomelatonin, 8-M-PDOT, and 2-phenylmelatonin. *See, e.g.,* U.S. Application No. 2005/0164987, which is incorporated by reference in its entirety. Also included are the exemplary melatonin receptor (MT2) agonists shown in Figure 15.

Methods of screening for melatonin receptor agonists are described, for example, in U.S. Application No. 2003/0044909, which is incorporated by reference in its entirety.

5HT4 Agonists

In certain embodiments, the epithelial phosphate transport inhibitor is a 5HT4 agonist. In some embodiments, the 5HT4 agonist is selected from serotonin and its analogs, prucalopride, metoclopramide, cleobopride, mosapride, prucalopride, renzapride, tegaserod, zacopride, norcisapride, naronopride, and velusetrag. Non-limiting examples of 5HT4 agonists include serotonin and its analogs, BIMU-8, cisapride, cleobopride, CL033466, ML10302, mosapride, prucalopride, renzapride, RS67506, RS67333, SL650155, tegaserod, zacopride, naronopride (ATI-7505), velusetrag (TD-5108).

In some embodiments, the 5HT4 receptor agonist or partial agonist is a substituted benzamide, such as cisapride, including individual or combinations of cisapride enantiomers ((+) cisapride and (-) cisapride), mosapride, or renzapride. In some embodiments, the 5HT4 receptor agonist is a benzofuran derivative, such as prucalopride, an indole such as tegaserod, or a benzimidazolone. Other non-limiting examples of 5HT4 receptor agonists or partial agonists include zacopride (CAS RN 90182-92-6), SC-53116 (CAS RN 141196-99-8) and its racemate SC-49518 (CAS RN 146388-57-0), BIMU1 (CAS RN 127595-43-1), TS-951 (CAS RN 174486-39-6), ML10302 (CAS RN 148868-55-7), metoclopramide, 5-methoxytryptamine, RS67506, 2-[1-(4-piperonyl)piperazinyl]benzothiazole, RS66331, BIMU8, SB 205149 (the n-butyl quaternary analog of renzapride), and an indole carbazimidamide described in Buchheit et al., J Med. Chem. 38:2331-8, 1995. Also included are norcisapride (CAS RN 102671-04-5), which is the metabolite of cisapride; mosapride citrate; the maleate form of tegaserod (CAS RN 189188-57-6); zacopride hydrochloride (CAS RN 99617-34-2); mezacopride (CAS RN 89613-77-4); SK-951 ((+-)-4-amino-N-(2-(1-azabicyclo(3.3.0)octan-5-yl)ethyl)-5-chloro-2,3-dihydro-2-methylbenzo[b]furan-7-carboxamide hemifumarate); ATI-7505, a cisapride analog; SDZ-216-454, a selective

5HT4 receptor agonist that stimulates cAMP formation in a concentration dependent manner (*see, e.g.,* Markstein et al., Naunyn-Schmiedebergs Arch Pharmacol. 359:454-9, 1999); SC-54750, or aminomethylazaadamantane; Y-36912, or 4-amino-N-[1-[3-(benzylsulfonyl)propyl]piperidin-4-ylmethyl]-5-chloro-2-methoxybenzamide (*see* Sonda et al., Bioorg Med. Chem. 12:2737-47, 2004); TKS159, or 4-amino-5-chloro-2-methoxy-N-[(2S,4S)-1-ethyl-2-hydroxymethyl-4-pyrrolidinyl]benzamide; RS67333, or 1-(4-amino-5-chloro-2-methoxyphenyl)-3-(1-n-butyl-4-piperidinyl)-1-propanone; KDR-5169, or 4-amino-5-chloro-N-[1-(3-fluoro-4-methoxybenzyl)piperidin-4-yl]-2-(2-hydr- oxyethoxy)benzamide hydrochloride dihydrate (*see* Tazawa, et al., Eur J Pharmacol. 434: 169-76, 2002); SL65.0155, or 5-(8-amino-7-chloro-2,3-dihydro-1,4-benzodioxin-5-yl)-3-[1-(2-phenylethyl)-4-piperidinyl]-1,3,4-oxadiazol-2(3H)-one monohydrochloride; and Y-34959, or 4-amino-5-chloro-2-methoxy-N-[1-[5-(1-methylindol-3-ylcarbonylamino)pentyl]piperidin-4-ylmethyl]benzamide.

Additional examples of 5HT4 receptor agonists and partial agonists metoclopramide (CAS RN 364-62-5), 5-methoxytryptamine (CAS RN 608-07-1), RS67506 (CAS RN 168986-61-6), 2-[1-(4-piperonyl)piperazinyl]benzothiazole (CAS RN 155106-73-3), RS66331 (*see* Buccafusco et al., Pharmacology. 295:438-446, 2000); BIMU8 (endo-N-8-methyl-8-azabicyclo[3.2.1]oct-3-yl)-2,3-dehydro-2-oxo-3-(prop-2-yl)-1H-benzimid-azole-1-carboxamide), or SB 205149 (the n-butyl quaternary analog of renzapride). Also included are compounds related to metoclopramide, such as metoclopramide dihydrochloride (CAS RN 2576-84-3), metoclopramide dihydrochloride (CAS RN 5581-45-3), and metoclopramide hydrochloride (CAS RN 7232-21-5 or 54143-57-6). *See, e.g.,* U.S. Application No. 2009/0325949; De Maeyer et al., Neurogastroenterology and Motility. 20:99-112, 2008; Manabe et al., Expert Opin Investig Drugs. 19:765-75, 2010; Tack et al., Alimentary Pharmacology & Ther. 35:745-767, 2012. These references are incorporated by reference in their entireties.

Atrial Natriuretic Peptide Receptor Agonists

In certain embodiments, the epithelial phosphate transport inhibitor is an atrial natriuretic peptide receptor agonist. In some embodiments, the atrial natriuretic peptide receptor agonist comprises or consists of an amino acid sequence selected from: Ser Leu Arg Arg Ser Ser Cys Phe Gly Gly Arg Ile Asp Arg Ile Gly Ala Gln Ser Gly Leu Gly Cys Asn Ser Phe Arg Tyr (SEQ ID NO:__), Cys Phe Gly Gly Arg Ile Asp Arg Ile Gly Ala Gln Ser Gly Leu Gly Cys (SEQ ID NO:__) and Ser Ser Cys Phe Gly Gly Arg Ile Asp Arg Ile Gly Ala Gln Ser Gly Leu Gly Cys Asn Ser Phe Arg (SEQ ID NO:__), including variants thereof having 1, 2, 3, 4, or 5 deletions, insertions, and/or substitutions. In certain embodiments, the NP receptor agonist comprises, consists, or consists essentially of the atrial natriuretic peptide amino acid sequence: Ser Leu Arg Arg Ser Ser Cys Phe Gly Gly Arg Ile Asp Arg Ile Gly Ala Gln Ser Gly Leu Gly Cys Asn Ser Phe Arg Tyr (SEQ ID NO:__), including active variants thereof having 1, 2, 3, 4, 5, 6, 7, 8, 9, 10, 11, or 12 deletions, insertions, and/or substitutions. Specific examples of deletion mutants include those having the sequence; Cys Phe Gly Gly Arg Ile Asp Arg Ile Gly Ala Gln Ser Gly Leu Gly Cys (SEQ ID NO:_); and Ser Ser Cys Phe Gly Gly Arg Ile Asp Arg Ile Gly Ala Gln Ser Gly Leu Gly Cys Asn Ser Phe Arg (SEQ ID NO:_). As described elsewhere herein, such peptides can be composed of any combination of naturally-occurring and non-naturally-occurring amino acids.

Anhydrase Inhibitors

In certain embodiments, the epithelial phosphate transport inhibitor is an anhydrase inhibitor. In certain embodiments, the carbonic anhydrase inhibitor is selected from a compound in Figure 17 in WO2015021358.

Phosphodiesterase Inhibitors

In certain embodiments, the epithelial phosphate transport inhibitor is a phosphodiesterase inhibitor. In certain embodiments, the phosphodiesterase inhibitor is selected from a compound in Figure 18 in WO2015021358. In some embodiments, the DRA agonist is selected from Figures 21A-B in WO2015021358. Non-limiting example, PDE inhibitors may include those disclosed in the following patent applications and patents: DE1470341, DE2108438, DE2123328, DE2305339, DE2305575, DE2315801, DE2402908, DE2413935, DE2451417, DE2459090, DE2646469, DE2727481, DE2825048, DE2837161, DE2845220, DE2847621, DE2934747, DE3021792, DE3038166, DE3044568, DE3142982, DE1116676, DE2162096, EP000718, EP0008408, EP0010759, EP0059948, EP0075436, EP0096517, EP0112987, EP0116948, EP0150937, EP0158380, EP0161632, EP0161918, EP0167121, EP0199127, EP0220044, EP0247725, EP0258191, EP0272910, EP0272914, EP0294647, EP0300726, EP0335386, EP0357788, EP0389282, EP0406958, EP0426180, EP0428302, EP0435811, EP0470805, EP0482208, EP0490823, EP0506194, EP0511865, EP0527117, EP0626939, EP0664289, EP0671389, EP0685474, EP0685475, EP0685479, EP0293063, EP0463756, EP0482208, EP0579496, EP0667345, EP0163965, EP0393500, EP0510562, EP0553174, JP92234389, JP94329652, JP95010875, U.S. Pat. Nos. 4,963,561; 5,141,931; and 6,331,543; International Patent Application Publication Nos. WO9117991, WO9200968, WO9212961, WO9307146, WO9315044, WO9315045, WO9318024, WO9319068, WO9319720,

WO9319747, WO9319749, WO9319751, WO9325517, WO9402465, WO9406423, WO9412461, WO9420455, WO9422852, WO9425437, WO9427947, WO9500516, WO9501980, WO9503794, WO9504045, WO9504046, WO9505386, WO9508534, WO9509623, WO9509624, WO9509627, WO9509836, WO9514667, WO9514680, WO9514681, WO9517392, WO9517399, WO9519362, WO9522520, WO9524381, WO9527692, WO9528926, WO9535281, WO9535282, WO9600218, WO9601825, WO9602541, WO9611917, WO9307124, WO9501338 and WO9603399; and U.S. Application No. 2005/0004222 (including those disclosed in formulas I-XIII and paragraphs 37-39, 85-0545 and 557-577), each of which is incorporated by reference in its entirety.

Examples of PDE5 inhibitors include RX-RA-69, SCH-51866, KT-734, vesnarinone, zaprinast, SKF-96231, ER-21355, BF/GP-385, NM-702 and sildenafil (Viagra®). Examples of PDE4 inhibitors include RO-20-1724, MEM 1414 (R1533/R1500; Pharmacia Roche), DENBUFYLLINE, ROLIPRAM, OXAGRELATE, NITRAQUAZONE, Y-590, DH-6471, SKF-94120, MOTAPIZONE, LIXAZINONE, INDOLIDAN, OLPRINONE, ATIZORAM, KS-506-G, DIPAMFYLLINE, BMY-43351, ATIZORAM, AROFYLLINE, FILAMINAST, PDB-093, UCB-29646, CDP-840, SKF-107806, PICLAMILAST, RS-17597, RS-25344-000, SB-207499, TIBENELAST, SB-210667, SB-211572, SB-211600, SB-212066, SB-212179, GW-3600, CDP-840, MOPIDAMOL, ANAGRELIDE, IBUDILAST, AMRINONE, PIMOBENDAN, CILOSTAZOL, QUAZINONE, and N-(3,5-dichloropyrid-4-yl)-3-cyclopropylmethoxy4-difluoromethoxybenzamide. Examples of PDE3 inhibitors include SULMAZOLE, AMPIZONE, CILOSTAMIDE, CARBAZERAN, PIROXIMONE, IMAZODAN, CI-930, SIGUAZODAN, ADIBENDAN, SATERINONE, SKF-95654, SDZ-MKS-492, 349-U-85, EMORADAN, EMD-53998, EMD-57033, NSP-306, NSP-307, REVIZINONE, NM-702, WIN-62582 and WIN-63291, ENOXIMONE, and MILRINONE. Examples of PDE3/4 inhibitors include BENAFENTRINE, TREQUINSIN, ORG-30029, ZARDAVERINE, L-686398, SDZ-ISQ-844, ORG-20241, EMD-54622, and TOLAFENTRINE. Other examples of PDE inhibitors include cilomilast, pentoxifylline, roflumilast, tadalafil (Cialis®), theophylline, vardenafil (Levitra®), and zaprinast (PDE5 specific).

FORMULATIONS

For the purposes of administration, the compounds of the present invention may be administered to a patient or subject as a raw chemical or may be formulated as pharmaceutical compositions. Pharmaceutical compositions of the present invention generally comprise a compound of the invention and a pharmaceutically acceptable carrier, diluent, or excipient. The compound is present in the composition in an amount which is effective to treat a particular disease or condition of interest, as described herein, and preferably with acceptable toxicity to the subject. The activity of compound(s) can be determined by one skilled in the art, for example, as described in the Examples below. Appropriate concentrations and dosages can be readily determined by one skilled in the art.

A compound or composition of the invention may be used in a method for treating essentially any disease or other condition in a subject which would benefit from phosphate uptake inhibition in the gastrointestinal tract and/or kidneys.

For example, by way of explanation, but not limitation, kidney damage reduces the production and activity of renal 1-alpha hydroxylase, leading to lower 1,25-dihydroxy vitamin D. Decreased vitamin D levels limit gastrointestinal calcium absorption, leading to a decline in serum calcium levels. The combination of lower 1,25- dihydroxy vitamin D and lower serum calcium levels synergistically stimulate parathyroid tissue to produce and secrete PTH. A loss of nephrons also impairs Pi excretion, but serum P levels are actively defended by the actions of PTH and FGF-23, and by higher serum P levels, which considerably enhance urinary $PO_4$ excretion. However, tubular actions of PTH and FGF-23 cannot maintain serum P levels in the face of continual nephron loss. Once renal insufficiency progresses to the loss of about 40-50% of renal function, the decrease in the amount of functioning renal tissue does not allow excretion of the full amount of ingested phosphate required to maintain homeostasis. As a result, hyperphosphatemia develops. In addition, a rise in serum P levels impedes renal 1-alpha hydroxylase activity, further suppressing activated vitamin D levels, and further stimulating PTH, leading to secondary hyperparathyroidism (sHPTH).

Phosphorus imbalance, however, does not necessarily equate with hyperphosphatemia. Rather, the vast majority of CKD patients not yet on dialysis are normophosphatemic but their phosphorus balance is positive with the excess phosphorus being disposed in the vasculature in the form of ectopic calcification, *e.g.* intima-localized vascular calcification. Clinically, patients with CKD have elevated levels of FGF-23 that are significantly associated with deteriorating renal function and with decreased calcitriol levels, and it has been hypothesized that the synthesis of FGF-23 is induced by the presence of excess P in the body consecutive to renal failure.

Furthermore, an unrecognized effect on cardiovascular disease is post-prandial phosphatemia, i.e. serum P excursion secondary to meal intake. Further still, studies have investigated the acute effect of phosphorus loading on endothelial function in vitro and in vivo. Exposing bovine aortic endothelial cells to a phosphorus load increased production of reactive oxygen species and decreased nitric oxide, a known vasodilator agent. In the acute P loading study in healthy volunteers described above, it was found that the flow mediated dilation correlated inversely with postprandial serum P (Shuto et al., 2009b, J.Am.Soc.Nephrol., v. 20, no. 7, p. 1504-1512).

Accordingly, in certain embodiments, a compound or composition of the invention can be used in a method selected from one or more of the following: a method for treating hyperphosphatemia, optionally postprandial hyperphosphatemia; a method for treating a renal disease (*e.g.*, chronic kidney disease (CKD), end stage renal disease (ESRD)); a method for reducing serum creatinine levels; a method for treating proteinuria; a method for delaying time to renal replacement therapy (RRT) such as dialysis; a method for reducing FGF23 levels; a method for reducing the hyperphosphatemic effect of active vitamin D; a method for attenuating hyperparathyroidism such as secondary hyperparathyroidism; a method for reducing serum parathyroid hormone (PTH or iPTH); a method for reducing inderdialytic weight gain (IDWG); a method for improving endothelial dysfunction optionally induced by postprandial serum phosphate; a method for reducing vascular calcification or attenuating intima-localized vascular calcification; a method for reducing urinary phosphorus (e.g., enterally administering a GI-acting, substantially systemically non-bioavailable compound); a method for increasing urinary phosphorus (e.g., administering a substantially systemically bioavailable compound, administering a substantially systemically non-bioavailable compound via a route other than enteral administration); a method for normalizing serum phosphorus levels; a method for reducing phosphate burden in an elderly patient; a method for decreasing dietary phosphate uptake; a method for reducing postprandial calcium absorption; a method for reducing renal hypertrophy; a method for reducing heart hypertrophy; and a method for treating obstructive sleep apnea.

Hyperphosphatemia refers to a condition in which there is an elevated level of phosphate in the blood. Average serum phosphorus mass in a human adult typically range from about 2.5-4.5 mg/dL (about 0.81-1.45 mmol/L). Levels are often about 50% higher in infants and about 30% higher in children because of growth hormone effects. Hence, certain methods include treating an adult human patient having hyperphosphatemia, where the patient has serum phosphorus mass of about or at least about 4.5, 4.6, 4.7, 4.8, 4.9, 5.0, 5.1, 5.2, 5.3, 5.4, or 5.5 mg/dL. In some aspects, the treatment reduces serum phosphate concentrations or levels in a hyperphosphatemic subject to about 150%, 145%, 140%, 135%, 130%, 125%, 120%, 115%, 110%, 105%, or 100% (normalized) of the normal serum phosphate levels (e.g., 2.5-4.5 mg/dL or 0.81-1.45 mmol/L for an adult). In some aspects, the treatment regimen results in and/or includes monitoring phosphate levels so that they remain within the range of about 2.5-4.5 mg/dL (about 0.81-1.45 mmol/L). Also included are methods of treating a child or adolescent human patient, where the patient has serum phosphorus mass of about or at least about 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0 mg/dL. As noted herein, in these and related embodiments, administration of a compound or composition described herein may reduce serum phosphorus mass in the subject by about or at least about 5%, 10%, 20%, 30%, 40%, 50%, 60%, 70%, 80%, 90%, 100%, 200% or more.

Certain embodiments relate to methods of treating chronic kidney disease (CKD), a condition characterized by the progressive loss of renal function. Common causes of CKD include diabetes mellitus, hypertension, and glomerulonephritis. Hence, certain methods include treating a subject with CKD, where the subject optionally also has one or more of the foregoing conditions.

In some aspects, a subject is classified as having CKD if they have a glomerular filtration rate (GFR) of less than 60 mL/min/1.73 $m^2$ for about 3 months, whether or not they also present with kidney damage. Certain methods thus include treating a subject with a GFR (e.g., an initial GFR, prior to treatment) of about or less than about 60, 55, 50, 45, 40, 30, 35, 20, 25, 20, 15, or 10 mL/min/1.73 $m^2$ or so. In certain embodiments, administration of a compound or composition described herein may result in an increase in GFR of about or at least about 5%, 10%, 20%, 30%, 40%, 50%, 60%, 70%, 80%, 90%, 100%, 200% or more.

CKD is most often characterized according to the stage of disease: Stage 1, Stage 2, Stage, 3, Stage 4, and Stage 5. Stage 1 CKD includes subjects with kidney damage and a normal or relatively high GFR of about or greater than about 90 mL/min/1.73 $m^2$. Stage 2 CKD includes subjects with kidney damage and a GFR of about 60-89 mL/min/1.73 $m^2$. Stage 3 CKD includes subjects with kidney damage and a GFR of about 30-59 mL/min/1.73 $m^2$. Stage 4 CKD includes subjects with kidney damage and a GFR of about 15-29 mL/min/1.73 $m^2$. Stage 5 CKD includes subjects with established kidney failure and a GFR of less than about 15 mL/min/1.73 $m^2$. Stage 5 CKD is also referred to as end-stage renal disease (ESRD). Accordingly, in certain methods, a subject has Stage 1, 2, 3, 4, or 5, CKD and one or more of its associated clinical characteristics (e.g., defined GFR, kidney damage). In some embodiments, the subject has ESRD and any one or more of its associated clinical characteristics, as described herein and known in the art.

CKD can be characterized according to the affected parts of the kidney. For instance, in certain aspects, CKD includes vascular-associated CKD, including large vessel disease such as bilateral renal artery stenosis, and small vessel disease such as ischemic nephropathy, hemolytic-uremic syndrome and vasculitis. In certain aspects, CKD includes glomerular-associated CKD, including primary glomerular disease such as focal segmental glomerulosclerosis and IgA nephritis, and secondary Glomerular diseases such as diabetic nephropathy and lupus nephritis. Also included is tubulointerstitial-associated CKD, including polycystic kidney disease, drug and toxin-induced chronic tubulointerstitial nephritis, and reflux nephropathy. Certain subjects being treated for CKD may thus have one or more foregoing CKD-associated characteristics.

Certain aspects relate to methods of treating a subject with kidney damage or one or more symptoms/clinical signs of kidney damage. Examples of kidney damage (e.g., CKD-associated kidney damage) and its related symptoms include pathological abnormalities and markers of damage, including abnormalities identified in blood testing (e.g., high blood or serum levels of creatinine, creatinine clearance), urine testing (e.g., proteinuria), and/or imaging studies.

Creatinine is a break-down product of creatine phosphate in muscle, and provides an easily-measured and

useful indicator of renal health. Normal human reference ranges for blood or serum creatinine range from about 0.5 to 1.0 mg/dL (about 45-90 μmol/l) for women and about 0.7 to 1.2 mg/dL (about 60-110 μmol/L) for men. Hence, certain subjects for treatment according to the methods described herein (e.g., initially, prior to treatment) may have blood or serum creatine levels that are about or greater than about 1.0, 1.1, 1.2, 1.3, 1.4, 1.5, 1.6, 1.7, 1.8, 1.9, 2.0 mg/dL. In these and related embodiments, administration of a compound or composition described herein may reduce overall blood or serum creatinine levels in a subject by about or at least about 5%, 10%, 20%, 30%, 40%, 50%, 60%, 70%, 80%, 90%, 100%, or 200% or more.

Creatinine clearance rate ($C_{Cr}$ or CrCl) refers to the volume of blood plasma that is cleared of creatinine per unit time; it is measured by comparing the levels of creatinine in blood relative to urine over a period of time (e.g., 24 hours). Creatine clearance is often measured as milliliters/minute (ml/min) or as a function of body mass (ml/min/kg). Depending on the test performed, normal values range from about 97-137 ml/min for males and about 88-128 ml/min for females. Reduced creatinine clearance provides a useful sign of kidney damage. Hence, certain male subjects for treatment according to the methods described herein (e.g., initially, prior to treatment) may have a $C_{Cr}$ of about or less than about 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50 or less. Certain female subjects for treatment according to the methods described herein (e.g., initially, prior to treatment) may have a $C_{Cr}$ of about or less than about 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 47, 46, 45, 44, 43, 42, 41, 40 or less. In some embodiments, administration of a compound or composition described herein may maintain or increase the $C_{Cr}$ in a subject by about or at least about 5%, 10%, 20%, 30%, 40%, 50%, 60%, 70%, 80%, 90%, 100%, or 200% or more.

Proteinuria refers to a condition of excess protein in the urine. It is associated with variety of disease conditions including kidney damage. Proteinuria is often characterized as a urine protein/creatinine ratio of greater than about 45 mg/mmol, or in specific tests an albumin/creatine ratio of greater than about 30 mg/mmol. Certain subjects for treatment according to the methods provided herein (e.g., prior to treatment) have proteinuria, alone or in combination with CKD or other kidney damage, including subjects with a urine protein/creatinine ratio of about or greater than about 45, 50, 55, 60, 65, 70, 75, 80, 85, 90, 95, 100, 105, 110, 115, or 120 mg/mmol and/or a urine albumin/creatinine ratio of about or greater than about 30, 35, 40, 50, 55, 60, 65, 70, 75, 80, 85, 90, 95, 100, 105, 110, 115, or 120 mg/mmol. In these and related embodiments, administration of a compound or composition described herein may treat proteinuria, for instance, by reducing the urine protein/creatinine ratio and/or the urine albumin/creatinine ratio by about or at least about 5%, 10%, 20%, 30%, 40%, 50%, 60%, 70%, 80%, 90%, 100%, or 200% or more.

CKD is associated with a variety of clinical symptoms. Examples include high blood pressure (hypertension), urea accumulation, hyperkalemia, anemia, hyperphosphatemia, hypocalcemia, metabolic acidosis, and atherosclerosis. Thus, in certain methods, a subject with CKD may also have or be at risk for having one or more of the foregoing clinical symptoms. In specific aspects, the subject with CKD has or is at risk for having hyperphosphatemia, as described herein.

Renal replacement therapy (RRT) relates to the various life-supporting treatments for renal failure, including those initiated in the later stages of CKD and ESRD. Examples of RRT include dialysis, hemodialysis, hemofiltration, and renal transplantation. In certain embodiments, a subject for treatment according to the methods provided herein is about to undergo, is undergoing, or has undergone one or more types of RRT. In some embodiments, the subject is not yet undergoing RRT, and administration of a compound described herein delays the time to initiating RRT (e.g., relative to an untreated state) by about or at least about 1, 2, 3, 4, 5, 6, 7, 8, 9, 10, 11, 12 weeks, or by about or at least about 1, 2, 3, 4, 5, 6, 7, 8, 9, 10, 11, 12 months, or by about or at least about 1, 2, 3, 4, 5, 6, 7, 8, 9, 10, 11, 12 years or more.

Fibroblast growth factor 23 (FGF23) regulates phosphorus and vitamin D metabolism. It also promotes phosphaturia and decreases production of calcitriol. Increased FGF23 levels associate with mortality, left ventricular hypertrophy (or left ventricular mass index), myocardial performance, endothelial dysfunction, and progression of CKD. Indeed, FGF23 levels increase progressively in early CKD, presumably as a physiological adaptation to maintain normal serum phosphate levels or normal phosphorus balance. FGF23 levels might also contribute directly to tissue injury in the heart, vessels, and kidneys. Certain embodiments thus relate to the treatment of subjects having increased FGF23 levels in blood or serum (*see, e.g.,* Kirkpantur et al., Nephrol Dial Transplant. 26:1346-54, 2011), including subjects with CKD and subjects undergoing dialysis/hemodialysis. In some aspects, administration of a compound or composition described herein reduces the logarithm of FGF23 levels in blood or serum by about or at least about 5%, 10%, 20%, 30%, 40%, 50%, 60%, 70%, 80%, 90%, 100%, or 200% or more.

Vitamin D stimulates, *inter alia,* the absorption of phosphate ions in the small intestine. Hence, excess levels or activity of Vitamin D can lead to increased phosphate levels and hyperphosphatemia. Certain embodiments thus relate to methods for reducing the hyperphosphatemic effect of active vitamin D, for instance, in a subject having elevated levels or activity of Vitamin D. In some aspects, the subject has Vitamin D toxicity due to over-ingestion of Vitamin D.

Hyperparathyroidism is a disorder in which the parathyroid glands produce too much parathyroid hormone (PTH). Secondary hyperparathyroidism is characterized by the excessive secretion of PTH in response to hypocalcemia and associated hypertrophy of the parathyroid glands. CKD is the most common cause of secondary hyperparathyroidism,

generally because the kidneys fail to convert sufficient vitamin D into its active form and to excrete sufficient phosphate. Insoluble calcium phosphate forms in the body and thus removes calcium from the circulation, leading to hypocalcemia. The parathyroid glands then further increase the secretion of PTH in an attempt to increase serum calcium levels. Certain subjects for treatment according to the methods provided herein may thus present (e.g., initially, prior to treatment) with hyperparathyroidism and/or increased PTH levels, optionally in combination with CKD, hyperphosphatemia, hypocalcemia, or other condition or symptom described herein. In some aspects, administration of a compound or composition described herein may reduce hyperparathyroidism including secondary hyperparathyroidism in a subject in need thereof. In some aspects, administration of a compound or composition described herein may reduce PTH levels by about or at least about 5%, 10%, 20%, 30%, 40%, 50%, 60%, 70%, 80%, 90%, 100%, or 200% or more, for instance, by reducing serum phosphate levels and the associated formation of insoluble calcium phosphate, increasing available calcium, and thereby reducing the hypocalcemia-induced production of PTH.

In certain embodiments, the administration of a compound described herein, for instance, a dual-active compound that inhibits both transport of Pi and NHE3-mediated antiport of sodium and hydrogen ions, can provide multiple therapeutic effects to a subject with CKD. In some instances, the administration of a dual-active compound reduces the logarithm of FGF23 levels and serum parathyroid hormone (PTH) levels by about or at least about 5%, 10%, 20%, 30%, 40%, 50%, 60%, 70%, 80%, 90%, 100%, or 200% or more relative to an untreated state, reduces blood pressure, and reduces proteinuria by at least about 5%, 10%, 20%, 30%, 40%, 50%, 60%, 70%, 80%, 90%, 100%, or 200% or more relative to an untreated state.

In particular embodiments, the administration of a compound described herein, for instance, a dual-active compound that inhibits both transport of Pi and NHE3-mediated antiport of sodium and hydrogen ions, can provide multiple therapeutic effects to a subject with ESRD (or Stage 5 CKD). In specific instances, the administration of a dual-active compound reduces serum phosphate concentrations or levels by about or at least about 5%, 10%, 20%, 30%, 40%, 50%, 60%, 70%, 80%, 90%, 100%, or 200% or more relative to an untreated state, and reduces inderdialytic weight gain (IDWG) by about or at least about 5%, 10%, 20%, 30%, 40%, 50%, 60%, 70%, 80%, 90%, 100%, or 200% or more relative to an untreated state. IDWG is an easily measurable parameter that is routinely assessed before, during, or after dialysis (see Sarkar et al., Semin Dial. 19:429-33, 2006).

Hyperphosphatemia can lead to endothelial dysfunction in both healthy subjects and those with kidney disease, independently of vascular calcification (see, *e.g.*, Di Marco et al., Kidney International. 83:213-222, 2013). Management of serum phosphate level by dietary phosphate restriction or phosphate binders can prevent such subjects from developing cardiovascular disease. Studies have also shown that dietary phosphate restriction can improve aortic endothelial dysfunction (e.g., in CKD with hyperphosphatemia) by increasing the activatory phosphorylation of endothelial nitric oxide synthase and Akt (*see, e.g.,* Van et al., J Clin Biochem Nutr. 51:27-32, 2012). Certain subjects for treatment according to the methods provided herein may have or be at risk for having endothelial dysfunction, optionally combined with hyperphosphatemia, kidney disease, or any other condition described herein. By reducing postprandial or dietary phosphate uptake, alone or in combination with dietary phosphate restriction, administration of a compound or composition described herein may reduce the risk of developing endothelial dysfunction, or may improve already-existing endothelial dysfunction, including endothelial dysfunction induced by postprandial serum phosphate.

Hyperphosphatemia is a primary inducer of vascular calcification (see Giachelli, Kidney Int. 75:890-897, 2009). Calcium phosphate deposition, mostly in the form of apatite, is the hallmark of vascular calcification and can occur in the blood vessels, myocardium, and cardiac valves. Together with passive deposition of calcium-phosphate in extra-skeletal tissues, inorganic phosphate can also induce arterial calcification directly through "ossification" of the tunica media in the vasculature. Moreover, vascular smooth muscle cells respond to elevated phosphate levels by undergoing an osteochondrogenic phenotype change and mineralizing their extracellular matrix through a mechanism requiring sodium-dependent phosphate cotransporters.

Intimal calcification is usually found in atherosclerotic lesions. Medial calcification is commonly observed in age-associated arteriosclerosis and diabetes, and is the major form of calcification observed in ESRD. Indeed, extensive calcification of the arterial wall and soft tissues is a frequent feature of patients with CKD, including those with ESRD. In valves, calcification is a defining feature of aortic valve stenosis, and occurs in both the leaflets and ring, predominantly at sites of inflammation and mechanical stress. These mechanical changes are associated with increased arterial pulse wave velocity and pulse pressure, and lead to impaired arterial distensibility, increased afterload favoring left ventricular hypertrophy, and compromised coronary perfusion (*see* Guerin et al., Circulation. 103:987-992, 2001). Both intimal and medial calcifications may thus contribute to the morbidity and mortality associated with cardiovascular disease, and are likely to be major contributors to the significant increase in cardiovascular mortality risk observed in CKD and ESRD patients. Control of serum phosphate may thus reduce the formation of calcium/phosphate products and thereby reduce vascular calcification. Accordingly, certain of the subjects for treatment according to the methods provided herein may have or be at risk for developing vascular calcification, including intimal and/or medial calcification, optionally combined with any of hyperphosphatemia, CKD, and ESRD. In some embodiments, administration of a compound or composition described herein reduces the risk of developing or reduces the formation or levels of vascular calcification in a subject in

need thereof. In particular embodiments, administration of a compound or composition described herein may reduce vascular calcification by about or at least about 5%, 10%, 20%, 30%, 40%, 50%, 60%, 70%, 80%, 90%, 100%, or 200% or more, for example, relative to an untreated state.

Elderly patients can be especially susceptible to increased phosphate. For instance, dietary and genetic manipulation studies provide *in vivo* evidence that phosphate toxicity accelerates the aging process and suggest a novel role for phosphate in mammalian aging (*see, e.g.,* Ohnishi and Razzaque, FASEB J. 24:3562-71, 2010). These studies show that excess phosphate associates with many signs of premature aging, including kyphosis, uncoordinated movement, hypogonadism, infertility, skeletal muscle wasting, emphysema, and osteopenia, as well as generalized atrophy of the skin, intestine, thymus, and spleen. Certain embodiments thus relate to reducing phosphate burden in an elderly patient, for instance, to reduce any one or more signs of premature aging, comprising administering to the elderly patient a compound described herein. In some instances, an elderly patient is about or at least about 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more years of age.

Hypertrophy refers to the increase in the volume of an organ or tissue due to the enlargement of its component cells. Hyperphosphatemia associates with myocardial hypertrophy including left ventricular hypertrophy (*see* Neves et al., Kidney Int. 66:2237-44, 2004; and Achinger and Ayus, Am Soc Nephrol. 17(12 Suppl 3):S255-61, 2006) and compensatory renal hypertrophy including glomerular hypertrophy, the latter being often-observed in CKD. Certain subjects for treatment according to the methods provided herein may have (e.g., initially, prior to treatment) myocardial hypertrophy, renal hypertrophy, or both, alone or in combination with CKD or kidney damage. In some embodiments, administration of a compound described herein may reduce myocardial hypertrophy and/or renal hypertrophy by about or at least about 5%, 10%, 20%, 30%, 40%, 50%, 60%, 70%, 80%, 90%, 100%, 200% or more relative to an untreated state.

Administration /Dosage forms:

Administration of the compositions of the invention can be carried out via any of the accepted modes of administration of agents for serving similar utilities. The pharmaceutical compositions of the invention can be prepared by combining a compound of the invention with an appropriate pharmaceutically acceptable carrier, diluent or excipient, and may be formulated into preparations in solid, semi-solid, liquid or gaseous forms, such as tablets, capsules, powders, granules, ointments, solutions, suppositories, injections, inhalants, gels, microspheres, and aerosols. Typical routes of administering such pharmaceutical compositions include, without limitation, oral, topical, transdermal, inhalation, parenteral, sublingual, buccal, rectal, vaginal, and intranasal. The term parenteral as used herein includes subcutaneous injections, intravenous, intramuscular, intrasternal injection or infusion techniques. Pharmaceutical compositions of the invention are formulated so as to allow the active ingredients contained therein to be bioavailable upon administration of the composition to a patient. Compositions that will be administered to a subject or patient take the form of one or more dosage units, where for example, a tablet may be a single dosage unit, and a container of a compound of the invention in aerosol form may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000). The composition to be administered will, in any event, contain a therapeutically effective amount of a compound of the invention, or a pharmaceutically acceptable salt thereof, for treatment of a disease or condition of interest in accordance with the teachings of this invention.

A pharmaceutical composition of the invention may be in the form of a solid or liquid. In one aspect, the carrier(s) are particulate, so that the compositions are, for example, in tablet or powder form. The carrier(s) may be liquid, with the compositions being, for example, an oral syrup, injectable liquid or an aerosol, which is useful in, for example, inhalatory administration.

When intended for oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavoring agent such as peppermint, methyl salicylate or orange flavoring; and a coloring agent.

When the pharmaceutical composition is in the form of a capsule, for example, a gelatin capsule, it may contain, in addition to materials of the above type, a liquid carrier such as polyethylene glycol or oil.

The pharmaceutical composition may be in the form of a liquid, for example, an elixir, syrup, solution, emulsion or suspension. The liquid may be for oral administration or for delivery by injection, as two examples. When intended for oral

administration, preferred composition contain, in addition to the present compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions of the invention, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

A liquid pharmaceutical composition of the invention intended for either parenteral or oral administration should contain an amount of a compound of the invention such that a suitable dosage will be obtained.

The pharmaceutical composition of the invention may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device.

The pharmaceutical composition of the invention may be intended for rectal administration, in the form, for example, of a suppository, which will melt in the rectum and release the drug. The composition for rectal administration may contain an oleaginous base as a suitable nonirritating excipient. Such bases include, without limitation, lanolin, cocoa butter and polyethylene glycol.

The pharmaceutical composition of the invention may include various materials, which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule.

The pharmaceutical composition of the invention in solid or liquid form may include an agent that binds to the compound of the invention and thereby assists in the delivery of the compound. Suitable agents that may act in this capacity include a monoclonal or polyclonal antibody, a protein or a liposome.

The pharmaceutical composition of the invention may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of compounds of the invention may be delivered in single phase, bi-phasic, or tri-phasic systems in order to deliver the active ingredient(s). Delivery of the aerosol includes the necessary container, activators, valves, subcontainers, and the like, which together may form a kit. One skilled in the art, without undue experimentation may determine preferred aerosols.

The pharmaceutical compositions of the invention may be prepared by methodology well known in the pharmaceutical art. For example, a pharmaceutical composition intended to be administered by injection can be prepared by combining a compound of the invention with sterile, distilled water so as to form a solution. A surfactant may be added to facilitate the formation of a homogeneous solution or suspension. Surfactants are compounds that non-covalently interact with the compound of the invention so as to facilitate dissolution or homogeneous suspension of the compound in the aqueous delivery system.

The compounds of the invention, or their pharmaceutically acceptable salts, are administered in a therapeutically effective amount, which will vary depending upon a variety of factors including the activity of the specific compound employed; the metabolic stability and length of action of the compound; the age, body weight, general health, sex, and diet of the patient; the mode and time of administration; the rate of excretion; the drug combination; the severity of the particular disorder or condition; and the subject undergoing therapy.

In certain embodiments, a typical dosage of the substantially impermeable or substantially systemically non-bioavailable, compound may be between about 0.2 mg per day and about 2 g per day, or between about 1 mg and about 1 g per day, or between about 5 mg and about 500 mg, or between about 10 mg and about 250 mg per day, which is administered to a subject in need of treatment.

The frequency of administration of the compounds and compositions described herein may vary from once-a-day (QD) to twice-a-day (BID) or thrice-a-day (TID), etc., the precise frequency of administration varying with, for example, the patient's condition, the dosage, etc.

Compounds of the invention, or pharmaceutically acceptable derivatives thereof, may also be administered

simultaneously with, prior to, or after administration of one or more other therapeutic or biologically active agents, dietary supplements, or any combination thereof. Such combination therapy includes administration of a single pharmaceutical dosage formulation which contains a compound of the invention and one or more additional active agents, as well as administration of the compound of the invention and each active agent in its own separate pharmaceutical dosage formulation. For example, a compound of the invention and the other active agent can be administered to the patient together in a single oral dosage composition such as a tablet or capsule, or each agent administered in separate oral dosage formulations. Where separate dosage formulations are used, the compounds of the invention and one or more additional active agents can be administered at essentially the same time, i.e., concurrently, or at separately staggered times, i.e., sequentially; combination therapy is understood to include all these regimens.

For example, in certain embodiments, the additional biologically active agent included in a pharmaceutical composition (or method) of the invention is selected, for example, from vitamin $D_2$ (ergocalciferol), vitamin $D_3$ (cholecalciferol), active vitamin D (calcitriol) and active vitamin D analogs (e.g. doxercalciferol, paricalcitol).

In some embodiments, the additional biologically active agent is an inhibitor of the intestinal sodium-dependent phosphate transporter (NaPi2b inhibitor). Examples of NaPi2b inhibitors can be found, for instance, in International Application Nos. PCT/US2011/043267; PCT/US2011/043261; PCT/US2011/043232; PCT/US2011/043266; and PCT/US2011/043263; and U.S. Patent No. 8,134,015, each of which is incorporated by reference in its entirety.

In certain embodiments, the additionally biologically active agent is niacin or nicotinamide.

It is understood that in the present description, combinations of substituents and/or variables of the depicted formulae are permissible only if such contributions result in stable or reasonably stable compounds.

It will also be appreciated by those skilled in the art that in the process described herein the functional groups of intermediate compounds may need to be protected by suitable protecting groups. Such functional groups include hydroxy, amino, mercapto, and carboxylic acid. Suitable protecting groups for hydroxy include trialkylsilyl or diarylalkylsilyl (for example, *t*-butyldimethylsilyl, *t*-butyldiphenylsilyl or trimethylsilyl), tetrahydropyranyl, benzyl, and the like. Suitable protecting groups for amino, amidino and guanidino include *t*-butoxycarbonyl, benzyloxycarbonyl, and the like. Suitable protecting groups for mercapto include -C(O)-R" (where R" is alkyl, aryl or arylalkyl), *p*-methoxybenzyl, trityl and the like. Suitable protecting groups for carboxylic acid include alkyl, aryl or arylalkyl esters. Protecting groups may be added or removed in accordance with standard techniques, which are known to one skilled in the art and as described herein. The use of protecting groups is described in detail in Green, T.W. and P.G.M. Wutz, Protective Groups in Organic Synthesis (1999), 3rd Ed., Wiley. As one of skill in the art would appreciate, the protecting group may also be a polymer resin such as a Wang resin, Rink resin or a 2-chlorotrityl-chloride resin.

It will also be appreciated by those skilled in the art, although such protected derivatives of compounds of this invention may not possess pharmacological activity as such, they may be administered to a mammal and thereafter metabolized in the body to form compounds of the invention which are pharmacologically active. Such derivatives may therefore be described as "prodrugs". All prodrugs of compounds of this invention are included within the scope of the invention.

Furthermore, all compounds of the invention which exist in free base or acid form can be converted to their pharmaceutically acceptable salts by treatment with the appropriate inorganic or organic base or acid by methods known to one skilled in the art. Salts of the compounds of the invention can be converted to their free base or acid form by standard techniques.

## EXAMPLES

### Example 1

**Cell-based activity under Prompt Conditions.** Rat or human NHE3-mediated $Na^+$-dependent $H^+$ antiport was measured using a modification of the pH sensitive dye method originally reported by Paradiso (PNAS USA. 81:7436-7440, 1984). Opossum kidney (OK) cells were obtained from the ATCC and propagated per their instructions. The rat NHE3 gene (GenBank M85300) or the human NHE3 gene (GenBank NM_004174.1) was introduced into OK cells via electroporation, and cells were seeded into 96 well plates and grown overnight. Medium was aspirated from the wells, cells were washed twice with NaCl-HEPES buffer (100 mM NaCl, 50 mM HEPES, 10 mM glucose, 5mM KCl, 2 mM $CaCl_2$, 1 mM $MgCl_2$, pH 7.4), then incubated for 30 min at room temperature with $NH_4Cl$-HEPES buffer (20 mM $NH_4Cl$, 80 mM NaCl, 50 mM HEPES, 5 mM KCl, 2mM $CaCl_2$, 1 mM $MgCl_2$, pH 7.4) containing 5 $\mu$M bis(acetoxymethyl) 3,3'-(3',6'-bis(acetoxymethoxy)-5-((acetoxymethoxy)carbonyl)-3-oxo-3H-spiro[isobenzofuran-1,9'-xanthene]-2',7'-diyl)dipropanoate (BCECF-AM).

Cells were washed twice with Ammonium free, $Na^+$-free HEPES (100 mM choline, 50 mM HEPES, 10 mM glucose, 5 mM KCl, 2 mM $CaCl_2$, 1 mM $MgCl_2$, pH 7.4) and incubated in the same buffer for 10 minutes at room temperature to lower intracellular pH. NHE3-mediated recovery of neutral intracellular pH was initiated by addition of Na-HEPES buffer containing 0.4 $\mu$M ethyl isopropyl amiloride (EIPA, a selective antagonist of NHE-1 activity that does not inhibit NHE3) and 0-30 $\mu$M test compound, and monitoring the pH sensitive changes in BCECF fluorescence ($\lambda_{ex}$ 505nm,

$\lambda_{em}$ 538nm) normalized to the pH insensitive BCECF fluorescence ($\lambda_{ex}$ 439nm, $\lambda_{em}$ 538nm). Initial rates were plotted as the average 2 or more replicates, and $pIC_{50}$ values were estimated using GraphPad Prism.

**Inhibition of intestinal sodium and phosphate absorption.** Urinary sodium concentration and fecal form were measured to assess the ability of selected example compounds to inhibit the absorption of sodium from the intestinal lumen. Eight-week old Sprague-Dawley rats were purchased from Charles River Laboratories (Hollister, CA), were housed 2 per cage, and acclimated for at least 3 days before study initiation. Animals were fed Harlan Teklad Global 2018 rodent chow (Indianapolis, IN) and water *ad libitum* throughout the study and maintained in a standard light/dark cycle of 6AM to 6PM. On the day of the study, between 4PM and 5PM, a group of rats (n=6) were dosed via oral gavage with test compound or vehicle (water) at a volume of 10 mL/kg.

After dose administration animals were placed in individual metabolic cages where they were also fed the same chow in meal form and watered *ad libitum.* At 16h post-dose, the urine samples were collected and fecal form was assessed by two independent observations. Fecal forms were scored according to a common scale associated with increasing fecal water to the wettest observation in the cage's collection funnel (1, normal pellet; 2, pellet adhering to sides of collection funnel due to moisture; 3, loss of normal pellet shape; 4, complete loss of shape with a blotting pattern; 5, liquid fecal streams evident). A rat's fecal form score (FFS) was determined by averaging both observational scores for all rats within a group (n=6). The vehicle group average was 1.

For urine samples, the volumes were determined gravimetrically and centrifuged at 3,600 $\times$ *g*. The supernatants were diluted 100-fold in deionized Milli-Q water then filtered through a 0.2 $\mu$m GHP Pall AcroPrep filter plate (Pall Life Sciences, Ann Arbor, MI) prior to analysis by ion chromatography. Ten microliters of each filtered extract was injected onto a Dionex ICS-3000 ion chromatograph system (Dionex, Sunnyvale, CA). Cations were separated by an isocratic method using 25 mM methanesulfonic acid as the eluent on an IonPac CS12A 2 mm i.d. $\times$ 250 mm, 8 $\mu$m particle size cation exchange column (Dionex). Sodium was quantified using standards prepared from a cation standard mix containing $Li^+$, $Na^+$, $NH_4^+$, $K^+$, $Mg^{2+}$, and $Ca^{2+}$ (Dionex). The mean mass of sodium urinated for every group in the 16 h period was determined with the vehicle group usually urinating approximately 21 mg sodium. The urine Na (uNa) for rats in the test groups were expressed as a percentage of the vehicle mean and the means were compared to that of the vehicle group by utilizing a one-way analysis of variance coupled with a Dunnett's *post hoc* test.

## EXAMPLE 2

## EFFECTS IN A RAT MODEL OF UREMIA-ASSOCIATED VASCULAR CALCIFICATION

Chronic kidney disease (CKD) has multiple pathogenic mechanisms, and advanced CKD is often characterized by disordered mineral metabolism (e.g., hyperphosphatemia, hypercalcemia) and vascular calcification. Studies were thus performed to test the effectiveness of Cpd 002 in a uremic rat model of CKD featuring vascular calcification. This model is characterized by renal insufficiency and regular active Vitamin $D_3$ administration to promote hyperphosphatemia and vascular calcification (*see* Lopez et al., J. Am. Soc. Nephrol. 17:795-804, 2006). The study utilized Spraque-Dawley rats treated as follows: $5/6^{th}$ nephrectomy by excision; regular calcitriol administration (active vitamin $D_3$) 80ng/kg i.p. 3/week; and fed a purified 0.9% P diet (inorganic phosphorus).

Rats were stratified into two experimental groups by serum creatinine levels of 0.8 to 1.5 mg/dl and body weight, fed drug-in-chow with powdered vehicle diet or the same diet with Cpd 002 (0.065mg/g chow) mixed-in, and monitored for weekly body weight and selected serum parameters, daily clinical observations, and endpoint calcification.

Selected experimental groups were fed vehicle (*n*=12) or Cpd 002 (*n*=12) at enrollment (day 0). Initial body weights and selected serum parameters such as serum phosphorus, serum calcium, serum creatinine, and blood urea nitrogen were comparable for both groups.

Selected endpoint plasma parameters from day 27 showed reduced plasma creatinine, reduced plasma phosphorus, and reduced plasma FGF-23.

Endpoint heart and kidney remnant weights showed that hypertrophy of the heart and kidney remnants was lessened in Cpd 002 treated rats. Given reduced plasma creatinine, these results suggest that the kidney remnant in Cpd 002 treated rats has more functionality with less mass.

Endpoint creatinine clearance ($C_{Cr}$) and plasma aldosterone levels suggested that treatment with Cpd 002 protected against loss of kidney function, and aldosterone increase suggests some volume depletion, which is consistent with lower Na intake.

Endpoint vascular and soft tissue calcification showed that treatment with Cpd 002 reduced calcium and phosphorus in the stomach, which is particularly sensitive to calcification, and also reduced vascular calcification as measured by aortic mineral content.

Overall, Cpd 002 was shown to improve kidney function, reduce both heart hypertrophy and renal hypertrophy, exhibit anti-hyperphosphatemic effects, and reduce associated vascular calcification. These effects and decreased moribundity were observed in the treatment group with a trend toward improved mortality outcome. While the benefits from

Cpd 002 can partly result from its effect on fluid overload and hemodynamics, because vascular calcification in this model is highly sensitive to dietary phosphate, the reduction in ectopic calcification points to a reduction in phosphate absorption.

**EXAMPLE 3**

**EFFECTS IN AN ADENINE-INDUCED UREMIC RAT MODEL**

The effects of Cpd 002( were tested in an adenine-induced uremic rat model. Rats were fed a diet including 0.75% adenine and 1.2% phosphorus during the nephritis induction phase. The basal diet during the treatment phase was normal chow including 0.3% adenine and 0.6% phosphorus for 2 weeks. The rats were pair-fed the first 5 days (groups 1 and 2 to group 3, 4 days apart), and fed *ad libitum* afterwards. The treatment groups were as follows: vehicle, n = 10; Cpd 002, 2 mg/kg/day drug-in-chow, n=10; and Cpd 002, 5 mg/kg/day drug-in-chow, n=12. Weekly measurements were taken for serum markers and kidney function..

Cpd 002 reduced serum phosphorus and serum creatinine at early time points. Here, this adenine-induced model is considered an acute renal injury characterized by a progressive recovery of renal function. Hence, the effects at early time points are significant.

Organ weight collection data from week three showed that treatment with Cpd 002 in this model showed a trend towards lesser heart and kidney remodeling, and a trend towards reduced heart and kidney calcification at the highest dose.

**EXAMPLE 4**

**EFFECT ON RENAL INSUFFICIENCY WITH HIGH SALT FEED IN NEPHRECTOMIZED RATS**

The effects of Cpd 002 were tested in a dietary salt-induced, partial renal ablation model of CKD. The study design is described in WO2014169094 which shows the effects of Cpd 002 on urinary excretion of phosphorus. In this study, Cpd 002 improved blood pressure, fluid overload, albuminuria, and heart and kidney hypertrophy, and also significantly reduced phosphorus urinary excretion. These data suggest an additive contribution for the phosphorus lowering effect of Cpd 002 on improvements in the renal and vascular functions.

**EXAMPLE 5**

**EFFECTS ON URINARY EXCRETION OF PHOSPHATE AND CALCIUM IN RATS**

The activity of compound 002 was tested for its effects on phosphorus and calcium levels in the urine of rats. Rats were dosed according to the schedule in **Table E7.**

**Table E7**

| 929uP groups | Dose #1 | Dose #2 10 min later |
|---|---|---|
| 1 | Water | Water |
| 2 | Renvela® (sevelamer), 48 mg/kg | Water |
| 3 | Water | Cpd 002, 0.1 mg/kg |
| 4 | Water | Cpd 002, 0.3 mg/kg |
| 5 | Water | Cpd 002, 1.0 mg/kg |
| 6 | Water | Cpd 002, 3.0 mg/kg |

The rats were kept for 16 hours overnight (in the dark, the typical feeding period) in individual metabolic cages, and urine was collected the following morning for analysis of phosphate and calcium levels. The study design is shown WO2014169094. The results are shown in Figures 3A and 3B. These results show that Cpd 002 reduced urine phosphorus mass relative to the vehicle-only control. Increasing dosages of Cpd 002 also significantly reduced urine phosphorus mass relative to 48 mg/kg Renvela®.

**EXAMPLE 6**

**EVALUATION OF ACTIVITY IN THE REDUCTION OF DIETARY PHOSPHORUS AT DOSE 15, 30 AND 60 MG BID IN A 7-DAY REPEAT**

**DOSE STUDY IN HEALTHY VOLUNTEERS**

A Phase 1, single-center, randomized, double-blind, placebo-controlled study was designed to evaluate the safety, tolerability, and pharmacodynamic activity (PD) on sodium and phosphorus excretion of different dosing regimens of compound 002 (CPD002) in healthy male and female subjects.

Subjects were screened within 3 weeks prior to enrollment and were allocated sequentially to cohorts in their order of completing screening assessments. Each cohort of 15 subjects checked into the clinical pharmacology unit (CPU) on Day -5 before dinner. Subjects were confined to the CPU, Na+-standardized meals (~1500 mg/meal) provided.

In each cohort, 12 subjects were randomized to receive CPD002 and 3 subjects to placebo. Subjects received doses of CPD002 with approximately 240 mL of non-carbonated water on Days 1 to 7 (just prior to the appropriate meals, depending on twice daily [bid, breakfast, dinner]. Subjects were provided standardized meals within 10 minutes after dosing.

**Selection of Study Population - Inclusion Criteria.** Subjects were eligible for inclusion in the study if they met all of the following criteria:

1. Healthy man or woman aged 19 to 65 years, inclusive.
2. Body mass index (BMI) between 18 and 29.9 $kg/m^2$, inclusive.
3. No clinically significant abnormalities in medical history, physical examination, or clinical laboratory evaluations at screening.
4. Able to understand and comply with the protocol.
5. Willing and able to sign informed consent.
6. Females were non-pregnant, non-lactating, and either postmenopausal for at least 12 months, as confirmed by follicle-stimulating hormone (FSH) test, surgically sterile (e.g., tubal ligation, hysterectomy, bilateral oophorectomy with appropriate documentation) for at least 90 days, or agreed to use from the time of signing the informed consent until 45 days after end of study 1 of the following forms of contraception: intrauterine device with spermicide, female condom with spermicide, contraceptive sponge with spermicide, diaphragm with spermicide, cervical cap with spermicide, male sexual partner who agrees to use a male condom with spermicide, sterile sexual partner, abstinence, an intravaginal system (e.g., NuvaRing®) with spermicide, or oral, implantable, transdermal, or injectable contraceptives with spermicide.
7. Males were either sterile, abstinent, or agreed to use, from check-in until 45 days from final study visit, 1 of the following approved methods of contraception: a male condom with spermicide; a sterile sexual partner; use by female sexual partner of an intrauterine device with spermicide, a female condom with spermicide, contraceptive sponge with spermicide, an intravaginal system (e.g., NuvaRing), a diaphragm with spermicide, a cervical cap with spermicide, or oral, implantable, transdermal, or injectable contraceptives).

**Selection of Study Population - Exclusion Criteria.** Subjects were excluded from the study if they met any of the following criteria:

1. Diagnosis or treatment of any clinically symptomatic biochemical or structural abnormality of the gastrointestinal system.
2. Any surgery on the small intestine or colon, excluding appendectomy or cholecystectomy.
3. Clinical evidence of significant cardiovascular, respiratory, renal, hepatic, gastrointestinal, hematologic, metabolic, endocrine, neurologic, psychiatric disease, or any condition that may interfere with the subject successfully completing the trial.
4. Loose stools (BSFS of 6 or 7) ≥2 days in the past 7 days.
5. Hepatic dysfunction (alanine aminotransaminase [ALT] or aspartate aminotransaminase [AST]) >1.5 times the upper limit of normal [ULN]) or renal impairment (serum creatinine >ULN).
6. Clinically significant laboratory results at screening as determined by the Investigator.
7. Any evidence of or treatment of malignancy, excluding non-melanomatous malignancies of the skin.
8. If, in the opinion of the Investigator, the subject was unable or unwilling to fulfill the requirements of the protocol or had a condition that rendered the results uninterpretable.
9. A diet, which in the opinion of the Investigator, could have impacted the results of the study.
10. Use of diuretic medications; medications that were known to affect stool consistency and/or gastrointestinal motility, including fiber supplements (unless required by study), anti-diarrheals, cathartics, antacids, opiates, narcotics, prokinetic drugs, enemas, antibiotics, probiotic medications or supplements; or salt or electrolyte supplements containing Na+, potassium, chloride, or bicarbonate formulations from CPU check in (Day -5) to CPU check out (Day 9).

11. Use of an investigational agent within 30 days prior to Day -5.

12. Positive virology (active hepatitis B infection [HBsAg], hepatitis C infection [HCV], or human immunodeficiency virus [HIV]), alcohol, or drugs of abuse test during screening,

13. Use of any prescription medication within 7 days before admission to the CPU, or required chronic use of any prescription or non-prescription medication, with the exception of hormonal replacement therapy (HRT) for post-menopausal women and hormonal contraceptives.

14. History of tobacco use, alcohol abuse, illicit drug use, significant mental illness, physical dependence to any opioid, or any history of drug abuse or addiction within 12 months of study enrollment.

15. Had significant blood loss (>450 mL) or had donated 1 or more units of blood or plasma within 8 weeks prior to study entry.

**Removal of Subjects from Therapy or Assessment.** Subjects were free to discontinue the study at any time, for any reason, and without prejudice to further treatment. The Investigator could have removed a subject if, in the Investigator's judgment, continued participation posed unacceptable risk to the subject or to the integrity of the study data. Subjects who withdrew early could have been replaced, pending discussion with the Sponsor.

**Efficacy evaluation - demographic and other baseline characteristics.** All subjects enrolled in the study received study treatment and all had at least 1 post-baseline PD assessment.

An overview of the demographic characteristics of the subjects enrolled in the study overall and by cohort is provided in **Table E8** below. Some variability was observed across cohorts (especially in terms of gender and race); however, the baseline characteristics of most cohorts mirrored that of the total population.

No clinically significant abnormal findings were noted for any subject during the physical examination performed at screening.

**Table E8**
**Demographic and Baseline Characteristics**

| Parameter | Cohort 1 30 mg bid (n=12) | Cohort 3 60 mg bid (n=12) | Cohort 4 15 mg bid (n=12) |
|---|---|---|---|
| Mean (SD) | 38.8 (16.49) | 37.8 (11.78) | 38.7 (12.91) |
| Median | 31.0 | 33.5 | 36.5 |
| Min, Max | 20, 63 | 22, 61 | 20, 60 |
| Female | 3 (25.0) | 3 (25.0) | 2 (16.7) |
| Male | 9 (75.0) | 9 (75.0) | 10 (83.3) |
| Mean (SD) | 73.7 (11.39) | 79.3 (9.98) | 78.7 (12.99) |
| Median | 71.7 | 75.7 | 79.7 |
| Min, Max | 58, 91 | 67, 103 | 60, 101 |
| Mean (SD) | 24.6 (2.69) | 26.1 (2.46) | 25.7 (2.87) |
| Median | 24.3 | 26.2 | 25.9 |
| Min, Max | 19, 29 | 22, 29 | 20, 30 |
| Asian | 1 (8.3) | 1 (8.3) | 0 |
| Black | 2 (16.7) | 6 (50.0) | 4 (33.3) |
| White | 7 (58.3) | 5 (41.7) | 6 (50.0) |
| Other | 2 (16.7) | 0 | 1 (8.3) |
| Missing | 0 | 0 | 1 (8.3) |

The schedule of events for screening and treatment period is provided in **Table E9** below.

| Table E9 | | | | | | | | | | | | | | | |
|---|---|---|---|---|---|---|---|---|---|---|---|---|---|---|---|
| | Screening and Baseline Day | | | | | | Double-blind Treatment Period Day | | | | | | | | Follow-up |
| Procedure | -26 to -5 | -5[a] | -4 | -3 | -2 | -1 | 1 | 2 | 3 | 4 | 5 | 6 | 7 | 8 | 9[a] | 23 ± 2 |
| Informed consent | X | | | | | | | | | | | | | | | |
| Inclusion/ exclusion | X | X[b] | | | | | | | | | | | | | | |
| Medical history | X | X[b] | | | | | | | | | | | | | | |
| Physical examination | X | | | | | | | | | | | | | | X | |
| Vital signs | X | X | X | X | X | X | X | X | X | X | X | X | X | X | X | |
| ECG evaluation | X | | | | | | | | | | | | | | X | |
| Safety laboratory evaluations | X | | X | | | | | | | | | | | | X | |
| Alcohol/ drug screen | X | X | | | | | | | | | | | | | | |
| FSH test | X | | | | | | | | | | | | | | | |
| Pregnancy test | X | X | | | | | | | | | | | | | X | |
| Randomization | | | | | | | X | | | | | | | | | |
| Dose administration | | | | | | | X | X | X | X | X | X | X | | | |
| 24-hr urine/stool collection | | | X | X | X | X | X | X | X | X | X | X | X | X | X | |
| Stool form/timing | | | X | X | X | X | X | X | X | X | X | X | X | X | X | |
| Pharmacodynamic laboratory evaluations | | | X | | | X | | | X | | | X | | | X | |
| AE assessment | | | | | | | X | X | X | X | X | X | X | X | X | X |

**Study drug.** CPD002 capsules or corresponding placebo capsules were administered with approximately 240 mL of non-carbonated water at multiples of 15 mg or placebo. CPD002 is an amorphous, off-white powder and was supplied as a white, size 0, hydroxypropylmethylcellulose (HPMC) capsule. Each capsule contained 15 mg of CPD002. Capsules were packaged in an opaque white high density polyethylene (HDPE) bottle (10/bottle). The drug product was formulated with no excipients.

Placebo was supplied as a white, size 0, HPMC capsule filled with methyl cellulose. Capsules were packaged in an opaque white HDPE bottle (10/bottle).

**Method of Assigning Subjects to Treatment Groups.** The clinical research organization statistician prepared the randomization scheme in accordance with its standard operating procedures (SOPs) and the randomization plan, which reflected GCP standards.

After obtaining informed consent, subjects were allocated sequentially to cohorts in their order of completing screening assessments.

Within each cohort, a computer generated randomization schedule was used to randomly assign subjects to active CPD002 or placebo in a 4:1 ratio.

Once a subject was deemed eligible for randomization, the next available randomization number was assigned sequentially and the subject received the treatment indicated on the randomization schedule. Subjects who withdrew early could be replaced, pending discussion with the Sponsor. Replacement subjects received the same blinded treatment as the original subject.

**Selection and Timing of Dose for Each Subject.** Subjects were allocated sequentially to cohorts consisting of 15 subjects each in their order of completing screening assessments and received either CPD002 or placebo based on

random assignment. **Table E10** provides the actual dosing regimen for each cohort. Because this was an adaptive design protocol, the dosing regimen of each cohort was based on blinded results from previous cohorts.

| Table E10 Dosing Regimen for Each Cohort | | | | |
|---|---|---|---|---|
| **Cohort No.** | **Subjects[a]** | **Dose/Administration** | **Regimen** | **Total Dose/Day** |
| 1 | 15 | 30 mg | bid | 60 mg |
| 3 | 15 | 60 mg | bid | 120 mg |
| 4 | 15 | 15 mg | bid | 30 mg |
| [a] Each cohort consisted of 12 subjects administered CPD002 and 3 subjects administered placebo. | | | | |

Dosing was administered immediately prior to breakfast and dinner. Subjects were not permitted to eat or drink anything from 8 hours before dosing at breakfast, with the exception of water up to 2 hours prior. Subjects were fed a standardized meal approximately 10 minutes after dosing.

The standardized diet included a Na+ content of approximately 1500 mg for each meal. Dietary phosphorus was not measured nor was it set to a predetermined value. It was expected to range within the typical value, i.e. 750 mg - 1250 mg per day.

Subjects did not have salt available to add to meals. Fluid intake was ad libitum (and recorded) except as specified before drug administration. Subjects were to refrain from strenuous physical activity (e.g., contact sports) during study participation.

**Blinding.** The treatment was administered in a double-blind fashion. Only the site pharmacist responsible for dispensing the product and the bioanalytical laboratory technician responsible for performing the bioanalysis of plasma CPD002 had knowledge of the treatments assigned.

The study was not unblinded for the safety reviews between cohorts.

A third party maintained the randomization schedule in a secure location with adequate controls to prevent unauthorized access.

One set of unblinding envelopes (sealed envelopes containing individual subject treatment assignment) was stored at the CPU.

The study was only unblinded once all data from the final cohort was collected and the database was locked.

**Prior and Concomitant Therapy.** This was a study in healthy subjects. Subjects with prior therapy specified in the exclusion criteria were not eligible for entry into the study.

With the exception of HRT for postmenopausal women and hormonal contraceptives, the use of concomitant medications was prohibited during the study unless needed to treat an AE.

All previous medication (prescription and over-the-counter), vitamin and mineral supplements, and herbs taken by the participant in the past 30 days were recorded in the CRF, including start and stop date, dose and route of administration, frequency and indication. Medications taken for a procedure were also included.

**Treatment Compliance.** All doses of study drug were given under the supervision of clinic staff, with time and dose administered recorded in the CRF. Clinical staff examined the subject's oral cavity and hands after drug administration to ensure that the capsule(s) was/were swallowed.

**Efficacy Variables.** The study consisted of a 3-week screening period followed by a 5-day baseline assessment, a 7-day double-blind treatment period with 2 days of follow-up for safety and PD assessments. Fourteen days after the treatment period subjects were contacted by telephone for a safety follow-up.

Subjects were admitted to the CPU 5 days prior to administration of the first dosing of study drug and were confined to the unit for the duration of the treatment period, being released on Day 9.

Safety assessments were performed starting with Day -5 and included physical examination; vital signs; 12-lead ECGs; routine serum chemistry, hematology, and urinalysis; and AE reporting. Pharmacodynamic assessments were performed daily from Day -4 through Day 9 and included urine and stool Na+ excretion, time to first bowel movement, and stool parameters (consistency, weight, and frequency). Pharmacodynamic laboratory assessments (plasma renin, aldosterone, and NT-pro BNP) were collected on Days -4, -1, 3, 6, and 9.

**Laboratory Assessments.** Blood and urine samples for clinical laboratory tests (hematology, chemistry, urinalysis) were collected during screening (to meet inclusion/exclusion criteria) and at Day -4, and Day 9 after waking and prior to breakfast.

In addition, blood was collected at screening and Day -5 for alcohol/drug screening, FSH test (postmenopausal females only), and pregnancy testing (all females). Virology screening for HBsAg, HCV, and HIV were performed at screening.

**Pharmacodynamic Variables.** The following PD parameters were monitored as a signal of potential drug

activity:

- Stool Na+ excretion
- Stool Phosphorus excretion

**Bowel movements.** Study participants were instructed to notify study personnel immediately before they had a bowel movement. Study personnel recorded the time of every bowel movement and assessed the stool parameters (e.g., consistency, weight). Bowel movements that occurred prior to leaving the bathroom were considered 1 bowel movement. All bowel movements were collected, weighed, and stored by the CPU for total Na+ and P analysis; collections were in 24-hour intervals.

**Pharmacodynamic Analyses - Stool Sodium and Phosphorus Analytical methods.** The human fecal samples were processed with nitric acid to give pre-digested sample ("Pre-digests") prior to laboratory determination of sodium and phosphorus contents. Pre-digest were digested further in nitric acid at 100°C followed by hydrochloric acid at 100°C and diluted with deionized water. Yttrium was added to the digestion as internal standard. Calibration standards and quality control samples were digested with the same procedure. Sodium and phosphorus concentrations were determined by an inductively coupled plasma optical emission spectrometric (ICP-OES) method. The light intensity of analyte and yttrium were measured at the SCD (array) detectors. The analyte-to-yttrium intensity ratios were converted to solution concentrations via the instrument software. Total sodium and phosphorus content in each sample was calculated using the sample volumes obtained during the pre-digestion process and the concentrations measured.

**Results.** Upon unblinding of the data, pharmacodynamic measurement of fecal and urine P and Na were assigned to the placebo group (3 subjects embedded in each cohort x 3 cohorts = 9 subjects) and to the 3 treated groups respectively. The data are illustrated in WO2014169094 which shows the mean average daily fecal excretion of Na (+/-SE), averaged over the 7-day treatment period ( Day 1 to Day 7) and reported as mEq/day. The mean average daily fecal excretion of phosphorus (+/-), averaged over the 7-day treatment period ( Day 1 to Day 7) and reported as mEq/day. Statistical analysis was performed by one-way ANOVA; (*); $p<0.05$, (**); $p<0.01$, (***); $p<0.001$.

## EXAMPLE 7

## EVALUATION OF ACTIVITY IN THE REDUCTION OF DIETARY PHOSPHORUS AT DOSE 15 MG BID IN A 7-DAY REPEAT DOSE

## CROSSOVER STUDY IN HEALTHY VOLUNTEERS

A Phase 1, single-center, randomized, 3-way cross-over, open label study was designed to evaluate the pharmacodynamics of CPD002 for three different formulations of CPD002 administered twice daily PO for 4 days in healthy male and female subjects taking a proton pump inhibitor (omeprazole), utilizing a three-way crossover design. Many potential patients take either PPIs or H2 antagonists for the treatment of gastroesophageal reflux disease (GERD). However, the in vitro dissolution profiles of CPD002 formulations can be affected by a high pH, where slower and/or incomplete dissolution is sometimes observed. In order to evaluate the pharmacodynamic activity of the drug in the context of elevated gastric pH, subjects in this study were required to be on omeprazole starting on Day -5 throughout the treatment period.

Subjects were screened within 3 weeks of enrollment. Each subject took Omeprazole 20 mg twice daily beginning on Day -5. Subjects checked in a Clinical Pharmacology Unit (CPU) on Day -2 before dinner. Each subject received a diet standardized for Na+ content while in the CPU. Subjects received one of three formulations of CPD002 BID with approximately 240 mL of non-carbonated water on Days 1 to 4, 7 to 10, and 13 to 16 (a different formulation each time). Subjects were fed breakfast and/or dinner within approximately 5 minutes after dosing. There was a two day wash out period between each treatment period.

While confined to the CPU, Na+-standardized meals were provided per CPU procedures. Pharmacodynamic assessment included 24-hour urinary sodium and phosphorus and fecal sodium and phosphorus measurements.

At least 18 healthy male and female subjects were randomized in this study.

### Subject Selection Criteria - Inclusion criteria.

1. Healthy man or woman aged 19 to 65 years, inclusive.
2. Body mass index between 18 and 29.9 kg/m2, inclusive.

3. No clinically significant abnormalities in the medical history, physical examinations, or clinical laboratory evaluations at screening.
4. Able to understand and comply with the protocol.
5. Willing and able to sign informed consent; signed and dated, written informed consent prior to any study specific procedures.
6. Females of child-bearing potential must have a negative pregnancy test at screening and on admission to the unit and must not be lactating.
7. Females of childbearing potential included in the study must use two effective methods of avoiding pregnancy (including oral, transdermal or implanted contraceptives, intrauterine device, female condom with spermicide, diaphragm with spermicide, cervical cap, or use of a condom with spermicide by sexual partner from screening to the follow-up visit.
8. Females of non-child bearing potential, confirmed at screening, must fulfill one of the following criteria:

   a. Post-menopausal defined as amenorrhea for at least 12 months or more; following cessation of all exogenous hormonal treatments and LH and FSH levels in the post- menopausal range; or
   b. Documentation of irreversible surgical sterilization by hysterectomy, bilateral oophorectomy or bilateral salpingectomy but not tubal ligation.

9. Males must be either be sterile, abstinent or agree to use, from check-in until 45 days from final study visit, one of the following approved methods of contraception: a male condom with spermicide; a sterile sexual partner; use by female sexual partner of an IUD with spermicide, a female condom with spermicide, contraceptive sponge with spermicide, an intravaginal system (eg, NuvaRing®), a diaphragm with spermicide, a cervical cap with spermicide, or oral, implantable, transdermal, or injectable contraceptives.
10. For inclusion in the optional genetic research, patients must fulfill all of the inclusion criteria described above and provide informed consent for the genetic sampling and analyses.

**Exclusion Criteria.** Subjects were excluded from the study if they met any of the following criteria:

1. Diagnosis or treatment of any clinically symptomatic biochemical or structural abnormality of the gastrointestinal (GI) tract.
2. Any surgery on the small intestine or colon, excluding appendectomy or cholecystectomy or any other condition known to interfere with absorption, distribution, metabolism or excretion of drugs.
3. Clinical evidence of significant cardiovascular, respiratory, renal, hepatic, gastrointestinal, hematologic, metabolic, endocrine, neurologic, psychiatric disease, or any condition that may interfere with the subject successfully completing the trial or that would present a safety risk to the subject.
4. History of severe allergy/hypersensitivity or ongoing allergy/hypersensitivity, as judged by the investigator or history of hypersensitivity to drugs with a similar chemical structure or class to CPD002.
5. Loose stools (Bristol Stool Form Score of 6 or 7) $\geq$ 2 days in the past 7 days.
6. Hepatic dysfunction (alanine aminotransaminase [ALT] or aspartate aminotransaminase [AST]) >1.5 times the upper limit of normal [ULN]) or renal impairment (serum creatinine >ULN).
7. Clinically significant laboratory results at screening as determined by the investigator.
8. Any evidence of or treatment of malignancy, excluding non- melanomatous malignancies of the skin.
9. If, in the opinion of the investigator the subject is unable or unwilling to fulfill the requirements of the protocol or has a condition, which would render the results uninterpretable.
10. Use of diuretic medications; medications that are known to affect stool consistency and/or GI motility, including fiber supplements (unless required by study), anti-diarrheals, cathartics, antacids, opiates, narcotics, prokinetic drugs, enemas, antibiotics, probiotic medications or supplements; or salt or electrolyte supplements containing sodium, potassium, chloride, or bicarbonate formulations from CPU check in (Day -2) to CPU check out (Day 17).
11. Use of an investigational agent within 30 days prior to Day -2.
12. Positive virology (active hepatitis B infection, hepatitis C infection, or human immunodeficiency virus), alcohol, or drugs of abuse test during screening.
13. Use of any prescription medication within 7 days before admission to the CPU, or required chronic use of any prescription or non-prescription medication , with the exception of hormonal replacement therapy for postmenopausal women and hormonal contraceptives.
14. History of tobacco use, alcohol abuse, illicit drug use, significant mental illness, physical dependence to any opioid, or any history of drug abuse or addiction within 12 months of study enrollment.
15. Have had significant blood loss (>450 mL) or have donated 1 or more units of blood or plasma within 8 weeks prior to study entry.

**Study drug.** CPD002-HCl capsules, CPD002-HCl tablets and CPD002 free base tablets. The CPD002 bis-HCl salt is an amorphous, off-white powder. The CPD002 free base is a white, crystalline solid. CPD002 is presented as either a white size 0 HPMC (hydroxypropylmethylcellulose) capsule or a round, white tablet. The capsules were manufactured at a dosage strength of 15 mg on the basis of the CPD002 dihydrochloride formula weight, which is equivalent to 14 mg of the CPD002 free base. To ensure comparable dosage strengths across this study, tablets of both the dihydrochloride salt and free base were manufactured at a dosage strength reflecting 14 mg on the basis of the free base. Capsules and tablets were packaged in a white HDPE (high-density polyethylene) bottle. Capsules and tablets of CPD002 were stored refrigerated (2 to 8°C) in the original packaging until use. The components of the tablets are described in **Table E11** below.

**Table E11**

| Component | Free Base | | Dihydrochloride Salt | |
|---|---|---|---|---|
| | % Form | Wt/Tablet (mg) | % Form | Wt/Tablet (mg) |
| CPD002 | 5.9 | 14.7[a] | 6.4 | 15.9[a] |
| Prosolv HD90 | 86.1 | 215.3 | 85.6 | 214.1 |
| Polyplasdone XL | 5.00 | 12.5 | 5.00 | 12.5 |
| Mg Stearate | 2.00 | 5.0 | 2.00 | 5.0 |
| Cabosil | 1.00 | 2.5 | 1.00 | 2.5 |
| Totals | 100.00 | 250.0 | 100.00 | 250.0 |

a. Corrected for purity, residual solvents, water content, and inorganic content.

**Dose and Route of Administration.** CPD002 capsules or tablets, 15 mg (14 mg free base equivalents) were administered with approximately 240 mL of non-carbonated water twice daily PO prior to breakfast and dinner for 4 consecutive days per treatment period, with 2 day wash out periods between treatments. Omeprazole 20 mg BID was administered to screened subjects beginning on day -5. All subjects took omeprazole 20 mg twice daily one hour before intake of CPD002 each day until their last dose of study drug on Day 16. See **Table E12** below.

**Table E12**

| Treatments | Subjects[a] | Dose/Administration[b] | Regimen | Formulation |
|---|---|---|---|---|
| 1 | 18 | 15 mg | BID | CPD002-HCl capsule |
| 2 | 18 | 15 mg | BID | CPD002-HCl tablet |
| 3 | 18 | 15 mg | BID | CPD002 tablet |

a. All subjects received all three treatments; 6 subjects/ treatment period. There was a 2 day wash out between each treatment period.
b. Doses are in equivalents of CPD002 free base (MW 1145.049).

Once a subject was deemed eligible for randomization, the next available randomization number was assigned sequentially and the subject received the sequence of treatment indicated on the randomization schedule. All doses of study drug were given under the supervision of clinic staff, with time, and dose administered recorded in the case report form (CRF). Clinical staff examined the subject's oral cavity and hands after drug administration to ensure that capsule was swallowed.

**Fluid and Food Intake.** Subjects participating in the study were given a standardized diet with an approximate sodium content (approximately 1500 mg for each meal). Dietary phosphorus was not measured nor was it set to a predetermined value. It was expected to range within the typical value, i.e. 750 mg - 1250 mg per day. Subjects did not have salt or any other sodium containing spices or sauces available to add to meals.

Fluid intake were ad libitum except as specified before drug administration. Daily menus (food and fluid) were similar during each treatment period.

**Pharmacodynamic variables.** The following parameters were monitored as signal of potential drug activity.

- Urine sodium excretion (daily)
- Fecal sodium excretion (daily)

Bowel movement and urine collection were performed as described earlier (Example 8); the pharmacodynamics activity of the three dosage forms was assessed as follows. A baseline fecal excretion of phosphorus or sodium was established as the average daily fecal excretion of phosphorus or sodium during Day-1 to Day 0, with the exception of one subject for whom the baseline was established during the first washout period, i.e., from Day 6 and Day 7. The daily fecal excretion of phosphorus or sodium upon treatment was measured by averaging fecal phosphorus or sodium excretion over the 4-day treatment period. The same method was used for urine.

**Results.** The results are shown in WO2014169094. Statistical analysis was performed by one-way ANOVA; (*) ; p<0.05, (**) ; p<0.01, (***) ; p<0.001.

The mean average daily excretion of phosphorus (+/-SE) is shown in WO2014169094.. A baseline fecal excretion of phosphorus or sodium was established as the average daily fecal excretion of phosphorus or sodium during Day-1 to Day 0, with the exception of one subject for whom the baseline was established during the first washout period, i.e. from Day 6 and Day 7 (referred to as "Predose"). The daily fecal excretion of phosphorus upon treatment with 15 mg BID HCl tablets was measured by averaging fecal phosphorus or sodium excretion over the 4-day treatment period.

A baseline fecal excretion of sodium was established as the average daily urinary excretion of sodium during Day-1 to Day 0, with the exception of one subject for whom the baseline was established during the first washout period, i.e. from Day 6 and Day 7 (referred to as "Predose"). The daily urinary excretion of sodium upon treatment with the three forms of drug products was measured by averaging urinary sodium excretion over the 4-day treatment period.

A baseline fecal excretion of phosphorus was established as the average daily urinary excretion of phosphorus during Day-1 to Day 0, with the exception of one subject for whom the baseline was established during the first washout period, i.e. from Day 6 and Day 7 (referred to as "Predose"). The daily urinary excretion of phosphorus upon treatment with the three forms of drug products was measured by averaging urinary sodium excretion over the 4-day treatment period.

**EXAMPLE 8**

**THE EFFECT OF RENVELA® ON THE PHARMACODYNAMICS OF CP002**

A Phase 1, single-center, randomized, open label study was designed to evaluate the effect of Renvela® on the pharmacodynamic activity of CP002 administered twice daily PO for 4 days in healthy male and female subjects.

Subjects were screened within 3 weeks of enrollment. Eighteen subjects checked in to the CPU on Day -2 before dinner. Each subject received a diet standardized for Na+ content while in the CPU. Subjects received 15 mg CP002 BID on Days 1 to 4, and Days 7 to 10.Subjects were fed breakfast and/or dinner within approximately 5 minutes after dosing. During one of the two treatment periods (randomly assigned), subjects received one Renvela® 800 mg tablet with breakfast, lunch and dinner (either Days 1 to 4 or Days 7 to 10). There was a two day wash out period between each treatment period. While confined to the CPU, Na+-standardized meals were provided per CPU procedures. Pharmacodynamic assessment included 24-hour fecal sodium and phosphorus measurements.

The subject selection criteria and description of the study drug were the same as described for Example 9 (*supra*). The schedule of assessments and procedures is shown in **Table E13** below.

**Table E13**

| **Study Procedure** | **Screening** | **Run-in** | | **Treatment Period 1** | | | | **Washout/ Run-in** | | **Treatment Period 2** | | | |
|---|---|---|---|---|---|---|---|---|---|---|---|---|---|
| Day | -21 to -3 | -2 | -1 | 1 | 2 | 3 | 4 | 5 | 6 | 7 | 8 | 9 | 10 |
| Renvela® administration | | | | X | X | X | X | | | X | X | X | X |
| CP002 administration | | | | X | X | X | X | | | X | X | X | X |
| 24 hour urine/ stool collection | | | X | X | X | X | X | X | X | X | X | X | X |
| Stool assessment | | X | X | X | X | X | X | X | X | X | X | X | X |
| PK blood sampling | | | | | | | X | | | | | | X |

**Pharmacodynamic variables.** A baseline fecal excretion of phosphorus or sodium was established as the

average daily fecal excretion of phosphorus or sodium during Day-1 to Day 0. The daily fecal excretion of phosphorus or sodium upon treatment was measured by averaging fecal phosphorus or sodium excretion over the 4-day treatment period. Sodium and phosphorus analytical methods were performed as described in Example 8 (*supra*).

**Results.** The data are shown in WO2014169094. Statistical analysis performed by one-way ANOVA followed by Tukey's multiple comparison's test; (*) ; $p<0.05$, (**) ; $p<0.01$, (***) ; $p<0.001$. vs. pre-Dose.

Here, a baseline fecal excretion of sodium was established as the average daily fecal excretion of phosphorus or sodium during Day-1 to Day 0, (referred to as "Predose"). The daily fecal excretion of sodium upon treatment with 15 mg BID HCI tablets was measured by averaging fecal sodium excretion over the 4-day treatment period.

The mean average daily fecal excretion of phoshorus (+/-SE) is shown in WO2014169094. A baseline fecal excretion of phosphorus was established as the average daily fecal excretion of phosphorus during Day-1 to Day 0, (referred to as "Predose"). The daily fecal excretion of phosphorus upon treatment with 15 mg BID HCI tablets was measured by averaging fecal phosphorus excretion over the 4-day treatment period.

## Example 9

### Combination Treatment with Tenapanor and Sevelamer

*Animals*

A total of 48, approximately 8-week-old male Sprague Dawley (SD) rats (Envigo, Livermore, CA), weighing approximately 250 g on arrival, were housed 2-per cage in micro-isolator cages for a 48-hr acclimation period prior to being assigned to study groups. Animals were housed in a temperature (65-75°F) and humidity (35-55%) controlled facility utilizing a reversed 12-hour light/dark cycle (7AM - 7PM dark cycle). Rats had *ad libitum* access to standard rodent chow, spiked with an additional 0.4% inorganic phosphorous (1:1 sodium:potassium salt, TD.160470, 1.1% w/w total phosphorous content, Harlan-Teklad, Madison, WI), and deionized drinking water for the duration of the study. Body weights were recorded at study initiation and daily for the remainder of the study. Animals were maintained in accordance with the Guide for the Care and Use of Laboratory Animals (National Research Council, 2011). The study was conducted under a protocol approved by the Institutional Animal Care and Use Committee.

*Study Design*

Rats were randomly assigned to one of eight groups (n=6/group) provided with a diet containing varying amounts of sevelamer (0-3% w/w) and were dosed by oral gavage twice daily with vehicle (0.01% Tween80) or tenapanor (0.15 mg/kg) for 11-consecutive days according to the study assignments shown in the following table.

| Study Group Assignments | | | | | | | | |
|---|---|---|---|---|---|---|---|---|
| **Group** | Group 1 | Group 2 | Group 3 | Group 4 | Group 5 | Group 6 | Group 7 | Group 8 |
| Oral Treatment | Vehicle | Tenapano r | Vehicle | Vehicle | Vehicle | Tenapano r | Tenapano r | Tenapano r |
| Tenapanor dose (mg/kg) | 0 | 0.15 | 0 | 0 | 0 | 0.15 | 0.15 | 0.15 |
| Dosing Vol (ml/kg) | 5 | 5 | 5 | 5 | 5 | 5 | 5 | 5 |
| Dose Route: | PO | PO | PO | PO | PO | PO | PO | PO |
| Dosing Schedule: | BID | BID | BID | BID | BID | BID | BID | BID |
| Sevelamer dose (% w/w) in chow | 0 | 0 | 0.75 | 1.5 | 3 | 0.75 | 1.5 | 3 |

Rats dosed orally with vehicle and provided diet without sevelamer (Group 1) served as the control group. Treatment

arms included groups treated with tenapanor alone (0.15 mg/kg, bid), sevelamer alone (0.75, 1,5 or 3% w/w in chow) and the combination of tenapanor (0.15 mg/kg, bid) with each dose level of sevelamer (0.75, 1,5 or 3% w/w in chow). Animals were dosed in their home cages while pair-housed for the first five treatment days. On treatment day 6, all animals were housed individually in metabolic cages for the remainder of the study and continued to be provided ad *libitum* access to diet containing 0-3% sevelamer w/w and dosed twice daily with vehicle or tenapanor. Following two days acclimation to the metabolic cages, 24-hour food intake, water intake and urinary volume were measured and recorded for the final four treatment days. Urinary sodium and phosphorous concentration was measured by ion chromatography (IC). 24-hour urinary sodium and phosphorous excretion were calculated by multiplying ion concentration by 24-hour urine volume. Urinary ion excretion was also normalized to daily dietary ion intake to account for daily fluctuations in food intake. A terminal blood sample was collected by cardiac puncture under isoflurane anesthesia, for the measurement of serum sodium and phosphate (Alera, Alpha Wassermann, West Caldwell, NJ) to allow the calculation of the renal clearance of sodium and phosphate to further assess renal ion handling.

*Dosing Solution and Diet Formulation*

Dosing solutions containing tenapanor (Ardelyx, Fremont CA) at a concentration of 0.03 mg/mL were made fresh weekly by adding the appropriate weight of tenapanor to 0.1% Tween 80 vehicle. The dose volume was 5 mL/kg for all groups. The phosphate spiked standard rodent chow, was weighed out into mixing bowls. The appropriate amount of sevelamer carbonate (Renvela, Genzyme) was added to the powdered diet (sevelamer 0, 0.75, 1.5 or 3% w/w) in the mixing bowl, placed on a stand mixer (KitchenAid) and set to speed Stir-2 for 10 min.

*Dose Administration*

Animals were dosed twice daily (bid) orally (po), immediately prior to lights out (7AM) and during the middle of dark cycle (2PM) with a standard gavage needle (38mm, 20G). Animals were removed from the holding or metabolic cages for dosing and then immediately returned to the cage of origin.

*Urinary Ion Analysis by Ion Chromatography*

Urine samples were analyzed for sodium and phosphorous content on an ion chromatography system (Thermo Fisher Scientific ICS-3000 or ICS-5000+) coupled with conductivity detectors. Chromatographic separation of cations was performed using an IonPac CS12A (Thermo Fisher) 2 × 250 mm analytical column with an isocratic elution using 25 mM methanesulfonic acid. Chromatographic separation of anions was performed using an IonPac AS18 (Thermo Fisher) 2 × 250 mm analytical column with an isocratic elution using 35 mM potassium hydroxide. Concentrations were interpolated from a standard curve (prepared in 10 mM hydrochloric acid) for each analyte ion based on retention time and peak area.

*Statistical Analysis*

All data sets for each group are represented by mean ± SEM. Statistical analysis was performed using one-way ANOVA followed by Tukey's post hoc test to correct for multiple comparisons and enable individual group comparisons. Statistical significance between groups was marked as *, P<0.05; **, P<0.01; and ***, P<0.001 compared to the vehicle group. The BLISS model of independence was used to statistically determine if the combination of tenapanor and sevelamer was independent, synergistic or antagonistic. The BLISS independence model is defined by the following equation comparing the predicted combination response calculated using the complete additivity of probability theory based on the observed effect of each individual agent administered alone, and compared to the observed effect of combination treatment:

$$Y_{ab,P} = Y_a + Y_b - Y_a Y_b$$

Where; $Y_a$ = % efficacy Drug A at Dose A, $Y_b$ = % efficacy Drug B at Dose B, $Y_{ab,P}$ = predicted efficacy of combination and $Y_{ab,O}$ = observed efficacy of combination

And comparing predicted combination efficacy to observed combination efficacy; $Y_{ab,O} > Y_{ab,P}$ = synergy, $Y_{ab,O} = Y_{ab,P}$ = independent (additive) and $Y_{ab,O} < Y_{ab,P}$ = antagonism

Results

*Effects on Body Weight, Food Intake and Calculating the Administered Sevelamer Dose*

Body weight and food intake were not significantly affected by tenapanor, sevelamer or combination treatment compared to control. As sevelamer was administered as a diet admixture, the actual dose of sevelamer delivered in the food was calculated based on food consumption. There was a linear increase in sevelamer dose with increasing sevelamer content in the food, the sevelamer dose was stable over time and co-administration of tenapanor did not significantly affect the dose of sevelamer consumed in the food. As a result, the dose of sevelamer was well matched between tenapanor and non-tenapanor treated groups consuming sevelamer allowing direct comparison of the combination effect. The sevelamer dose administered on the final day of the study, along with the conversion to human equivalent dose based on body surface area conversion (1/7) is summarized in the following table.

Mean ± SEM Sevelamer Dose Administered via Diet Admixture on the Final Treatment Day in SD Rats with and without Tenapanor, along with the Approximate Human Equivalent Dose of Sevelamer Based on Body Surface Area Correction

| Group Number | Sevelamer %w/w Diet Admix | Tenapanor Dose (mg/kg, po bid) | Sevelamer Dose (mg/kg/day) | ~Human Equivalent Dose (g/day) * |
|---|---|---|---|---|
| 3 | 0.75 | 0 | 567 ± 19 | 4.9 |
| 4 | 1.5 | 0 | 1126 ± 21 | 9.6 |
| 5 | 3 | 0 | 2397 ± 71 | 20.5 |
| 6 | 0.75 | 0.15 | 522 ± 28 | 4.5 |
| 7 | 1.5 | 0.15 | 1121 ± 26 | 9.6 |
| 8 | 3 | 0.15 | 2305 ± 31 | 19.8 |
| SEM = Standard Error Mean<br>*60 kg human; rat to human surface area dose conversion factor 1/7 | | | | |

The recommended human starting dose of sevelamer is 2.4 to 4.8 g/day; therefore, on a dose conversion basis, the 0.75% sevelamer dose in rats is roughly equivalent to the recommended human starting dose of sevelamer.

*Effect on Urinary Phosphorous Excretion*

24-hr urinary phosphorous excretion over the final four days of treatment is shown in Figure 4A, with the final treatment day shown in Figure 4B for clarity. Tenapanor treatment alone, significantly decreased urinary phosphorous excretion compared to vehicle control. Sevelamer significantly and dose-dependently decreased urinary phosphorous excretion and in combination with tenapanor, sevelamer dose-dependently decreased urinary phosphorous excretion such that combination reductions were significantly greater than either tenapanor or the equivalent dose of sevelamer alone across all sevelamer dose levels. Normalization of urinary phosphorous excretion to dietary phosphorous intake to account for any fluctuations in daily intake over the final four days of treatment is shown in Figure 4C, with the final treatment day shown alone in Figure 4D for clarity. The enhanced urinary phosphorous lowering of the tenapanor and sevelamer combination, relative to tenapanor or the equivalent dose of sevelamer alone is confirmed by this analysis and rules out variations in dietary phosphorous intake as a confounding influence.

The BLISS model of independence was used to assess the interaction between tenapanor and sevelamer and is summarized in the following table.

Comparison of Observed and BLISS Model Predicted Mean Reductions in Urinary Phosphorous Excretion Compared to the Control Group Across the Four Day Collection Period

| | % Reduction# Urinary Phosphorous Excretion When Dosed Alone | Predicted* combination % Reduction Urinary Phosphorous Excretion | Observed combination % Reduction Urinary Phosphorous Excretion | Outcome | Interaction |
|---|---|---|---|---|---|
| Tenapanor | 33.2% | N/A | N/A | N/A | N/A |
| Sevalamer 0.75% | 21.0% | 47.2% | 64.8% | Observed > predicted | Synergy |
| Sevalamer 1.5% | 57.9% | 71.2% | 80.0% | Observed > predicted | Synergy |
| Sevalamer 3.0% | 76.5% | 84.3% | 90.2% | Observed > predicted | Synergy |
| # Calculated relative to control group 1<br>*prediction based on BLISS model<br>N/A not applicable | | | | | |

The average urinary phosphorous excretion over the entire 4-day collection period for each treatment group was used for this analysis to ensure all collected data points were included in the assessment of combination effects. The observed combination treatment reductions in urinary phosphorous excretion relative to vehicle control (Group 1), were then compared to the predicted combination treatment reductions in urinary phosphorous excretion based on the BLISS model. The observed combination treatment reduction in urinary phosphorous excretion was greater than the predicted reduction based on the single agent effects according to the BLISS model for all sevelamer doses in combination with tenapanor; an indicator of synergy. The difference in the observed (64.8% reduction) from predicted (47.2% reduction) combination effect with tenapanor was greatest at the lowest (0.75% w/w) sevelamer dose. This suggests tenapanor and sevelamer behave synergistically to reduce urinary phosphorous excretion, an index of intestinal phosphate absorption, especially at lower, clinically relevant sevelamer doses.

*Effect on Urinary Sodium Excretion*

Tenapanor treatment significantly decreased urinary sodium excretion compared to vehicle control, whereas sevelamer alone did not significantly affect urinary sodium excretion. Tenapanor also significantly reduced urinary sodium excretion compared to control when co-administered with all doses of sevelamer. The 0.75% and 1.5% w/w doses of sevelamer did not significantly affect the magnitude of tenapanor's sodium lowering effect. However, the highest dose of sevelamer (3% w/w) did modestly, but significantly attenuate the sodium lowering effect of tenapanor. Normalization of urinary sodium excretion to dietary sodium intake to account for any fluctuations in daily intake over the final four days of treatment demonstrated that the observations on urinary sodium excretion were not confounded by variations in sodium intake. The BLISS model confirms that the observed urinary sodium lowering with the combination of tenapanor and 3% w/w sevelamer was less than the predicted urinary sodium lowering based on individual agent treatment (table below), indicating that the high dose of sevelamer (3% w/w), equivalent to approximately 20 g/day of sevelamer in humans and likely not clinically relevant, attenuates the sodium lowering pharmacodynamic effect of tenapanor.

Comparison of Observed and BLISS Model Predicted Mean Reductions in Urinary Sodium Excretion Compared to the Control Group Across the Four Day Collection Period

| | % Reduction# Urinary Sodium Excretion When Dosed Alone | Predicted* combination % Reduction Urinary Sodium Excretion | Observed combination % Reduction Urinary Sodium Excretion | Outcome | Interaction |
|---|---|---|---|---|---|
| Tenapanor | 82.1% | N/A | N/A | N/A | N/A |
| Sevalamer 0.75% | -8.4% | 80.6% | 86.1% | Observed ~ predicted | Independent |
| Sevalamer 1.5% | 7.7% | 83.5% | 76.8% | Observed ~ predicted | Independent |

(continued)

| | % Reduction# Urinary Sodium Excretion When Dosed Alone | Predicted* combination % Reduction Urinary Sodium Excretion | Observed combination % Reduction Urinary Sodium Excretion | Outcome | Interaction |
|---|---|---|---|---|---|
| Sevalamer 3.0% | -10.7% | 80.2% | 55.6% | Observed < predicted | Antagonism |
| # Calculated relative to control group 1<br>*prediction based on BLISS model<br>N/A not applicable | | | | | |

*Effects on Renal Clearance of Phosphorous and Sodium*

Tenapanor and sevelamer alone significantly decreased renal clearance of phosphorous, reflective of the appropriate renal response in the face of decreased intestinal phosphorous absorption. In combination with tenapanor, sevelamer further and dose-dependently reduced phosphorous clearance such that combination reductions were significantly more than either tenapanor or the equivalent dose of sevelamer alone. Tenapanor alone significantly decreased renal sodium clearance, reflective of the appropriate renal response to conserve sodium in the face of decreased intestinal sodium absorption. Sevelamer alone had no effect on renal sodium handling since it did not affect intestinal sodium absorption. Renal sodium clearance was not significantly affected by sevelamer at 0.75% or 1.5% w/w when combined with tenapanor; however, compared to tenapanor alone, combination with sevelamer at 3% w/w resulted in a significantly higher renal sodium clearance reflective of the attenuated sodium lowering pharmacodynamic effect of tenapanor when combined with this high dose of sevelamer.

**DISCLOSED ITEMS**

The following disclosed items also form part of the invention:

1. A method for lowering serum phosphate in a patient comprising administering an effective amount of an epithelial phosphate transport inhibitor in combination with a phosphate binder, wherein the amount of the phosphate binder administered is less than the amount that would be administered as a single agent.

2. The method of item 1, wherein phosphate binder is selected from the group consisting of sevelamer, lanthanum carbonate, calcium carbonate, calcium acetate, calcium acetate/magnesium carbonate, MCI-196, ferric citrate, sucroferric oxyhydroxide, magnesium iron hydroxycarbonate, aluminum hydroxide, APS1585, SBR-759, and PA-21.

3. The method of item 1, wherein the epithelial phosphate transport inhibitor is a compound that lowers the pH of epithelial cells.

4. The method of item 3, wherein the compound is selected from the group consisting of an NHE3 inhibitor, guanylate cyclase C receptor (GC-C) agonist, P2Y agonist, adenosine A2b receptor agonist, soluble guanylate cyclase agonist, adenylate cyclase receptor agonist, imidazoline-1 receptor agonist, cholinergic agonist, prostaglandin EP4 receptor agonist, dopamine D1 agonist, melatonin receptor agonist, 5HT4 agonist, atrial natriuretic peptide receptor agonist, carbonic anhydrase inhibitor, phosphodiesterase inhibitor, and Down-Regulated in Adenoma (DRA or SLC26A3) agonist.

5. The method of any one of items 1-4, wherein the epithelial phosphate transport inhibitor is an NHE3 inhibitor having a structure of Formula (I) or (IX):

NHE-Z (I)

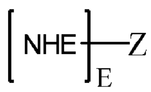

(IX)

wherein:

NHE is a NHE-binding small molecule that comprises (i) a hetero-atom containing moiety, and (ii) a cyclic or heterocyclic scaffold or support moiety bound directly or indirectly thereto, the heteroatom-containing moiety being selected from a substituted guanidinyl moiety and a substituted heterocyclic moiety, which may optionally be fused with the scaffold or support moiety to form a fused bicyclic structure; and,
Z is a moiety having at least one site thereon for attachment to the NHE-binding small molecule, the resulting NHE-Z molecule possessing overall physicochemical properties that render it substantially impermeable or substantially systemically non-bioavailable; and,
E is an integer having a value of 1 or more.

6. The method of any of items 1-4, wherein the compound is an oligomer, dendrimer or polymer, and further wherein Z is a Core moiety having two or more sites thereon for attachment to multiple NHE-binding small molecules, either directly or indirectly through a linking moiety, L, the compound having the structure of Formula (X):

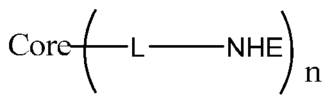

(X)

wherein L is a bond or linker connecting the Core to the NHE-binding small molecule, and n is an integer of 2 or more, and further wherein each NHE-binding small molecule may be the same or differ from the others.

7. The method of item 6, wherein the NHE-binding small molecule has the structure of Formula (IV):

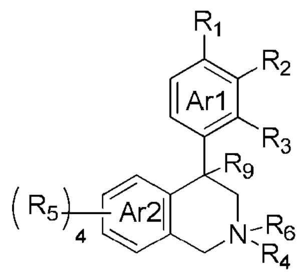

(IV)

or a stereoisomer, prodrug or pharmaceutically acceptable salt thereof,
wherein:

each $R_1$, $R_2$, $R_3$, $R_5$ and $R_9$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or a bond linking the NHE-binding small molecule to L, provided at least one is a bond linking the NHE-binding small molecule to L;
$R_4$ is selected from H, $C_1$-$C_7$ alkyl, or a bond linking the NHE-binding small molecule to L;
$R_6$ is absent or selected from H and $C_1$-$C_7$ alkyl; and
Ar1 and Ar2 independently represent an aromatic ring or a heteroaromatic ring.

8. The method of item 6, wherein the NHE-binding small molecule has the following structure:

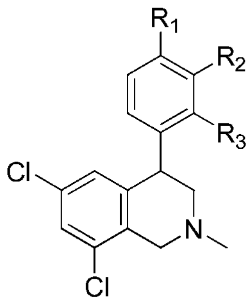

or a stereoisomer, prodrug or pharmaceutically acceptable salt thereof,
wherein:
each $R_1$, $R_2$ and $R_3$ are independently selected from H, halogen, $-NR_7(CO)R_8$, $-(CO)NR_7R_8$, $-SO_2-NR_7R_8$, $-NR_7SO_2R_8$, $-NR_7R_8$, $-OR_7$, $-SR_7$, $-O(CO)NR_7R_8$, $-NR_7(CO)OR_8$, and $-NR_7SO_2NR_8$, where $R_7$ and $R_8$ are independently selected from H or a bond linking the NHE-binding small molecule to L, provided at least one is a bond linking the NHE-binding small molecule to L.

9. The method of item 6, wherein the NHE-binding small molecule has one of the following structures:

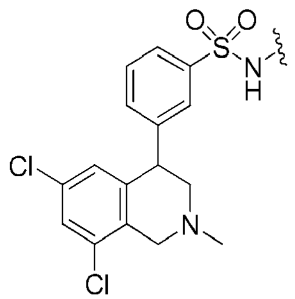

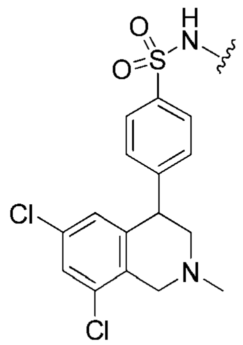

or

or a stereoisomer, prodrug or pharmaceutically acceptable salt thereof.

10. The method of item 1, wherein the NHE3 inhibitor is

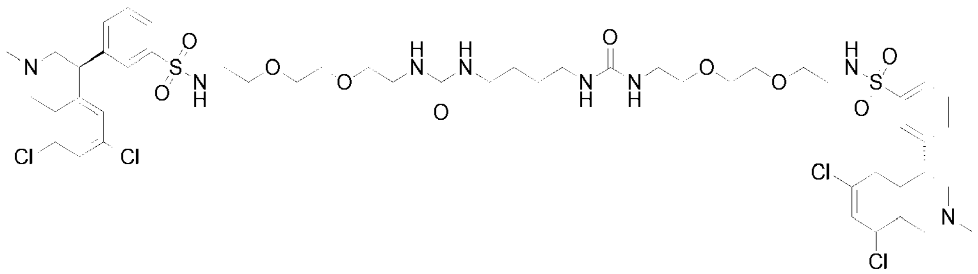

or a pharmaceutically acceptable salt thereof.

11. A pharmaceutical composition comprising an epithelial phosphate transport inhibitor and a phosphate binder, wherein the amount of the phosphate binder administered is less than the amount that would be administered as a single agent.

12. The composition of item 11, wherein the epithelial phosphate transport inhibitor is tenapanor.

13. The composition of item 11, wherein the amount of phosphate binder is 50% of the amount that would be administered as a single agent.

14. The composition of item 11, wherein the amount of phosphate binder is about 40% of the amount that would be

administered as a single agent.

15. The composition of item 11, wherein the amount of phosphate binder is about 30% of the amount that would be administered as a single agent.

16. The composition of item 11, wherein the amount of phosphate binder is about 20% of the amount that would be administered as a single agent.

## Claims

**1.** A combination comprising tenapanor and a phosphate binder for use in lowering serum phosphate in a patient, wherein the amount of the phosphate binder to be administered in the combination is 5%-50% of the amount of said phosphate binder that would be administered as a single agent.

**2.** The combination for the use of claim 1, wherein the amount of phosphate binder is 5%-10%, 10%-15%, 15%-20%, 20%-25%, 25%-30%, 30%-35%, 35%-40%, 40%-45%, or 45%-50% of the amount that would be administered as a single agent.

**3.** The combination for the use of claim 1 or claim 2, wherein the phosphate binder is sevelamer, preferably wherein the amount of sevelamer is about 5-10% the amount that would be administered as a single agent.

**4.** The combination for the use of claim 3, wherein the amount of sevelamer is 100-200 mg.

**5.** The combination for the use of any one of claims 1-4, wherein the amount of tenapanor is 30 mg, to be administered twice per day.

**6.** The combination for the use of claim 1, wherein the phosphate binder is ferric citrate.

**7.** The combination for the use of claim 6, wherein the amount of ferric citrate is about 30% the amount that would be administered as a single agent.

**8.** The combination for the use of claim 1 or claim 2, wherein the phosphate binder is sucroferric oxyhydroxide, preferably wherein the amount of sucroferric oxyhydroxide is 5%-10% of the amount that would be administered as a single agent.

**9.** A pharmaceutical composition comprising tenapanor and a phosphate binder, wherein the amount of the phosphate binder administered is 5%-50% of the amount of said phosphate binder that would be administered as a single agent.

**10.** The pharmaceutical composition of claim 9, wherein the amount of phosphate binder is 5%-10%, 10%-15%, 15%-20%, 20%-25%, 25%-30%, 30%-35%, 35%-40%, 40%-45%, or 45%-50% of the amount that would be administered as a single agent.

**11.** The composition of claim 9 or claim 10, wherein the phosphate binder is sevelamer.

**12.** The composition of claim 11, wherein the amount of sevelamer is about 5-10% of the amount that would be administered as a single agent.

**13.** The composition of claim 12, wherein the phosphate binder is sevelamer in an amount of about 100-200 mg.

**14.** The composition of claim 9 or claim 10, wherein the phosphate binder is sucroferric oxyhydroxide.

**15.** The composition of claim 14, wherein the amount of sucroferric oxyhydroxide is about 5%-10% of the amount that would be administered as a single agent.

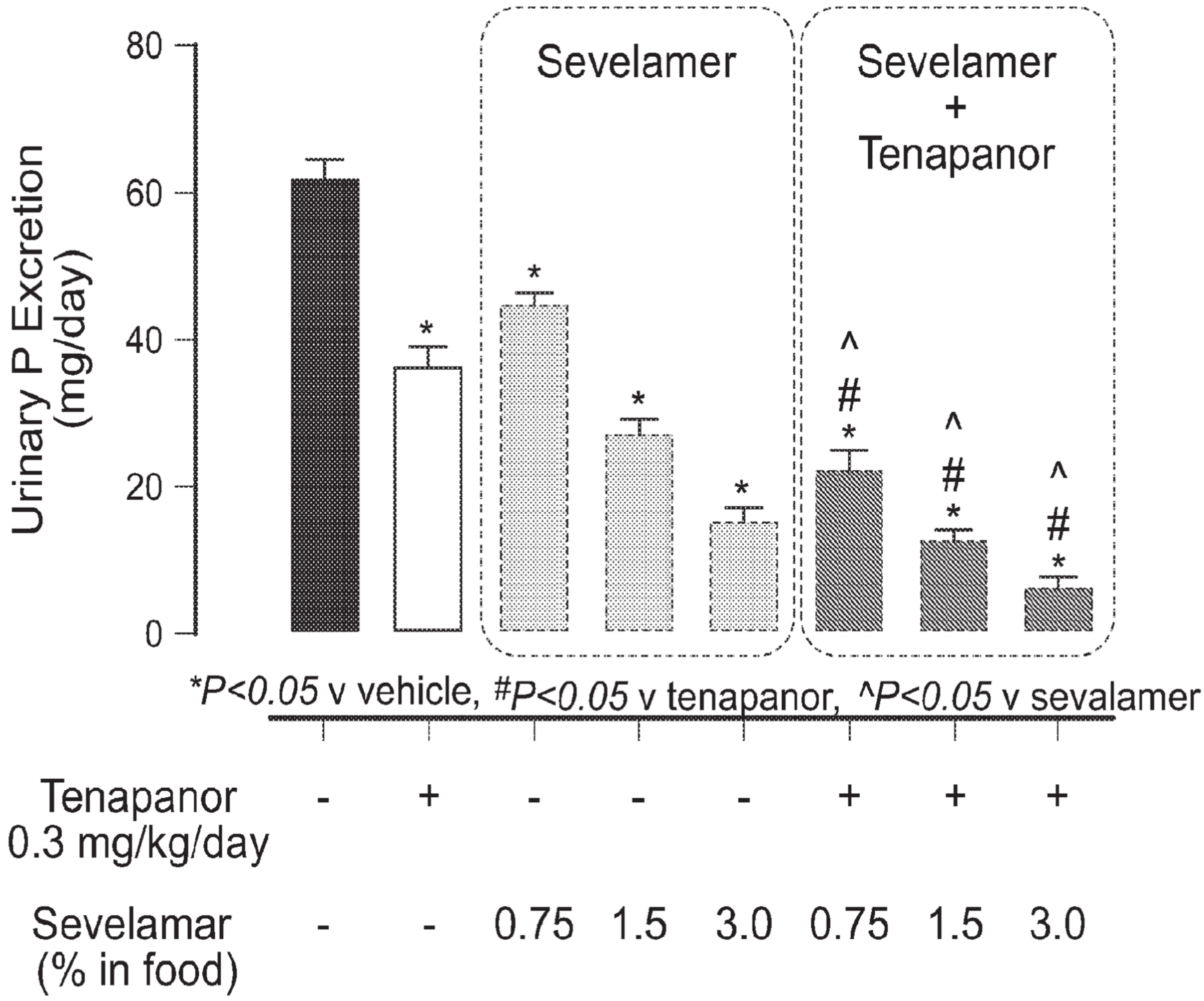
Urinary P Excretion (mg/day)
80
60
40
20
0
Sevelamer
Sevelamer + Tenapanor
*P<0.05 v vehicle, #P<0.05 v tenapanor, ^P<0.05 v sevalamer
Tenapanor 0.3 mg/kg/day - + - - - + + +
Sevelamar (% in food) - - 0.75 1.5 3.0 0.75 1.5 3.0

FIGURE 1

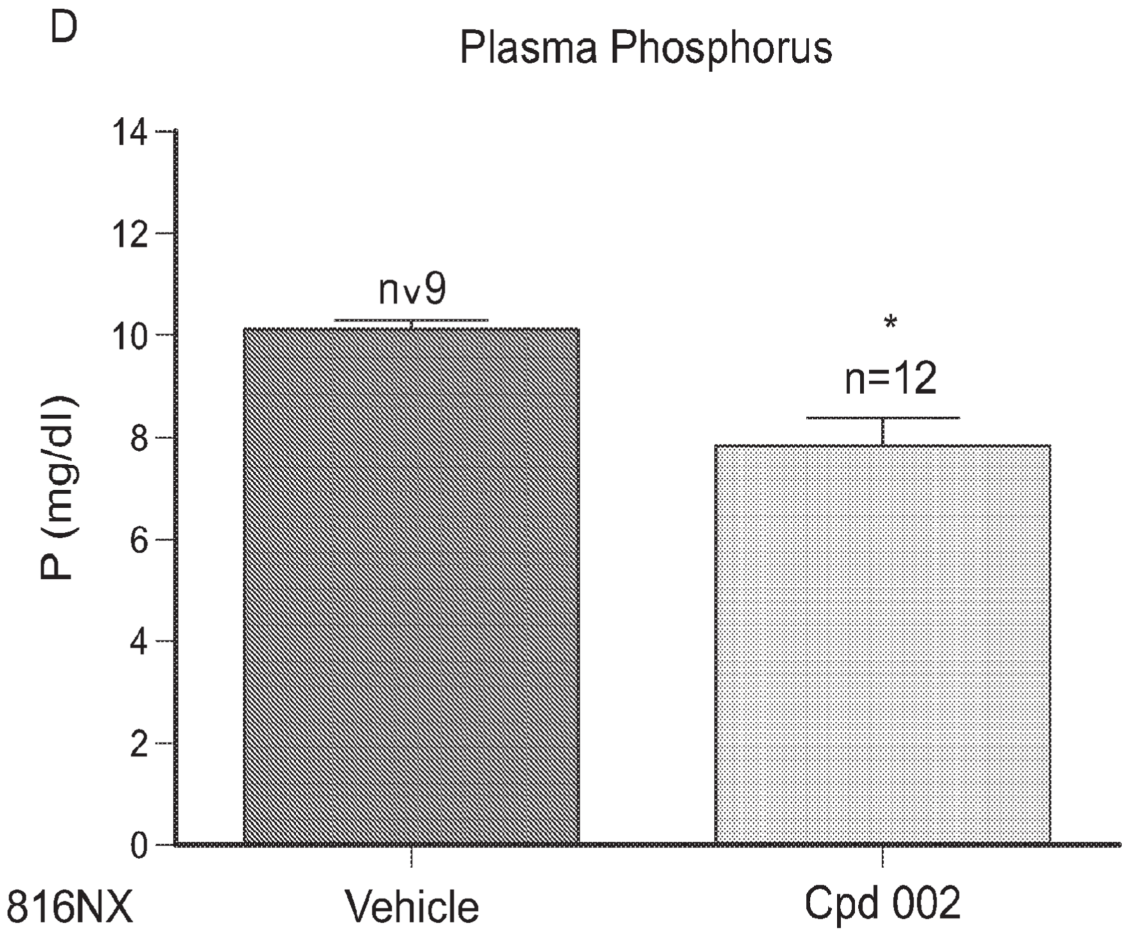
D
Plasma Phosphorus
14
12
10
8
6
4
2
0
P (mg/dl)
nv9
*
n=12
816NX
Vehicle
Cpd 002

FIGURE 2

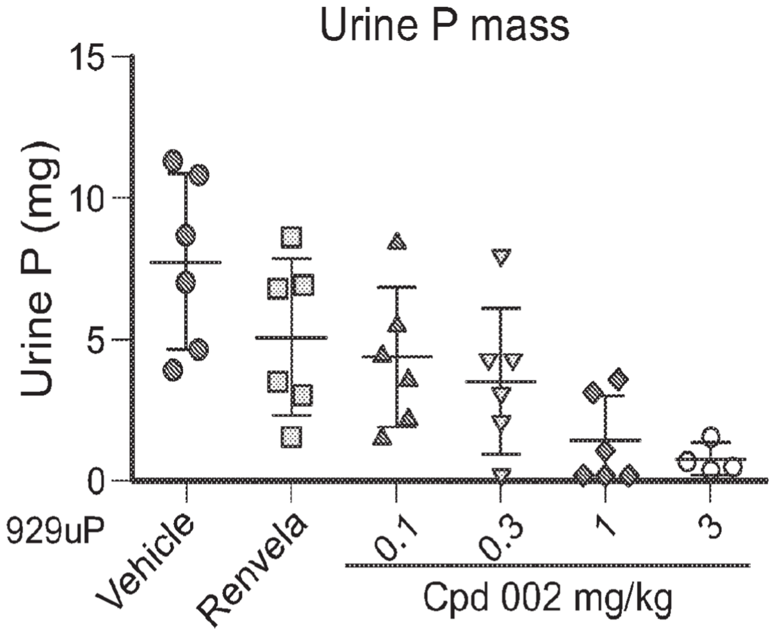


FIGURE 3A

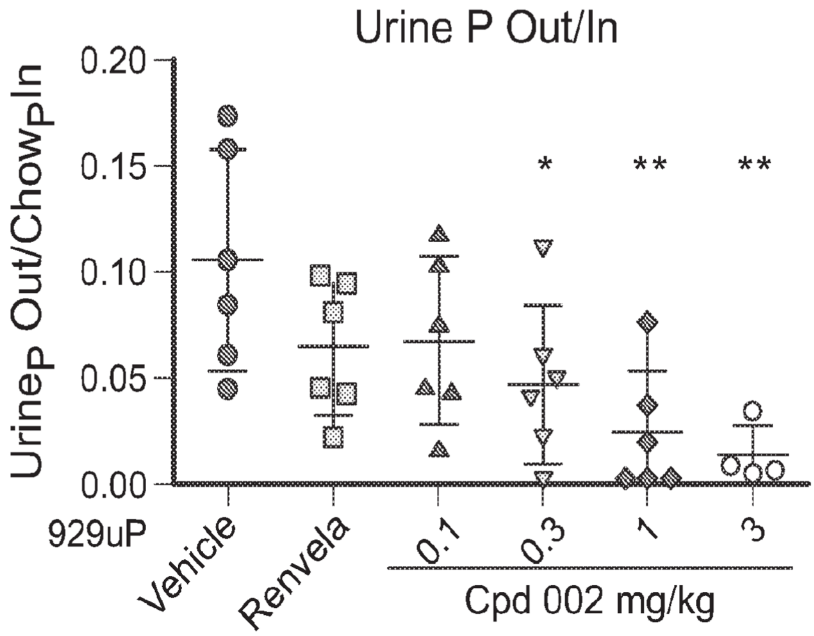


FIGURE 3B

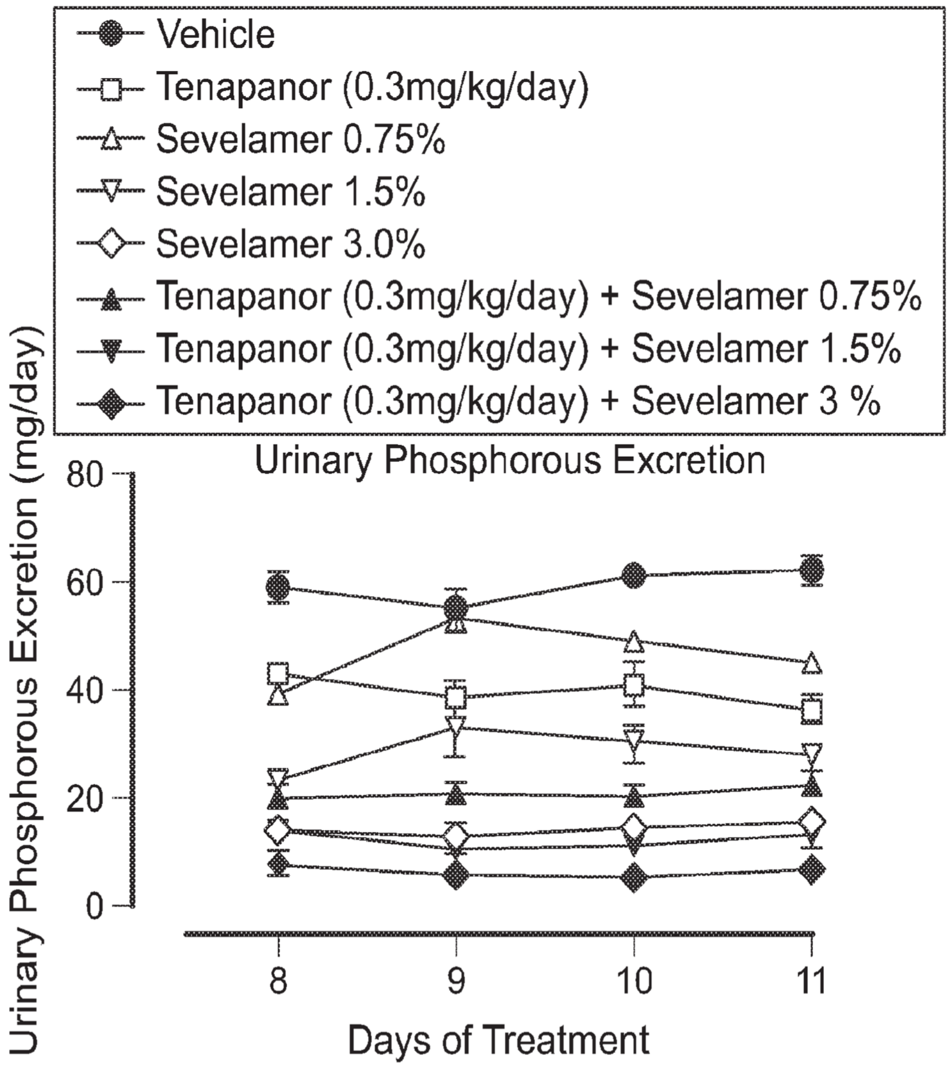


FIGURE 4A

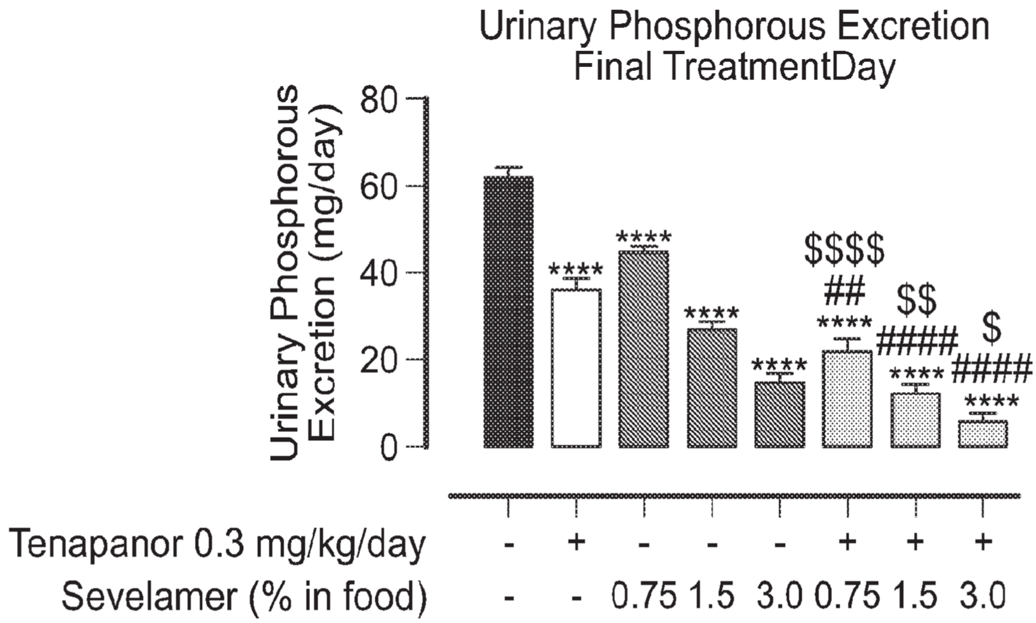


FIGURE 4B

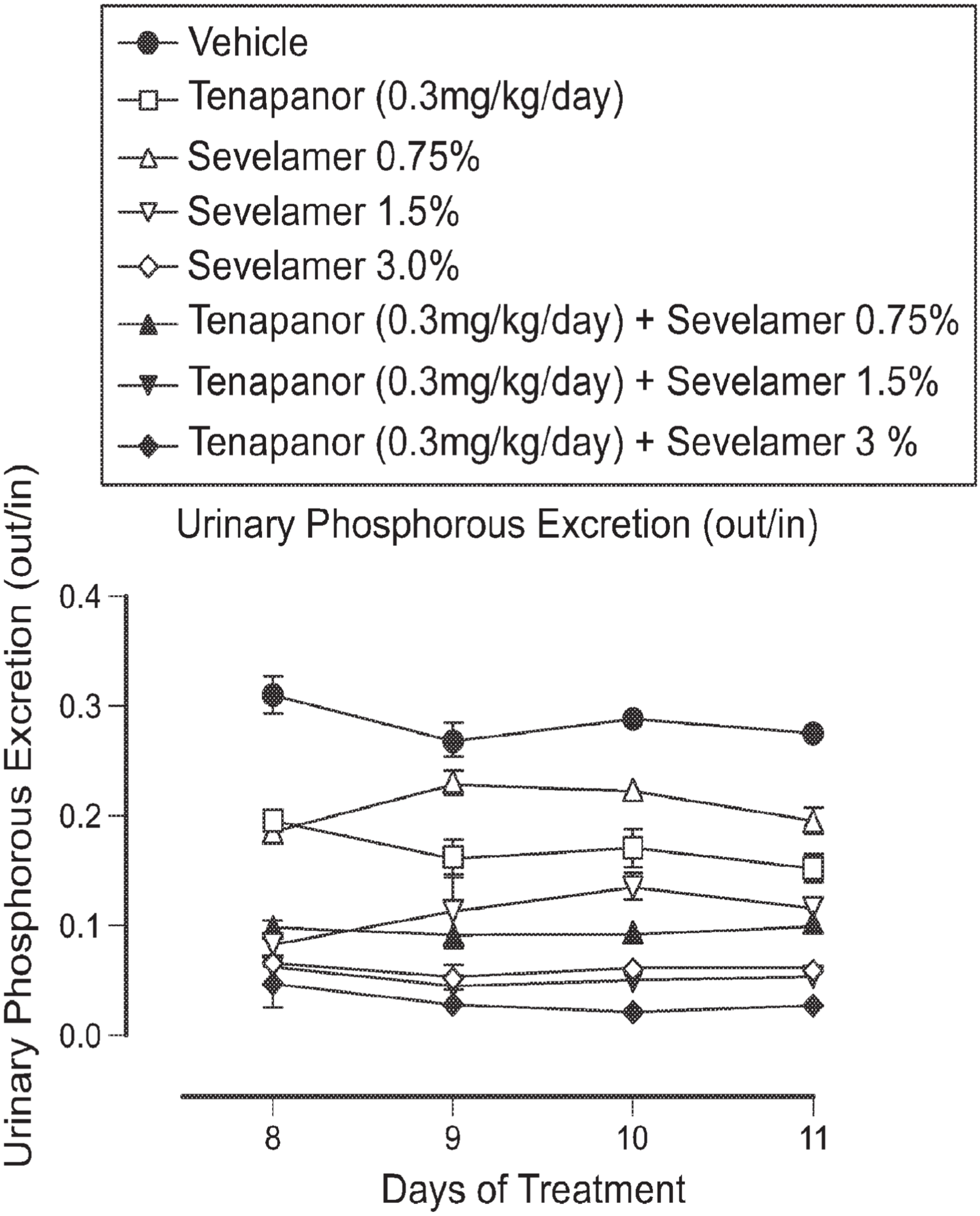
Vehicle
Tenapanor (0.3mg/kg/day)
Sevelamer 0.75%
Sevelamer 1.5%
Sevelamer 3.0%
Tenapanor (0.3mg/kg/day) + Sevelamer 0.75%
Tenapanor (0.3mg/kg/day) + Sevelamer 1.5%
Tenapanor (0.3mg/kg/day) + Sevelamer 3 %
Urinary Phosphorous Excretion (out/in)
Urinary Phosphorous Excretion (out/in)
0.4
0.3
0.2
0.1
0.0
8
9
10
11
Days of Treatment

FIGURE 4C

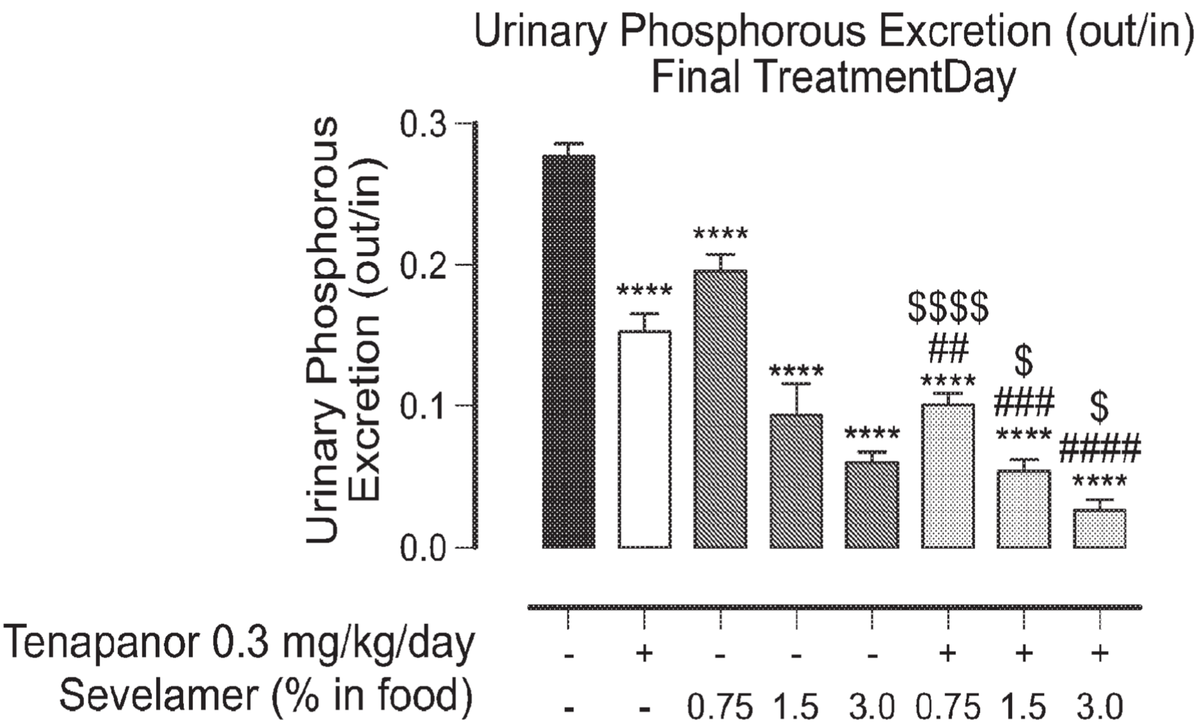
Urinary Phosphorous Excretion (out/in)
Final TreatmentDay
Urinary Phosphorous Excretion (out/in)
0.3
0.2
0.1
0.0
****
****
****
****
$$$$
##
****
$
###
****
$
####
****
Tenapanor 0.3 mg/kg/day - + - - - + + +
Sevelamer (% in food) - - 0.75 1.5 3.0 0.75 1.5 3.0

FIGURE 4D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62851099
- US 62852299
- WO 2010078449 A
- US 6399824 B
- US 20050054705 A
- CA 2241531
- WO 9724113 A
- US 5900436 A
- EP 0822182 B1
- US 6911453 B
- US 6703405 B
- US 20040039001 A
- US 20040224965 A
- US 20050113396 A
- US 20050020612 A
- US 200500020612 A
- WO 20050020612 A
- US 6005010 A
- US 6166002 A
- US 20080194621 A
- US 20070225323 A
- US 5866610 A
- US 6887870 B
- US 6737423 B
- US 7326705 B
- US 55824691 B
- WO 94026709 A
- WO 02024637 A
- US 20040339001 A
- WO 02020496 A
- WO 03055490 A
- WO 01072742 A
- CA 2387529
- WO 01021582 A
- CA 02241531
- WO 97024113 A
- WO 03051866 A
- US 20070135383 A
- US 20070135385 A
- US 20050244367 A
- US 20070270414 A
- CA 2177007
- EP 0744397 A
- WO 2015021358 A
- US 7041786 B
- US 7304036 B
- US 7371727 B
- US 7494979 B
- US 7704947 B
- US 7799897 B
- US 7745409 B
- US 7772188 B
- US 7879802 B
- US 7910546 B
- US 8034782 B
- US 8080526 B
- US 8101579 B
- US 8114831 B
- US 8110553 B
- US 8357775 B
- US 8367800 B
- US 20130096071 A
- US 20130190238 A
- US 20120040892 A
- US 20120040025 A
- US 20120213846 A
- US 20120289460 A
- US 20110118184 A
- US 20100152118 A
- US 20100048489 A
- US 20100120694 A
- US 20100261877 A
- US 20090253634 A
- US 20090192083 A
- US 20090305993 A
- WO 2006086653 A
- WO 2002098912 A
- US 7087644 B
- WO 2013101830 A
- US 5350864 A
- US 4871764 A
- US 20110171195 A
- US 20060004090 A
- US 20110077292 A
- US 4954642 A
- US 20120202288 A
- US 20010056060 A
- US 20020040149 A
- US 20050164949 A
- US 20110098481 A
- US 4219479 A
- US 4049582 A
- US 4423067 A

- US 4474802 A
- US 4692464 A
- US 4708963 A
- US 5010065 A
- US 5013758 A
- US 6747037 B
- US 7776896 B
- EP 0084856 A
- CA 1248525
- US 20040102499 A
- US 2005049227 A
- US 2005228185 A
- US 2006106088 A
- US 2006111430 A
- US 20070010495 A
- US 20070123568 A
- US 20070123569 A
- US 20050020686 A
- US 20080234337 A
- US 20100010222 A
- US 20100216689 A
- US 20040198701 A
- US 20040204590 A
- US 20050227969 A
- US 20050239872 A
- US 20060154899 A
- US 20060167081 A
- US 20060258726 A
- US 20060270721 A
- US 20090105234 A
- US 20090105321 A
- US 20090247596 A
- US 20090258918 A
- US 20090270395 A
- US 20040087624 A
- US 20040102508 A
- US 20060252799 A
- US 20090030061 A
- US 20090170931 A
- US 20100022650 A
- US 20090312388 A
- US 20090318523 A
- US 20100069457 A
- US 20100076048 A
- US 20070066618 A
- US 20040259921 A
- US 20050065133 A
- US 20070191319 A
- WO 20044071428 A
- WO 2006052630 A
- WO 2006047476 A
- WO 2006058080 A
- WO 2004065365 A
- WO 2003047513 A
- WO 2004085421 A
- WO 2004085430 A
- WO 2005116010 A
- WO 2007014454 A
- WO 2006080323 A
- WO 2006137472 A
- US 20050164987 A
- US 20030044909 A
- US 20090325949 A
- DE 1470341
- DE 2108438
- DE 2123328
- DE 2305339
- DE 2305575
- DE 2315801
- DE 2402908
- DE 2413935
- DE 2451417
- DE 2459090
- DE 2646469
- DE 2727481
- DE 2825048
- DE 2837161
- DE 2845220
- DE 2847621
- DE 2934747
- DE 3021792
- DE 3038166
- DE 3044568
- DE 3142982
- DE 1116676
- DE 2162096
- EP 000718 A
- EP 0008408 A
- EP 0010759 A
- EP 0059948 A
- EP 0075436 A
- EP 0096517 A
- EP 0112987 A
- EP 0116948 A
- EP 0150937 A
- EP 0158380 A
- EP 0161632 A
- EP 0161918 A
- EP 0167121 A
- EP 0199127 A
- EP 0220044 A
- EP 0247725 A
- EP 0258191 A
- EP 0272910 A
- EP 0272914 A
- EP 0294647 A
- EP 0300726 A
- EP 0335386 A
- EP 0357788 A
- EP 0389282 A
- EP 0406958 A
- EP 0426180 A
- EP 0428302 A
- EP 0435811 A
- EP 0470805 A
- EP 0482208 A

- EP 0490823 A
- EP 0506194 A
- EP 0511865 A
- EP 0527117 A
- EP 0626939 A
- EP 0664289 A
- EP 0671389 A
- EP 0685474 A
- EP 0685475 A
- EP 0685479 A
- EP 0293063 A
- EP 0463756 A
- EP 0579496 A
- EP 0667345 A
- EP 0163965 A
- EP 0393500 A
- EP 0510562 A
- EP 0553174 A
- JP 92234389 B
- JP 94329652 B
- JP 95010875 B
- US 4963561 A
- US 5141931 A
- US 6331543 B
- WO 9117991 A
- WO 9200968 A
- WO 9212961 A
- WO 9307146 A
- WO 9315044 A
- WO 9315045 A
- WO 9318024 A
- WO 9319068 A
- WO 9319720 A
- WO 9319747 A
- WO 9319749 A
- WO 9319751 A
- WO 9325517 A
- WO 9402465 A
- WO 9406423 A
- WO 9412461 A
- WO 9420455 A
- WO 9422852 A
- WO 9425437 A
- WO 9427947 A
- WO 9500516 A
- WO 9501980 A
- WO 9503794 A
- WO 9504045 A
- WO 9504046 A
- WO 9505386 A
- WO 9508534 A
- WO 9509623 A
- WO 9509624 A
- WO 9509627 A
- WO 9509836 A
- WO 9514667 A
- WO 9514680 A
- WO 9514681 A
- WO 9517392 A
- WO 9517399 A
- WO 9519362 A
- WO 9522520 A
- WO 9524381 A
- WO 9527692 A
- WO 9528926 A
- WO 9535281 A
- WO 9535282 A
- WO 9600218 A
- WO 9601825 A
- WO 9602541 A
- WO 9611917 A
- WO 9307124 A
- WO 9501338 A
- WO 9603399 A
- US 20050004222 A
- US 2011043267 W
- US 2011043261 W
- US 2011043232 W
- US 2011043266 W
- US 2011043263 W
- US 8134015 B
- WO 2014169094 A

**Non-patent literature cited in the description**

- **KING et al.** *Sci Transl Med.*, 29 August 2018, vol. 10, 456
- **AHMAD, S. et al.** Aminoimidazoles as Bioisosteres of Acylguanidines: Novel, Potent, Selective and Orally Bioavailable Inhibitors of the Sodium Hydrogen Exchanger Isoform-1. *Boorganic & Med. Chem. Lett*, 2004, 177-180
- Chemistry of NHE Inhibitors. **LANG, H. J.** The Sodium-Hydrogen Exchanger. Kluwer Academic Publishers, 2003
- **B. MASEREEL et al.** An Overview of Inhibitors of Na+ / H+ Exchanger. *European J. of Med. Chem.*, 2003, vol. 38, 547-554
- **VIGNE et al.** *J. Biol. Chem.*, 1982, vol. 257, 9394
- **AHMAD, S. et al.** Aminoimidazoles as Bioisosteres of Acylguanidines: Novel, Potent, Selective and Orally Bioavailable Inhibitors of the Sodium Hydrogen Exchanger Isoform-1. *Boorganic & Med. Chem. Lett.*, 2004, 177-180
- **WILEY, R. A** ; **RICH, D. H.** *Medical Research Reviews*, 1993, vol. 13 (3), 327-384

- **TAKEUCHI et al.** *Am. J. Physiol.*, 1997, vol. 272 (1), G646-53
- **KAMENETSKY et al.** *J. Mol. Biol.*, 2006, vol. 362, 623-639
- **TRESGUERRES et al.** *Kidney Int.*, 2011, vol. 79, 1277-1288
- **HARTZELL** ; **BUDNITZ**. *Molecular Pharmacology*, 1992, vol. 41, 880-888
- **LAURENZA et al.** *Mol Pharmacol.*, 1987, vol. 32, 133-9
- **LAL et al.** *Bioorg Med Chem.*, 1998, vol. 6, 2075-83
- **MORI et al.** *J. Cardiovasc. Pharmacol.*, 2004, vol. 24, 310-6
- **LEVIN**. *Tetrahedon Letters*, 1996, vol. 37, 3079-3082
- **LAL et al.** *Indian J. Chemistry.*, 2006, vol. 45B, 232-246
- **KHANDELWAL et al.** *J Med Chem.*, 1988, vol. 31, 1872-9
- **CUNLIFFE et al.** *Electrophoresis*, 2007, vol. 28, 1913-20
- **KONYA et al.** *Pharmacol Ther.*, 2013, vol. 138, 485-502
- **ZHANG et al.** *Med Res Rev*, 2009, vol. 29, 272-94
- **YVONNE CONNOLLY MARTIN**. *International Journal of Medicinal Chemistry*, 2011, vol. 2011, 8
- **SALMI et al.** *CNS Drug Rev.*, 2004, vol. 10, 230-42
- **BOURNE**. *CNS Drug Rev*, 2001, vol. 7, 399-414
- **JIANG et al.** *Acta Pharmacol Sin.*, 2005, vol. 26, 1181-6
- **SJÖBLOM et al.** *J Clin Invest.*, 2001, vol. 108, 625-33
- **SJÖBLOM** ; **FLEMSTROM**. *J. Pineal Res.*, 2003, vol. 34, 288-293
- **RIVARA et al.** *Curr Top Med Chem.*, 2008, vol. 8, 954-68
- **SUGEN et al.** *Pigment Cell Research.*, 2004, vol. 17, 454-460
- *CHEMICAL ABSTRACTS*, 90182-92-6
- *CHEMICAL ABSTRACTS*, 141196-99-8
- *CHEMICAL ABSTRACTS*, 146388-57-0
- *CHEMICAL ABSTRACTS*, 127595-43-1
- *CHEMICAL ABSTRACTS*, 174486-39-6
- *CHEMICAL ABSTRACTS*, 148868-55-7
- **BUCHHEIT et al.** *J Med. Chem.*, 1995, vol. 38, 2331-8
- *CHEMICAL ABSTRACTS*, 102671-04-5
- *CHEMICAL ABSTRACTS*, 189188-57-6
- *CHEMICAL ABSTRACTS*, 99617-34-2
- *CHEMICAL ABSTRACTS*, 89613-77-4
- **MARKSTEIN et al.** *Naunyn-Schmiedebergs Arch Pharmacol.*, 1999, vol. 359, 454-9
- **SONDA et al.** *Bioorg Med. Chem.*, 2004, vol. 12, 2737-47
- **TAZAWA et al.** *Eur J Pharmacol.*, 2002, vol. 434, 169-76
- *CHEMICAL ABSTRACTS*, 364-62-5
- *CHEMICAL ABSTRACTS*, 608-07-1
- *CHEMICAL ABSTRACTS*, 168986-61-6
- *CHEMICAL ABSTRACTS*, 155106-73-3
- **BUCCAFUSCO et al.** *Pharmacology.*, 2000, vol. 295, 438-446
- *CHEMICAL ABSTRACTS*, 2576-84-3
- *CHEMICAL ABSTRACTS*, 5581-45-3
- *CHEMICAL ABSTRACTS*, 7232-21-5
- *CHEMICAL ABSTRACTS*, 54143-57-6
- **MAEYER et al.** *Neurogastroenterology and Motility.*, 2008, vol. 20, 99-112
- **MANABE et al.** *Expert Opin Investig Drugs.*, 2010, vol. 19, 765-75
- **TACK et al.** *Alimentary Pharmacology & Ther.*, 2012, vol. 35, 745-767
- **SHUTO et al.** *J.Am.Soc.Nephrol., v.*, 2009, vol. 20 (7), 1504-1512
- **KIRKPANTUR et al.** *Nephrol Dial Transplant.*, 2011, vol. 26, 1346-54
- **SARKAR et al.** *Semin Dial.*, 2006, vol. 19, 429-33
- **DI MARCO et al.** *Kidney International.*, 2013, vol. 83, 213-222
- **VAN et al.** *J Clin Biochem Nutr.*, 2012, vol. 51, 27-32
- **GIACHELLI**. *Kidney Int.*, 2009, vol. 75, 890-897
- **GUERIN et al.** *Circulation.*, 2001, vol. 103, 987-992
- **OHNISHI** ; **RAZZAQUE**. *FASEB J.*, 2010, vol. 24, 3562-71
- **NEVES et al.** *Kidney Int.*, 2004, vol. 66, 2237-44
- **ACHINGER** ; **AYUS**. *Am Soc Nephrol.*, 2006, vol. 17 (3), S255-61
- Remington: The Science and Practice of Pharmacy. Philadelphia College of Pharmacy and Science, 2000
- **GREEN, T.W.** ; **P.G.M. WUTZ**. Protective Groups in Organic Synthesis. Wiley, 1999
- **PARADISO**. *PNAS USA.*, 1984, vol. 81, 7436-7440
- **LOPEZ et al.** *J. Am. Soc. Nephrol.*, 2006, vol. 17, 795-804